# EUROPEAN PATENT APPLICATION

(11) **EP 0 902 028 A1**
(43) Date of publication of application: **17.03.1999**
(21) Application number: 98115314.1
(22) Date of filing: 14.08.1998
(51) Int. Cl.: C07D 405/04, C07D 405/14, A01N 43/713

(54) **Tetrazoline herbicides**

(30) Priority: 11.09.1997 US 58361 P; 21.07.1998 US 119789
(71) Applicant: E.I. DU PONT DE NEMOURS AND COMPANY, Wilmington Delaware 19898 (US)
(72) Inventor: Rorer, Morris Padgett, Newark, Delaware 19711 (US)
(74) Representative: Woodman, Derek

(57) **Abstract**

Compounds of Formula I, and their *N*-oxides and agriculturally suitable salts, are disclosed which are useful for controlling undesired vegetation 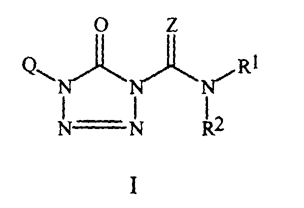 wherein
Q is 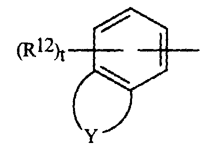
and Z, Y, R¹, R², R¹², and t are as defined in the disclosure.

Also disclosed are compositions containing the compounds of Formula I and a method for controlling undesired vegetation which involves contacting the vegetation or its environment with an effective amount of a compound of Formula I.

## Description

### BACKGROUND OF THE INVENTION

This invention relates to certain tetrazolinones, their *N*-oxides, their agriculturally suitable salts, compositions thereof, and methods of their use for controlling undesirable vegetation.

The control of undesired vegetation is extremely important in achieving high crop efficiency. Achievement of selective control of the growth of weeds especially in such useful crops as rice, soybean, sugar beet, corn (maize), potato, wheat, barley, tomato and plantation crops, among others, is very desirable. Unchecked weed growth in such useful crops can cause significant reduction in productivity and thereby result in increased costs to the consumer. The control of undesired vegetation in noncrop areas is also important. Many products are commercially available for these purposes, but the need continues for new compounds which are more effective, less costly, less toxic, environmentally safer or have different modes of action.

U.S. Patent 4,618,365 discloses compounds of Formula i as herbicides: 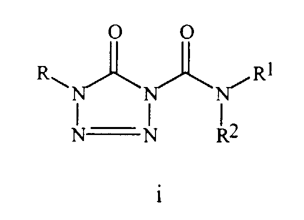 wherein, *inter alia*,
R is phenyl optionally substituted with methylenedioxy; and
R¹ and R² are independently C₁-C₆ alkyl, C₃-C₆ alkenyl or C₅-C₆ cycloalkyl.

The tetrazolinones of the present invention are not disclosed in this publication.

### SUMMARY OF THE INVENTION

This invention is directed to compounds of Formula I including all geometric and stereoisomers, *N*-oxides, and agriculturally suitable salts thereof, as well as agricultural compositions containing them and a method of their use for controlling undesirable vegetation: 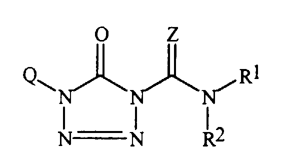 wherein
Q is 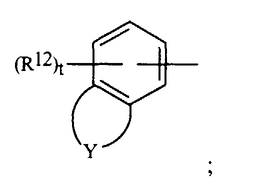
Y is, together with the carbons to which it is attached, a fully or partially saturated 5-, 6- or 7-membered heterocyclic ring which contains one or two X^{a} and is optionally substituted with 1-4 R^{3a}, provided that when said heterocyclic ring contains two X^{a}, then one X^{a} is other than O; or
Y is, together with the carbons to which it is attached, a 5- or 6-membered aromatic heterocyclic ring which contains one, two or three X^{b}, and is optionally substituted with 1-2 R^{3b}, provided that (a) when Y together with the carbons to which it is attached form a 6-membered aromatic heterocyclic ring, then X^{b} is N, and (b) when X^{b} is N substituted with R^{3b}, then R^{3b} is other than halogen;
X^{a} is O, S(O)ₙ or NR¹⁰,
X^{b} is O, S or N;
Z is O or S;
R¹ is C₁-C₆ alkyl, C₁-C₆ haloalkyl, C₃-C₆ alkenyl, C₃-C₆ haloalkenyl, C₃-C₆ alkynyl, C₃-C₆ haloalkynyl, C₁-C₆ alkoxy, C₂-C₆ alkoxyalkyl, or C₂-C₆ haloalkoxyalkyl; or R¹ is C₃-C₇ cycloalkyl or C₃-C₇ cycloalkenyl, each optionally substituted with 1-4 R¹³; or R¹ is phenyl optionally substituted with C₁-C₃ alkyl, C₁-C₃ alkoxy, C₁-C₃ haloalkoxy, C₁-C₃ haloalkyl, halogen, cyano, nitro or C₂-C₄ alkoxycarbonyl;
R² is C₁-C₆ alkyl, C₁-C₆ haloalkyl, C₃-C₇ cycloalkyl, C₃-C₆ alkenyl, C₃-C₆ haloalkenyl, C₃-C₆ alkynyl, C₃-C₆ haloalkynyl, C₁-C₆ alkoxy, C₂-C₆ alkoxyalkyl, C₂-C₆ haloalkoxyalkyl or NR⁴R⁵; or
R² is 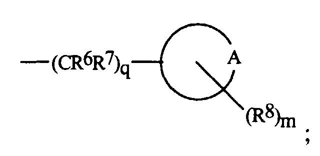 or
R¹ and R² can be taken together as -CH₂CH₂-, -CH₂CH₂CH₂-, -CH₂CH₂CH₂CH₂-, -CH₂CH₂CH₂CH₂CH₂- or -CH₂CH₂OCH₂CH₂-;
each R^{3a} is independently halogen, C₁-C₄ alkyl or C₁-C₄ alkoxy; or when two R^{3a} are attached to the same carbon, then said two R^{3a} groups can be taken together as C(=O);
each R^{3b} is independently halogen, C₁-C₄ alkyl, C₁-C₄ haloalkyl or C₁-C₄ alkoxy;
R⁴ is C₁-C₆ alkyl, C₁-C₆ haloalkyl, C₃-C₆ alkenyl, C₃-C₆ haloalkenyl, C₃-C₆ alkynyl, C₃-C₆ haloalkynyl; or R⁴ is C₃-C₇ cycloalkyl or C₃-C₇ cycloalkenyl, each optionally substituted with 1-4 R¹³; or R⁴ is a saturated or partially saturated 5-, 6- or 7-membered heterocyclic ring containing 1 to 2 heteroatoms independently selected from the group nitrogen, oxygen, and sulfur, and each heterocyclic ring is optionally substituted with 1-4 R^{3a}; or R⁴ is phenyl optionally substituted on the phenyl ring with C₁-C₃ alkyl, C₁-C₃ haloalkyl, halogen, cyano or nitro; or
R¹ and R⁴ can be taken together with the two nitrogen atoms to which they are attached to form a saturated or partially saturated 5-, 6- or 7-membered heterocyclic ring containing an optional third heteroatom selected from oxygen, sulfur or nitrogen, and said heterocyclic ring can be optionally substituted with 1-4 R⁹;
R⁵ is H or C₁-C₄ alkyl; or
R⁴ and R⁵ can be taken together with the nitrogen atom to which they are attached to form a saturated or partially saturated 5-, 6- or 7-membered heterocyclic ring containing an optional second heteroatom selected from oxygen, sulfur or nitrogen, and said heterocyclic ring can be optionally substituted with 1-4 R⁹;
R⁶ is H or C₁-C₄ alkyl;
R⁷ is H or C₁-C₄ alkyl;
each R⁸ is independently C₁-C₄ alkyl;
each R⁹ is independently C₁-C₄ alkyl; or when two R⁹ are attached to the same carbon, then said two R⁹ groups can be taken together as C(=O);
A is, together with the carbon to which it is attached, a fully or partially saturated 5-, 6- or 7-membered heterocyclic ring containing one or two X, provided that (a) when X is other than O or S(O)ₙ, then only one X may be present; (b) when two X are present in the ring, they cannot be bonded directly to each other; and (c) said heterocyclic ring is bonded to the group (CR⁶R⁷)_{q} through other than X;
X is O, S(O)ₙ, NR¹⁰ or Si(R¹¹)₂;
R¹⁰ is H, C₁-C₄ alkyl, C₁-C₄ haloalkyl, C₃-C₄ alkenyl, C₃-C₄ alkynyl, C₂-C₄ alkoxycarbonyl or C₂-C₄ alkylcarbonyl; or R¹⁰ is phenyl optionally substituted on the phenyl ring with C₁-C₃ alkyl, halogen, cyano, nitro or C₂-C₄ alkoxycarbonyl;
each R¹¹ is independently C₁-C₄ alkyl;
R¹² is halogen, C₁-C₄ alkyl, C₁-C₄ haloalkyl, C₁-C₄ alkoxy, C₁-C₄ haloalkoxy, C₁-C₄ alkylthio, C₁-C₄ thioalkoxy, C₁-C₄ halothioalkoxy or C₂-C₄ alkoxycarbonyl;
each R¹³ is independently halogen, C₁-C₄ alkyl or C₁-C₄ alkoxy; or when two R¹³ are attached to the same carbon, then said two R¹³ groups can be taken together as C(=O);
m is 0, 1, 2, 3 or 4;
each n is independently 0, 1 or 2;
q is 0, 1 or 2; and
t is 0, 1 or 2.

In the above recitations, the term "alkyl", used either alone or in compound words such as "alkylthio" or "haloalkyl" includes straight-chain or branched alkyl, such as, methyl, ethyl, *n*-propyl, *i*-propyl, or the different butyl, pentyl or hexyl isomers. The term "1-2 alkyl" indicates that one or two of the available positions for that substituent may be alkyl which are independently selected. "Alkenyl" includes straight-chain or branched alkenes such as ethenyl, 1-propenyl, 2-propenyl, and the different butenyl, pentenyl and hexenyl isomers. "Alkenyl" also includes polyenes such as 1,2-propadienyl and 2,4-hexadienyl. "Alkynyl" includes straight-chain or branched alkynes such as ethynyl, 1-propynyl, 2-propynyl and the different butynyl, pentynyl and hexynyl isomers. "Alkynyl" can also include moieties comprised of multiple triple bonds such as 2,5-hexadiynyl. "Alkoxy" includes, for example, methoxy, ethoxy, *n*-propyloxy, isopropyloxy and the different butoxy, pentoxy and hexyloxy isomers. "Alkoxyalkyl" denotes alkoxy substitution on alkyl. Examples of "alkoxyalkyl" include CH₃OCH₂, CH₃OCH₂CH₂, CH₃CH₂OCH₂, CH₃CH₂CH₂CH₂OCH₂ and CH₃CH₂OCH₂CH₂. "Alkylthio" includes branched or straight-chain alkylthio moieties such as methylthio, ethylthio, and the different propylthio, butylthio, pentylthio and hexylthio isomers. "Alkylsulfinyl" includes both enantiomers of an alkylsulfinyl group. Examples of "alkylsulfinyl" include CH₃S(O), CH₃CH₂S(O), CH₃CH₂CH₂S(O), (CH₃)₂CHS(O) and the different butylsulfinyl, pentylsulfinyl and hexylsulfinyl isomers. Examples of "alkylsulfonyl" include CH₃S(O)₂, CH₃CH₂S(O)₂, CH₃CH₂CH₂S(O)₂, (CH₃)₂CHS(O)₂ and the different butylsulfonyl, pentylsulfonyl and hexylsulfonyl isomers. "Alkylamino", "dialkylamino", "alkenylthio", "alkenylsulfinyl", "alkenylsulfonyl", "alkynylthio", "alkynylsulfinyl", "alkynylsulfonyl", and the like, are defined analogously to the above examples. "Cycloalkyl" includes, for example, cyclopropyl, cyclobutyl, cyclopentyl, and cyclohexyl. The term "cycloalkoxy" includes the same groups linked through an oxygen atom such as cyclopentyloxy and cyclohexyloxy. "Cycloalkenyl" includes groups such as cyclopentenyl and cyclohexenyl as well as groups with more than one double bond such as 1,3- and 1,4-cyclohexadienyl. The term "aromatic ring system" denotes fully unsaturated carbocycles and heterocycles in which the polycyclic ring system is aromatic (where aromatic indicates that the Hückel rule is satisfied for the ring system). The term "aromatic heterocyclic ring system" includes fully aromatic heterocycles and heterocycles in which at least one ring of a polycyclic ring system is aromatic (where aromatic indicates that the Hückel rule is satisfied). The heterocyclic ring systems can be attached through any available carbon or nitrogen by replacement of a hydrogen on said carbon or nitrogen. One skilled in the art will appreciate that not all nitrogen containing heterocycles can form *N*-oxides since the nitrogen requires an available lone pair for oxidation to the oxide; one skilled in the art will recognize those nitrogen containing heterocycles which can form *N*-oxides. One skilled in the art will also recognize that tertiary amines can form *N*-oxides. Synthetic methods for the preparation of *N*-oxides of heterocycles and tertiary amines are very well known by one skilled in the art including the oxidation of heterocycles and tertiary amines with peroxy acids such as peracetic and *m*-chloroperbenzoic acid (MCPBA), hydrogen peroxide, alkyl hydroperoxides such as *t*-butyl hydroperoxide, sodium perborate, and dioxiranes such as dimethyldioxirane. These methods for the preparation of *N*-oxides have been extensively described and reviewed in the literature, see for example: T. L. Gilchrist in *Comprehensive Organic Synthesis*, vol. 7, pp 748-750, S. V. Ley, Ed., Pergamon Press; M. Tisler and B. Stanovnik in *Comprehensive Heterocyclic Chemistry*, vol. 3, pp 18-20, A. J. Boulton and A. McKillop, Eds., Pergamon Press; M. R. Grimmett and B. R. T. Keene in *Advances in Heterocyclic Chemistry*, vol. 43, pp 149-161, A. R. Katritzky, Ed., Academic Press; M. Tisler and B. Stanovnik in *Advances in Heterocyclic Chemistry*, vol. 9, pp 285-291, A. R. Katritzky and A. J. Boulton, Eds., Academic Press; and G. W. H. Cheeseman and E. S. G. Werstiuk in *Advances in Heterocyclic Chemistry*, vol. 22, pp 390-392, A. R. Katritzky and A. J. Boulton, Eds., Academic Press.

The term "halogen", either alone or in compound words such as "haloalkyl", includes fluorine, chlorine, bromine or iodine. The term "1-2 halogen" indicates that one or two of the available positions for that substituent may be halogen which are independently selected. Further, when used in compound words such as "haloalkyl", said alkyl may be partially or fully substituted with halogen atoms which may be the same or different. Examples of "haloalkyl" include F₃C, ClCH₂, CF₃CH₂ and CF₃CCl₂. The terms "haloalkenyl", "haloalkynyl", "haloalkoxy", "haloalkylthio", and the like, are defined analogously to the term "haloalkyl". Examples of "haloalkenyl" include (Cl)₂C=CHCH₂ and CF₃CH₂CH=CHCH₂. Examples of "haloalkynyl" include HC≡CCHCl, CF₃C≡C, CCl₃C≡C and FCH₂C≡CCH₂. Examples of "haloalkoxy" include CF₃O, CCl₃CH₂O, HCF₂CH₂CH₂O and CF₃CH₂O. Examples of "haloalkylthio" include CCl₃S, CF₃S, CCl₃CH₂S and ClCH₂CH₂CH₂S. Examples of "haloalkylsulfinyl" include CF₃S(O), CCl₃S(O), CF₃CH₂S(O) and CF₃CF₂S(O). Examples of "haloalkylsulfonyl" include CF₃S(O)₂, CCl₃S(O)₂, CF₃CH₂S(O)₂ and CF₃CF₂S(O)₂.

The total number of carbon atoms in a substituent group is indicated by the "Cᵢ-Cⱼ" prefix where i and j are numbers from 1 to 7. For example, C₁-C₃ alkylsulfonyl designates methylsulfonyl through propylsulfonyl; C₂ alkoxyalkyl designates CH₃OCH₂; C₃ alkoxyalkyl designates, for example, CH₃CH(OCH₃), CH₃OCH₂CH₂ or CH₃CH₂OCH₂; and C₄ alkoxyalkyl designates the various isomers of an alkyl group substituted with an alkoxy group containing a total of four carbon atoms, examples including CH₃CH₂CH₂OCH₂ and CH₃CH₂OCH₂CH₂. Examples of "alkylcarbonyl" include C(O)CH₃, C(O)CH₂CH₂CH₃ and C(O)CH(CH₃)₂. Examples of "alkoxycarbonyl" include CH₃OC(=O), CH₃CH₂OC(=O), CH₃CH₂CH₂OC(=O), (CH₃)₂CHOC(=O) and the different butoxy- or pentoxycarbonyl isomers. In the above recitations, when a compound of Formula I is comprised of one or more heterocyclic rings, all substituents are attached to these rings through any available carbon or nitrogen by replacement of a hydrogen on said carbon or nitrogen.

When a compound is substituted with a substituent bearing a subscript that indicates the number of said substituents can exceed 1, said substituents (when they exceed 1) are independently selected from the group of defined substituents. Further, when the subscript indicates a range, e.g. (R)ᵢ₋ⱼ, then the number of substituents may be selected from the integers between i and j inclusive.

When a group contains a substituent which can be hydrogen, for example R¹⁰, then, when this substituent is taken as hydrogen, it is recognized that this is equivalent to said group being unsubstituted.

The compounds of this invention thus include compounds of Formula I, geometric and stereoisomers thereof, N-oxides thereof, and agriculturally suitable salts thereof. The compounds of the invention can exist as one or more stereoisomers. The various stereoisomers include enantiomers, diastereomers, atropisomers and geometric isomers. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers. The compounds of the invention may be present as a mixture of stereoisomers, individual stereoisomers, or as an optically active form.

The salts of the compounds of the invention include acid-addition salts with inorganic or organic acids such as hydrobromic, hydrochloric, nitric, phosphoric, sulfuric, acetic, butyric, fumaric, lactic, maleic, malonic, oxalic, propionic, salicylic, tartaric, 4-toluenesulfonic or valeric acids. The salts of the compounds of the invention also include those formed with organic bases (e.g., pyridine, ammonia, or triethylamine) or inorganic bases (e.g., hydrides, hydroxides, or carbonates of sodium, potassium, lithium, calcium, magnesium or barium) when the compound contains an acidic group such as a carboxylic acid or phenol.

Preferred compounds of the invention for reasons of better activity and/or ease of synthesis are:
Preferred 1. Compounds of Formula I above, geometric and stereoisomers thereof, *N*-oxides thereof, and agriculturally suitable salts thereof, wherein
   Q is 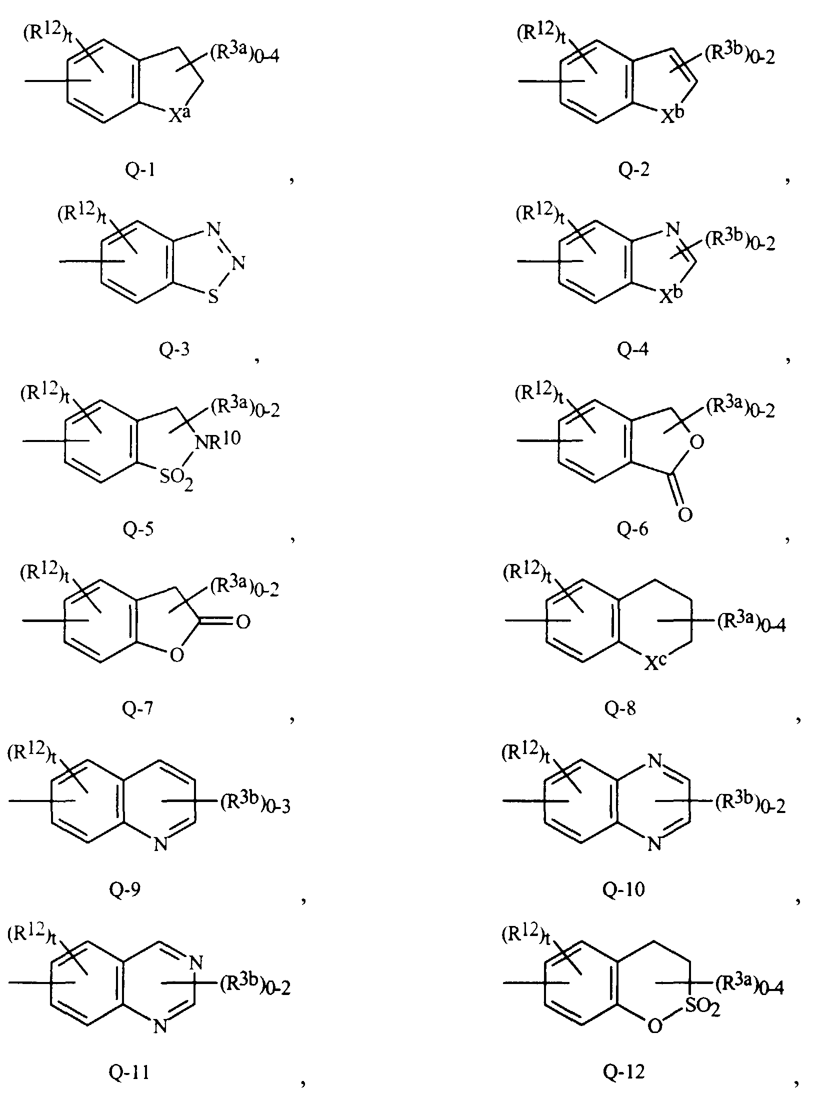 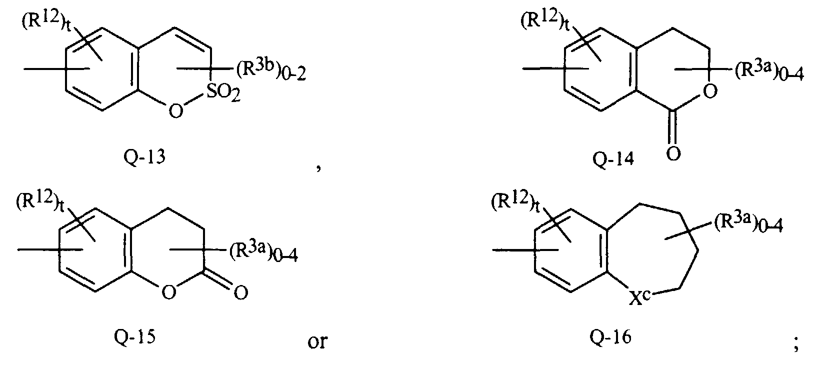 and
   X^{c} is O or S(O)ₙ.
Preferred 2. Compounds of Preferred 1 wherein
   Q is Q-1, Q-2 or Q-8.
Preferred 3. Compounds of Preferred 2 wherein
   Q is 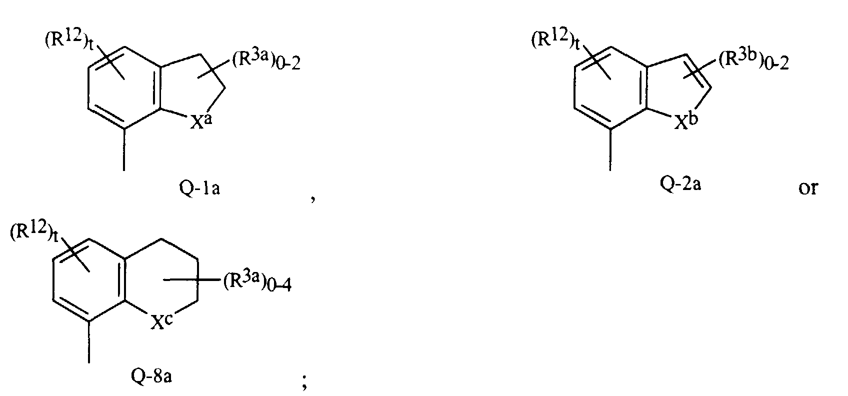 and
   R^{3a} is C₁-C₄ alkyl.
Preferred 4. Compounds of Preferred 3 wherein
   X^{a}, X^{b}, and X^{c} are independently O or S.
Preferred 5. Compounds of Preferred 4 wherein
   R¹ is C₁-C₆ alkyl or C₁-C₆ haloalkyl; or R¹ is C₃-C₇ cycloalkyl or C₃-C₇ cycloalkenyl, each optionally substituted with 1-4 R¹³;
   R² is C₁-C₆ alkyl or C₃-C₇ cycloalkyl; or
   R¹ and R² can be taken together as -CH₂CH₂-, -CH₂CH₂CH₂-, -CH₂CH₂CH₂CH₂-, -CH₂CH₂CH₂CH₂CH₂- or -CH₂CH₂OCH₂CH₂-.
Preferred 6. Compounds of Preferred 4 wherein
   R¹ is C₁-C₆ alkyl or C₁-C₆ haloalkyl; or R¹ is C₃-C₇ cycloalkyl or C₃-C₇ cycloalkenyl, each optionally substituted with 1-4 R¹³;
   R² is 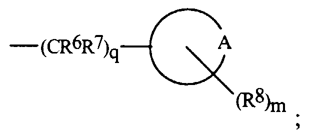
   A together with the carbon to which it is attached form a fully or partially saturated 5-, 6- or 7-membered heterocyclic ring containing one X;
   X is O, S or NR¹⁰; and
   q is 0.
Preferred 7. Compounds of Preferred 4 wherein
   R¹ is C₃-C₇ cycloalkyl or C₃-C₇ cycloalkenyl, each optionally substituted with 1-4 R¹³;
   R² is NR⁴R⁵; and
   R⁴ is C₁-C₆ alkyl.
Preferred 8. Compounds of Preferred 4 wherein
   R¹ is C₁-C₆ alkyl;
   R² is NR⁴R⁵; and
   R⁴ is C₃-C₇ cycloalkyl or C₃-C₇ cycloalkenyl, each optionally substituted with 1-4 R¹³.

Most preferred is the compound of Preferred 4 which is 4-(2,3-dihydro-2-methyl-7-benzofuranyl)-*N,N*-diethyl-4,5-dihydro-5-oxo-1*H*-tetrazol-1-carboxamide.

This invention also relates to herbicidal compositions comprising herbicidally effective amounts of the compounds of the invention and at least one of a surfactant, a solid diluent or a liquid diluent. The preferred compositions of the present invention are those which comprise the above preferred compounds.

This invention also relates to a method for controlling undesired vegetation comprising applying to the locus of the vegetation herbicidally effective amounts of the compounds of the invention (e.g., as a composition described herein). The preferred methods of use are those involving the above preferred compounds.

### DETAILS OF THE INVENTION

The compounds of Formula I can be prepared by one or more of the following methods and variations as described in Schemes 1-26. The definitions of A, Q, Z, R¹-R¹³, m, and n in the compounds of Formulae 1-30 below are as defined above in the Summary of the Invention.

Scheme 1 illustrates the preparation of compounds of Formula I whereby a tetrazolinone of Formula 1 is reacted with a carbamyl chloride or thiocarbamyl chloride of Formula 2 in the presence of a suitable acid acceptor agent. Suitable acid acceptor agents include alkali carbonates, alkali bicarbonates, alkyl tertiary amines such as triethylamine, pyridine, and, preferably, 4-dimethylaminopyridine (DMAP). Furthermore, DMAP can be used as a catalyst in the presence of another suitable acid acceptor agent in order to selectively synthesize a compound of Formula I. The reaction is carried out in an inert solvent such as tetrahydrofuran, acetone, chloroform, chlorobenzene or preferably acetonitrile or toluene, and at a temperature range between 0 °C and 110 °C by methods known in the art (or slight modification of these methods); for example, see Yanagi, A. et al. EP 646,577; Goto, T. et al. EP 708,097; Covey, R. A. et al. U.S. Patent 4,618,365; Goshima, T. et al. JP 9,100,272.

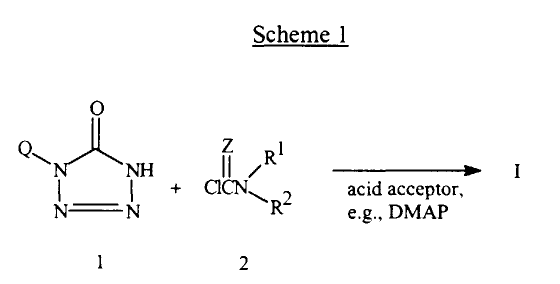

Alternatively, compounds of Formula I wherein Z is O can be prepared whereby a tetrazolinone of Formula 1 in an inert solvent such as toluene or ethyl acetate is reacted with phosgene and a suitable tertiary amine base such as triethylamine, and the product of such reaction is reacted with a secondary amine (or hydrazine when R² is NR⁴R⁵) of Formula 3, optionally in the presence of a suitable base such as pyridine (Scheme 2). This type of reaction can be carried out by methods known in the art (or slight modification of these methods); see, for example, Covey, R. A. et al. U.S. Patent 5,019,152.

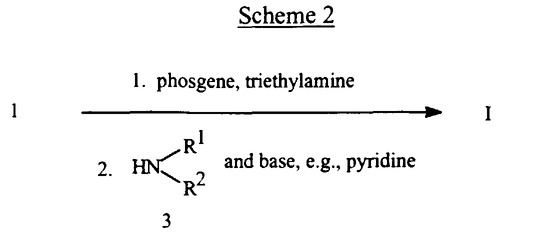

Scheme 3 illustrates a preferred method for preparing tetrazolinones of Formula 1 whereby an isocyanate of Formula 4 is reacted with refluxing trimethylsilylazide (also known as azidotrimethylsilane), followed by treatment of the product of such reaction with a protic solvent such as water or preferably with methanol. This type of reaction can be carried out by methods known in the art (or slight modification of these methods); see, for example, Tsuge, O. et al. *J. Org*. *Chem*. (1980), *45*, 5130; Goto, T. et al. EP 695,748 and EP 692,482.

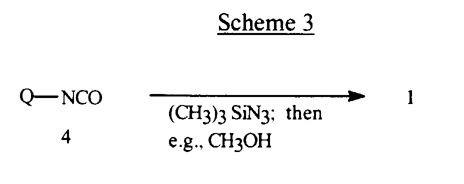

Scheme 4 illustrates another preferred method for preparing tetrazolinones of Formula 1 whereby an isocyanate of Formula 4 is reacted with sodium azide and aluminum chloride in an inert solvent such as *N,N*-dimethylformamide (DMF) followed by addition of water and a mineral acid in excess, such as hydrochloric acid. This type of reaction can be carried out by methods known in the art (or slight modification of these methods); see for example, Horwitz, J. P. et al. *J. Am. Chem. Soc*. (1959), *81*, 3076; Yanagi, A. et al. U.S. Patent 5,530,135; Covey, R. A. et al. U.S. Patent 4,618,365.

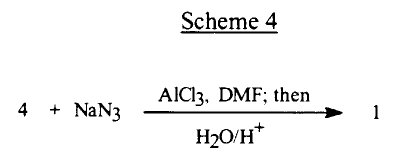

Many tetrazolinones of Formula 1 can also be prepared as illustrated in Scheme 5 whereby a phenyl carbamate of Formula 5 is reacted in an inert solvent such as dimethylformamide with sodium azide in the presence of aluminum chloride. This type of reaction can be carried out by methods known in the art (or slight modification of these methods); see for example, Goto, T. et al. EP 692,482 and EP 695,748.

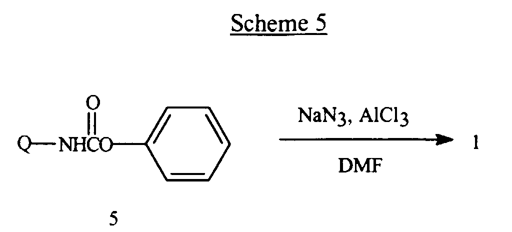

Phenyl carbamates of Formula 5 can be prepared by reaction of an appropriate amine with phenyl chloroformate in pyridine by methods known in the art (or slight modification of these methods); see, for example, Goto, T. et al. EP 692,482 and EP 695,748.

In addition, many tetrazolinones of Formula 1 can be prepared as illustrated in Scheme 6, whereby an appropriate acid chloride of Formula 6 is refluxed with excess trimethylsilylazide, and the product of such reaction is treated with a protic solvent such as water or, preferably with methanol. This type of reaction can be carried out by methods known in the art (or by slight modification of these methods); see, for example, Toselli, M. et al. *J. Chem. Soc. Perkin Trans 1*, (1992), 1101; Goto, T. et al. EP 695,748 and EP 692,482; Horwitz, J. P. et al. *J. Am. Chem. Soc*. (1959), *81*, 3076.

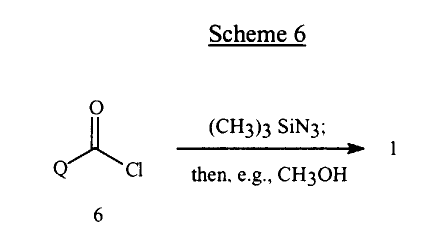

Alternatively, many tetrazolinones of Formula I can be prepared as illustrated in Scheme 7, whereby an appropriate mercaptotetrazole of Formula 7 is reacted in a solvent such as tetrahydrofuran, water or ethanol with an alkylene oxide such as propylene oxide and a suitable acid acceptor such as sodium hydroxide, followed by acidification with hydrochloric acid. This type of reaction can be carried out by methods known in the art (or slight modification of these methods); see, for example, Goto, T. et al. EP 711,761 and Yanagi, A. et al. EP 643,049.

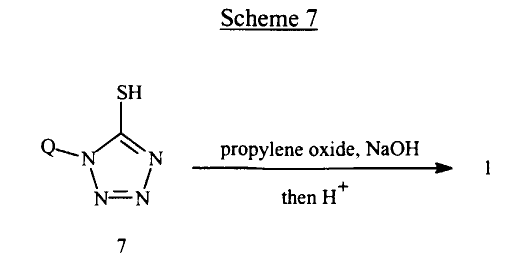

As illustrated in Scheme 8, many tetrazolinones of Formula 1 can also be prepared from tetrazolemethanesulfones of Formula 8 by reaction with sodium or potassium hydroxide in a solvent such as methanol or water, followed by acidification with hydrochloric acid. This type reaction can be carried out by methods known in the art (or slight modification of these methods); see, for example, Goto, T. et al. EP 692,482.

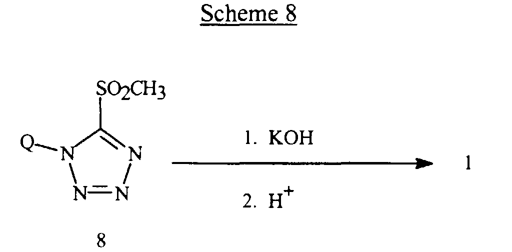

Sulfones of Formula 8 can be prepared by methylating a mercaptotetrazole of Formula 7 followed by an oxidation procedure with per acetic acid or Ozone® by generally known methods; see, for example, Goto, T. et al. EP 692,482.

Many carbamyl chlorides and thiocarbamyl chlorides of Formula 2 are well known in the art; see, for example, Goto, T. et al. EP 711,761, EP 692,482 and EP 695,748; Goshima, T. et al. JP 09,100,272.

Carbamyl chlorides of Formula 2a can be made by treating compounds of Formula 9 or Formula 10 with either phosgene or a phosgene equivalent such as diphosgene in the presence of a suitable base such as triethylamine (Scheme 9).

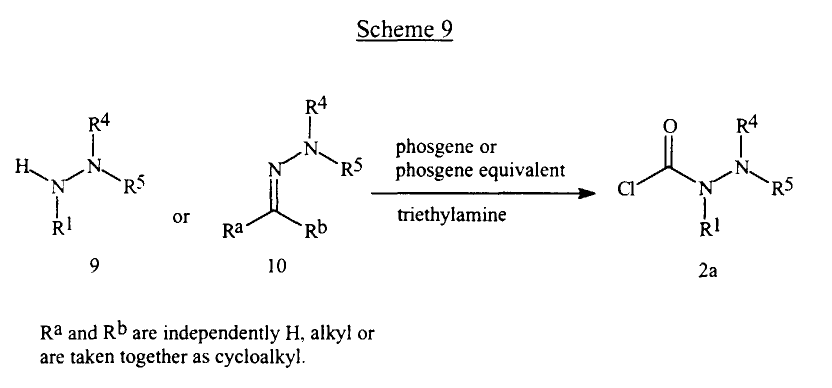

Hydrazines of Formula 9 can be made by the sequence of reactions shown in Scheme 10 whereby a ketone of Formula 11 is treated with an appropriate hydrazine of Formula 12 and the formed intermediate hydrazone of Formula 10 is subsequently reduced to the hydrazine of Formula 9. This type of reaction can be carried out by methods known in the art (or slight modification of these methods); see, for example, Takahashi, H. et al. *Chem*. *Pharm. Bull*. (1981), *29*, p 3387 and Spialter, L. et al. *J. Org*. *Chem*. (1965), *30*, p 3278.

Hydrazines of Formula 9 can also be made starting from commercially-available amines by methods well established in the art; for example, see Witte, J. et al. *J Org. Chem.* (1972), *37*, 2849.

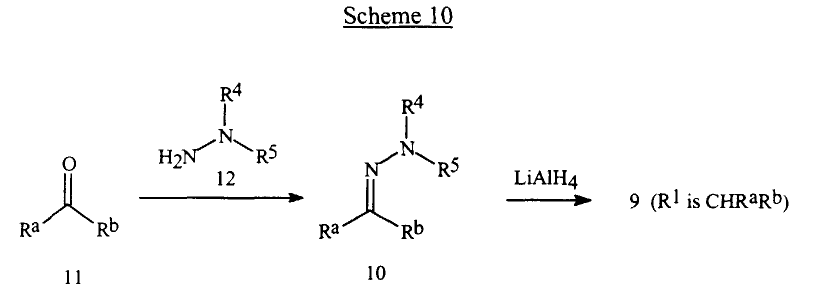

The ketones of Formula 11 are commercially available or can be prepared by methods well established in the art.

Carbamyl chlorides of Formula 2b can be made by treating amines of Formula 13 with phosgene or a phosgene equivalent such as diphosgene in the presence of a suitable base such as triethylamine (Scheme 11).

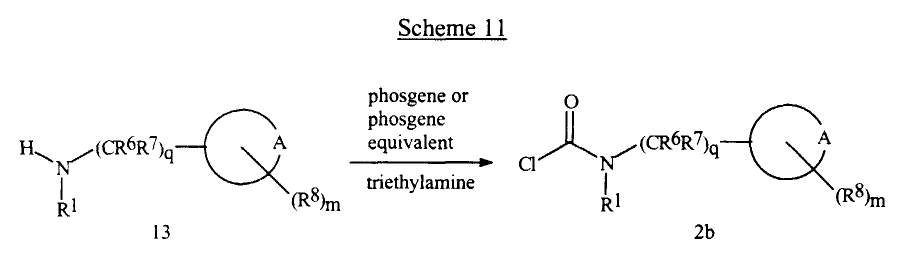

Amines of Formula 13 (wherein q is 0) can be made by the sequence of reactions shown in Scheme 12 whereby a ketone of Formula 14 is treated with an appropriate amine and the formed intermediate imine is subsequently reduced to the amine. This type of reaction can be carried out by methods known in the art (or slight modification of these methods); see, for example, Borch, R. *Org. Synthesis*, Collective Volume VI, (1988), p 499 and Bomann M. et al. *J Org. Chem.* (1995), *60*, p 5995.

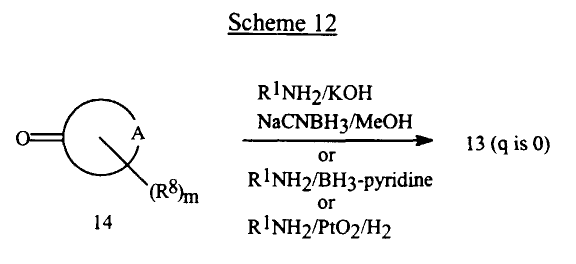

Many ketones of Formula 14 are either commercially available or can be prepared by methods known in the art; for example, see Soderquist, J. and Negron, A. *J. Org. Chem*. (1989), *54*, pp 2464-2466 for the preparation of 1-silacyclohexan-4-ones or Howard, E. and Lindsey, R. *J. Am. Chem. Soc*. (1960), *82*, pp 158-164 for the preparation of 5-*m*-dithianones.

Carbamyl chlorides of Formula 2c wherein a carbon-carbon double bond is present within the heterocyclic ring can be made as illustrated in Scheme 13. A ketone of Formula 15 is reacted with an appropriate amine in the presence of a dehydrating agent and the resulting imine intermediate of Formula 16 is treated with phosgene in the presence of a base such as triethylamine to yield the carbamyl chloride of Formula 2c.

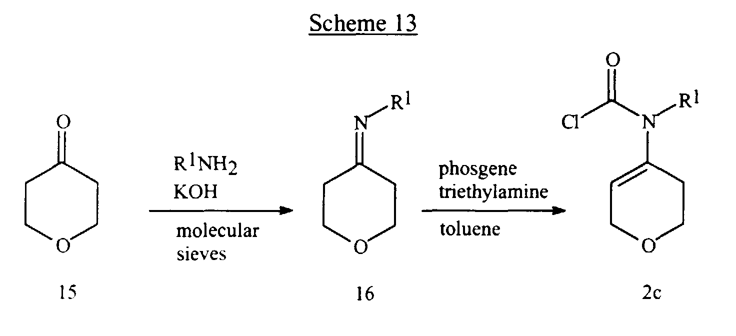

Carbamyl chlorides of Formula 2d can be made as illustrated in Scheme 14. An amine of Formula 17 is reacted with an appropriate acid anhydride in the presence of a base to provide the amide of Formula 18. Reduction of the amide with lithium aluminum hydride results in the amine of Formula 19 which is treated with phosgene in the presence of a base such as triethylamine to yield the carbamyl chloride of Formula 2d. Compounds of Formula 18 are either commercially available or can be prepared by methods well established in the art.

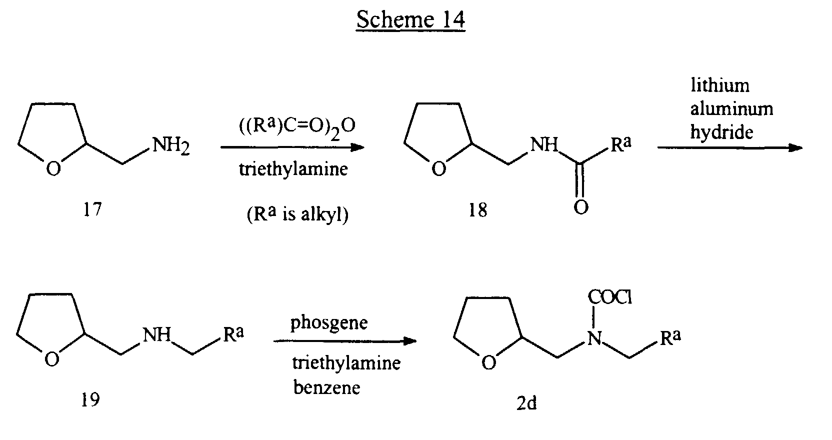

Carbamyl chlorides of Formula 2e can be made as illustrated in Scheme 15. An amine of Formula 17 is reacted with an appropriate carbonyl compound in the presence of molecular sieves, followed by reduction of the imine with sodium cyanoborohydride to give the amine of Formula 20. The amine is treated with phosgene in the presence of a base such as triethylamine to yield the carbamyl chloride of Formula 2e.

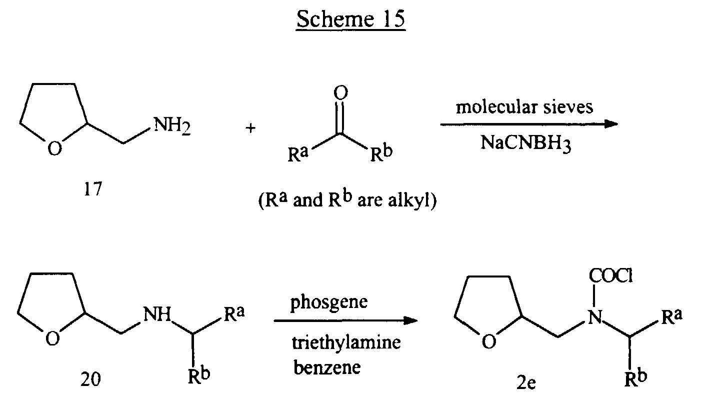

Scheme 16 illustrates the preparation of mercaptotetrazoles of Formula 7, whereby an isothiocyanate of Formula 21 is reacted with sodium azide in an inert solvent such as water or methanol, followed by acidification. This type of reaction can be carried out by methods known in the art (or slight modification of these methods); see, for example, Lieber, E. et al. *Can. J Chem*. (1959), *37*, 101; Shanker, R. B. et al. U.S. Patent 5,455,222.

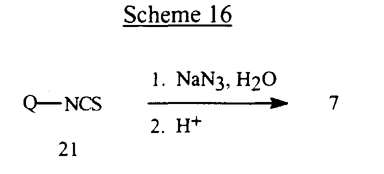

Isothiocyanates of Formula 21 can be prepared by reaction of an appropriate amine with thiophosgene, optionally in the presence of a base such as sodium bicarbonate, in an inert solvent such as methylene chloride or methylene chloride/water by methods generally known in the art.

Alternatively, as illustrated in Scheme 17, many mercaptotetrazoles can be prepared by a sequence of reactions whereby (1) an amino compound of Formula 22 is reacted with carbon disulfide and a tertiary amine base such as triethylamine either without solvent or preferably in a solvent such as ethanol; (2) the product of such reaction is reacted with an alkylating agent such as methyl iodide to form the corresponding methyl dithiocarbamate, and (3) subsequent reaction of this product with sodium azide in a solvent such as water provides a mercaptotetrazole of Formula 7. These type reactions can be carried out by methods known in the art (or slight modification of these methods); see, for example, Fairfull, A. E. *J. Chem. Soc*. (1955), 796; Knott, E. B. *J. Chem. Soc*. (1956), 1644; Shen, M. U. S. Patent 4,515,958: Goto, T. et al. EP 711,761.

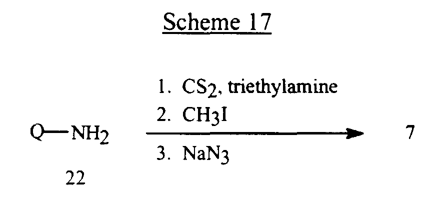

Many isocyanates of Formula 4 can be prepared by Curtius rearrangement of appropriate acid chlorides of Formula 6 using methods generally known in the art (Scheme 18); see, for example, March, J. *Advanced Organic Chemistry*, 3rd edition; John Wiley & Sons, 1985: pp 984-985 and 380.

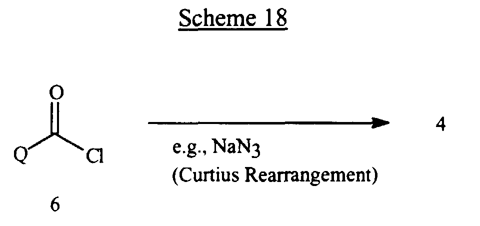

Acid chlorides of Formula 6 can be prepared by reacting an acid of Formula 23 with oxalyl chloride (or thionyl chloride) and optionally a catalytic amount of dimethylformamide (Scheme 19). This chlorination is well known in the art; see, for example, Michaely, W. J. EP 369,803; Goto, T. et al. EP 695,748. Other methods are also well known in the art for converting carboxylic acids to acid chlorides; see, for example, Ogliaruso, M. A. et al. *Synthesis of Carboxylic Acids, Esters and Their Derivatives*; John Wiley & Sons, 1991, pp 172-174.

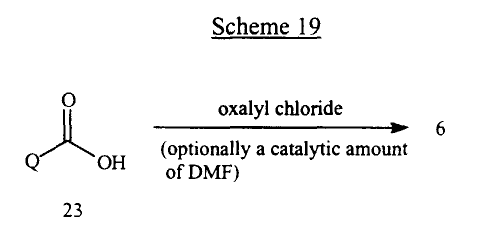

Scheme 20 illustrates the preparation of many carboxylic acids of Formula 23 whereby a bromide of Formula 24 is treated with *n*-butyllithium (or magnesium) and the lithium salt (or the Grignard reagent) generated *in situ* is then reacted with carbon dioxide followed by acidification with an acid such as hydrochloric acid. This conversion is carried out by using methods known in the art (or by slights modification of these methods); see for example, Ogliaruso, M. A. et al. *Synthesis of Carboxylic Acids, Esters and Their Derivatives*; John Wiley & Sons; pp 27-28; Bridges, A. J. et al. *J. Org. Chem*. (1990), *55*, 773; Franke, C. et al. *Angew. Chem. Int. Ed*. (1969), *8*, 68. Protecting and deprotecting functional groups not compatible with the reaction conditions may be necessary for compounds with such functional groups.

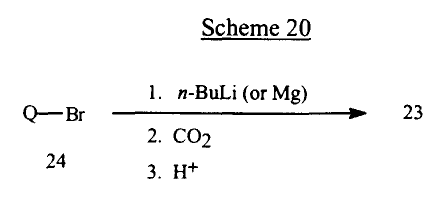

Many carboxylic acids of Formula 23 can also be prepared, as illustrated in Scheme 21, whereby a cyanide compound of Formula 25 is hydrolyzed or saponified. Alternatively, cyanide 25 can be partially hydrolyzed or saponified to form an amide of Formula 26 which can then be reacted with a nitrosating agent such as nitrosonium tetrafluoroborate in acetonitrile to form a carboxylic acid of Formula 23. Cyanides of Formula 25 are prepared by reacting a bromo compound of Formula 24 with cuprous cyanide in an inert solvent such as DMF. These reactions can be carried out by methods known in the art (or slight modifications of these methods); see, for example, Ogliaruso, M. A. et al. *Synthesis of Carboxylic Acids, Esters and Their Derivatives*; John Wiley & Sons, 1991; pp 7-9; Olah, G. A. et al. *J. Org. Chem*. (1965), *30*, 2386.

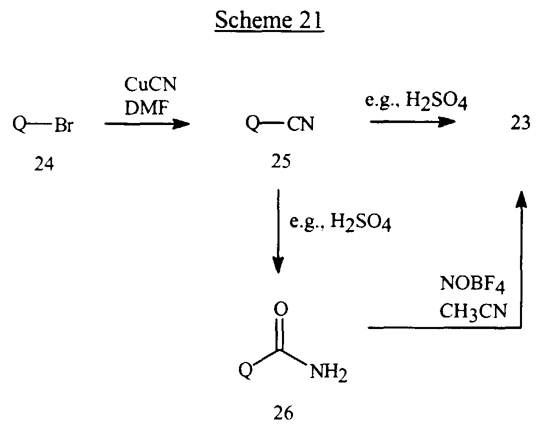

Many isocyanates of Formula 4 can also be prepared by treatment of the corresponding amines of Formula 22 with phosgene or known phosgene equivalents (e.g., diphosgene or triphosgene) by methods generally known in the art (Scheme 22); see for example, March, J. *Advanced Organic Chemistry*, 3rd edition; John Wiley & Sons, 1985, p 370; *Chem. Rev*. (1972), *72,* pp 457-496; Sandler, R. S. et al. *Organic Functional Group Preparations*, 2nd edition; Academic Press; Vol. II, pp 152 and 260; Lehman, G. et al. *Preparative Organic Chemistry*; John Wiley & Sons, 1972; p 472.

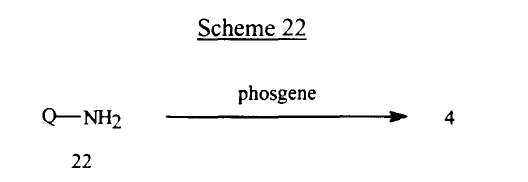

Amines of Formula 22 can be prepared by reduction of the corresponding nitro compounds of Formula 27 (Scheme 23). A wide variety of methods are documented in the chemical literature for carrying out such transformations; see for example, Rorer, M. P. U.S. Patent 4,511,392; Ohme, R. et al. *Preparative Organic Chemistry*; John Wiley & Sons, 1972; p 557; Groggins *Unit Processes in Organic Chemistry*; McGraw-Hill Book Co.: New York, 1947; pp 73- 128; March, J. *Advanced Organic Chemistry*, 3rd edition; John Wiley & Sons, 1985; pp 1103-1104.

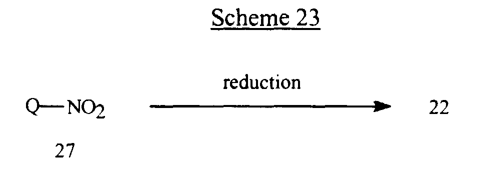

Scheme 24 illustrates the preparation of nitro compounds of Formula 27a by a Claisen rearrangement procedure, whereby an ether of Formula 28 is heated in a solvent such as *ortho*-dichlorobenzene (ODB) and the isolated phenolic product is cyclized by heating in an acid such as 48% aqueous hydrobromic acid in acetic acid. Alternatively, an ether of Formula 28 is heated with a catalyst such as anhydrous magnesium chloride, either neat or in a solvent such as ODB, to directly form a nitro compound of Formula 27a. These reactions and the preparation of ethers of Formula 28 are carried out by methods known in the art (or slight modification of these procedures); see, for example, Rorer, M. P. U. S. Patent 4,514,211 and references therein.

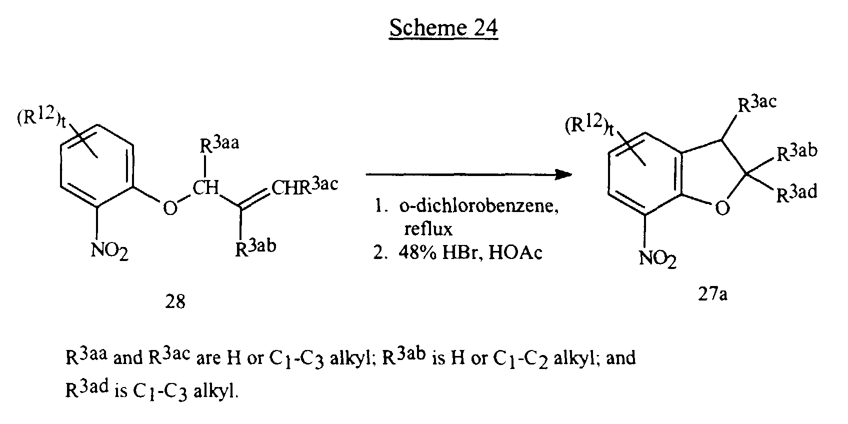

Scheme 25 illustrates the preparation of nitro compounds of Formula 27b, whereby an oxime of Formula 29 is heated in ethanol saturated with hydrogen chloride. This reaction and the preparation of oximes of Formula 29 are carried out by methods known in the art (or slight modification of these methods); see, for example, Mooradian, A. U.S. Patent 3,577,441; Kaminsky, D. et al. U.S. Patent 3,452,033.

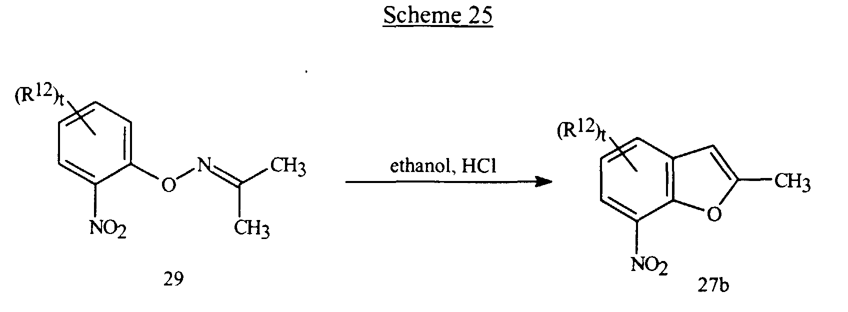

Scheme 26 illustrates the preparation of nitro compounds of Formula 27c, whereby a diphenoxypropane of Formula 30 is reacted with aluminum chloride in an inert solvent such as refluxing benzene to form an intermediate chroman; subsequent nitration with 70% nitric acid in acetic acid provides nitro compounds of Formula 27c. These reactions and the preparation diphenoxypropanes of Formula 30 are carried out by methods known in the art (or slight modification of these methods); see, for example, Deady, L. W. et al. *J. Chem. Soc.* (1963), 2094; Brancaccio, G. et al. *J. Het. Chem*. (1973), *10*, 623.

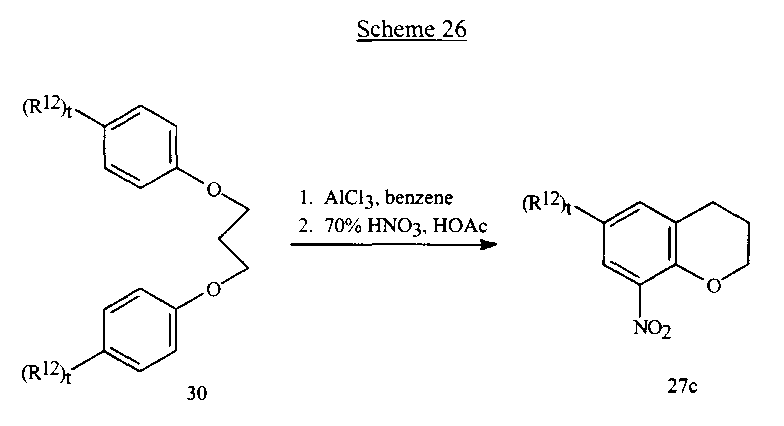

In general, other nitro compounds of Formula 27, and bromo compounds of Formula 24, can be prepared by methods known in the art (or by obvious modification of these methods); see, for example, Rorer, M. P. U.S. Patent 4,514,211; Pasteris, R. J. U.S. Patent 4,586,950; Pasteris, R. J. U.S. Patent 4,720,298; Ehrenfreund, J. U.S. Patent 4,634,465; Taylor, E. C. *The Chemistry of Heterocyclic Compounds*; Wiley & Sons; Volume 47, Parts 1 and 2; Raulins, N. R. et. al. *J Het. Chem*. (1968), *5*, 1.

It is recognized that some reagents and reaction conditions described above for preparing compounds of Formula I may not be compatible with certain functionalities present in the intermediates. In these instances, the incorporation of protection/deprotection sequences or functional group interconversions into the synthesis will aid in obtaining the desired products. The use and choice of the protecting groups will be apparent to one skilled in chemical synthesis (see, for example, Greene, T. W.; Wuts, P. G. M. *Protective Groups in Organic Synthesis*, 2nd ed.; Wiley: New York, 1991). One skilled in the art will recognize that, in some cases, after the introduction of a given reagent as it is depicted in any individual scheme, it may be necessary to perform additional routine synthetic steps not described in detail to complete the synthesis of compounds of Formula I. One skilled in the art will also recognize that it may be necessary to perform a combination of the steps illustrated in the above schemes in an order other than that implied by the particular sequence presented to prepare the compounds of Formula I.

One skilled in the art will also recognize that compounds of Formula I and the intermediates described herein can be subjected to various electrophilic, nucleophilic, radical, organometallic, oxidation, and reduction reactions to add substituents or modify existing substituents.

Without further elaboration, it is believed that one skilled in the art using the preceding description can utilize the present invention to its fullest extent. The following Examples are, therefore, to be construed as merely illustrative, and not limiting of the disclosure in any way whatsoever. Percentages are by weight except for chromatographic solvent mixtures or where otherwise indicated. Parts and percentages for chromatographic solvent mixtures are by volume unless otherwise indicated. ¹H NMR spectra are reported in ppm downfield from tetramethylsilane; s = singlet, d = doublet, t = triplet, q = quartet, m = multiplet, dd = doublet of doublets, dt = doublet of triplets, br s = broad singlet.

### EXAMPLE 1

### Step A: Preparation of 4-(2,3-dihydro-2-methyl-7-benzofuranyl)-1,4-dihydro-5H-tetrazol-5-one

12.4 g (0.083 mol) of 7-amino-2,3-dihydro-2-methylbenzofuran (Pilgram, K. H. et al. U.S. Patent 4,554,283) dissolved in 20 mL of ethyl acetate was added dropwise to a solution under a nitrogen atmosphere containing 32.8 g (0.166 mol) of trichloromethyl chloroformate dissolved in 60 mL of ethyl acetate, while maintaining the reaction temperature at about 5 °C with external cooling. After addition was complete, the suspension was refluxed under nitrogen for 5.5 h, then evaporated to dryness under reduced pressure. The residue was azeotroped with ethyl acetate (twice with about 40 mL at 90 °C) to yield 14.5 g of an oil. This oil was added to 19.1 g (0.166 mol) of azidotrimethylsilane (Aldrich). The suspension was refluxed under nitrogen for 24 h, then evaporated to dryness under reduced pressure at about 90 °C to remove excess azidotrimethylsilane. After cooling the residue to 25 °C, 50 mL of methanol was added. The suspension was stirred about 0.25 h and then concentrated under reduced pressure at about 90 °C. The residue was purified by flash column chromatography on silica gel with hexane:ethyl acetate (8:2, then 100% ethyl acetate) as eluant to yield an oil which was crystallized from 1-chlorobutane to yield 9.3 g of the title compound of Step A as a solid melting at 124-127 °C.
¹H NMR (CDCl₃): δ 1.5 (d, 3H), 2.59 (m, 1H), 3.45 (m, 1H), 5.1 (m, 1H), 6.95 (m, 1H), 7.25 (m, 2H).

### Step B: Preparation of 4-(2,3-dihydro-2-methyl-7-benzofuranyl)-N,N-diethyl-4,5-dihydro-5-oxo-1H-tetrazole-1-carboxamide

To a suspension containing 1.5 g (0.0069 mol) of the title compound of Step A in 20 mL of toluene under a nitrogen atmosphere was added 1.01 g (0.0083 mol) of 4-dimethylaminopyridine. After stirring for several minutes 1.1 g (0.0083 mol) of diethylcarbamyl chloride (Aldrich) was added. The suspension was heated to reflux, about 10 mL of acetonitrile was added, and the suspension was refluxed 5 h, then cooled to 25 °C and added to excess water. The aqueous suspension was extracted twice with methylene chloride and the combined organic layers were dried over magnesium sulfate and concentrated under reduced pressure. The solid residue was purified by flash column chromatography on silica gel with hexane:ethyl acetate (8.5:1.5, then 1:1) as eluant to yield 1.2 g of the title compound of Step B, a compound of this invention, as a solid melting at 124-126 °C.
¹H NMR (CDCl₃): δ 1.3 (t, 6H), 1.5 (d, 3H), 2.9 (m, 1H), 3.4 (m, 1H), 3.45 (m, 2H), 3.55 (m, 2H), 5.05 (m, 1H), 6.9 (m, 1H), 7.25 (m, 2H).

### EXAMPLE 2

### Step A: Preparation of tetrahydro-N-(1-methylethyl)-4H-pyran-4-imine

To a dry flask under a nitrogen atmosphere was added 2.5 g of tetrahydro-4*H*-pyran-4-one followed by 4.5 mL of isopropylamine. The reaction mixture was stirred at room temperature for 10 minutes followed by the addition of 1.8 g of powdered KOH. After stirring overnight at room temperature, 20 mL of anhydrous benzene was added and the reaction mixture was filtered over a thin pad of Celite®. The Celite® pad was washed with an additional 20 mL of benzene. The filtrate was concentrated under reduced pressure to give 3.1 g of the title compound of Step A as a liquid which was used as a starting material in the next step without further purification.

### Step B: Preparation of (5,6-dihydro-2H-pyran-4-yl)(1-methylethyl)carbamic chloride

To a dry flask under a nitrogen atmosphere was added 3 g of the title compound of Step A, followed by 3.24 mL of triethylamine and 30 mL of anhydrous benzene. The flask was cooled to 0 °C and 11.6 mL of a 20% solution of phosgene in toluene was added over a period of 10 minutes. After stirring at room temperature for 90 minutes, the reaction mixture was partitioned between benzene/ethyl acetate and water. The benzene/ethyl acetate layer was successively washed with saturated aqueous ammonium chloride and brine, and was then dried over sodium sulfate and concentrated to yield 4.1 g of the title compound of Step B as a liquid (solidified on standing) which was used as a starting material in the next step without further purification.
¹H NMR (CDCl₃): δ 1.2 (d, 6H), 2.3 (br s, 2H), 3.86 (br s, 2H), 4.28 (m, 2H), 4.38 (m, 1H), 5.71 (s, 1H).

### Step C: Preparation of (+/-)-4-(2,3-dihydro-2-methyl-7-benzofuranyl)-N-(5,6-dihydro-2H-pyran-4-yl)-4,5-dihydro-N-(1-methylethyl)-5-oxo-1H-tetrazole-1-carboxamide

To a suspension containing 0.6 g (0.00275 mol) of the title compound of Step A in Example 1 and 0.56 (0.0075 mol) of the title compound of Step B in Example 2 in 10 mL of toluene under nitrogen atmosphere was added 0.335 g (0.00275 mol) of 4-dimethylamino-pyridine. The suspension was refluxed 1.5 h then concentrated under reduced pressure. The residue was partitioned between a solution of aqueous ammonium chloride and ethyl acetate (twice with 30 mL), and the organic layer was separated, dried over magnesium sulfate and concentrated under reduced pressure. The residue was purified by flash column chromatography on silica gel using hexane:ethyl acetate (4:1 then 3.5:1.5) as eluant to yield 0.75 g of the title compound of Step C in Example 2, a compound of this invention, as an oil.
¹H NMR (CDCl₃): δ 1.4 (d, 6H), 1.5 (d, 3H), 2.35 (m, 2H), 2.9 (m, 1H), 3.4 (m, 1H), 3.8 (m, 2H), 4.15 (m, 2H), 4.45 (m, 1H), 5.05 (m, 1H), 5.7 (m, 1H), 6.9 (m, 1H), 7.2 (m, 1H), 7.25 (m, 1H).

### EXAMPLE 3

### Step A: Preparation of N-(1-methylethylidene)-1-piperidinamine

5 mL of acetone was slowly added to 5 g (0.05 mol) of 1-aminopiperidine and the mixture was stirred at 25 °C for 2 h, then concentrated under reduced pressure to yield 6.5 g of the title compound of Step A as an oil which was used in the next step without further purification.

### Step B: Preparation of N-(1-methylethyl)-1-piperidinamine

To a suspension containing 3.35 g (0.088 mol) of lithium aluminum hydride in 160 mL of diethyl ether, cooled at 0 °C, was added dropwise a solution containing 10.99 g (0.0785 mol) of the title compound of Step A in 60 mL of diethyl ether. After the exotherm slowed the suspension was refluxed for 3.5 h and then cooled to 0 °C. Water was carefully added dropwise under a nitrogen atmosphere until bubbling ceased. An additional 14 mL of water was added followed by 3.4 mL of 15% aqueous sodium hydroxide. After stirring at 25 °C for 1 h, the suspension was filtered through Celite® with diethyl ether as eluant. The organic layer was separated and concentrated under reduced pressure. A solution containing the residue in dichloromethane was dried over sodium sulfate and concentrated under reduced pressure to yield the title compound of Step B as an oil which was used in the next step without further purification.

### Step C: Preparation of (1-methylethyl)(1-piperidinyl)carbamic chloride

To a solution containing 1.99 g (0.014 mol) of the title compound of Step B in 20 mL of dichloromethane, cooled at 0 °C, was added dropwise 10 mL (0.035 mol) of a 3.5*M* solution of phosgene in toluene followed by 3.8 mL (0.027 mol) of triethylamine. The cooling bath was removed, the suspension was stirred at 25 °C for 10 min, and then poured into 40 mL of saturated aqueous sodium bicarbonate solution. The suspension was extracted with dichloromethane (three times with 40 mL) and the combined extracts were dried over sodium sulfate and concentrated under reduced pressure to yield the title compound of Step C as an oil which was used in the next step without further purification.

### Step D: Preparation of 4-(2,3-dihydro-2-methyl-7-benzofuranyl)-4,5-dihydro-N-(1-methylethyl)-5-oxo-N-(1-piperidinyl)-1H-tetrazole-1-carboxamide

A suspension containing 0.845 g (0.00386 mol) of the title compound of Step A in Example 1, 1.57 g (0.00767 mol) of the title compound in Step C of Example 3 and 0.81 g (0.00663 mol) of 4-dimethylaminopyridine in 15 mL of toluene was refluxed for 0.5 h, then cooled to 25 °C, and poured into 30 mL of 1*N* aqueous hydrochloric acid. The suspension was extracted with ethyl acetate (three times with 30 mL) and the extracts were combined, dried over sodium sulfate and concentrated under reduced pressure. The residue was purified by flash column chromatography on silica gel with hexane:ethyl acetate (8:2, then 7:3, then 6:4) as eluant to yield 0.73 g of the title compound of Step D, a compound of this invention, as a solid melting at 153-154 °C.
¹H NMR (CDCl₃): δ 1.45-1.7 (m, 15H), 2.65 (m, 2H), 2.9 (m, 1H), 3.05 (m, 2H), 3.4 (m, 1H), 3.9 (m, 1H), 5.05 (m, 1H), 6.9 (m, 1H), 7.25 (m, 2H).

By the procedures described herein together with methods known in the art, the following compounds of Tables 1 to 6 can be prepared. The following notations have been used in Tables 1-6.

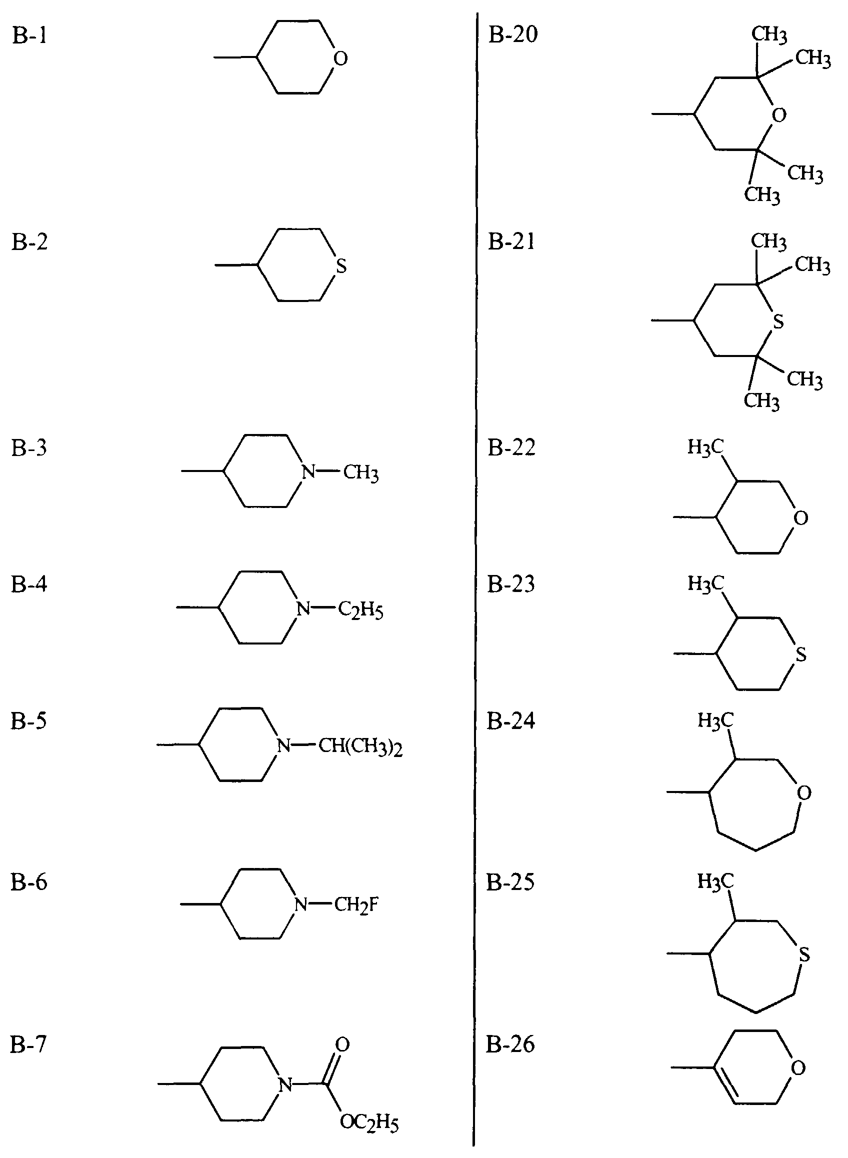

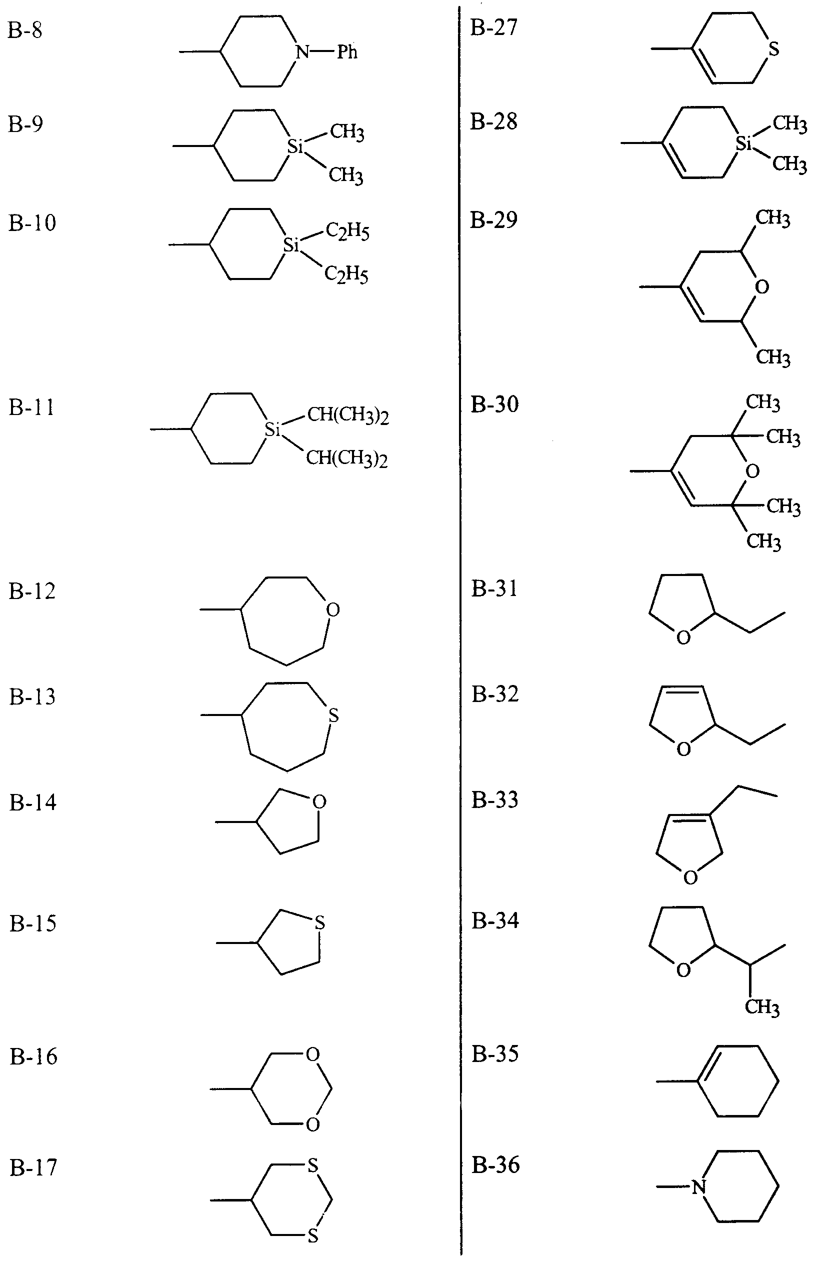

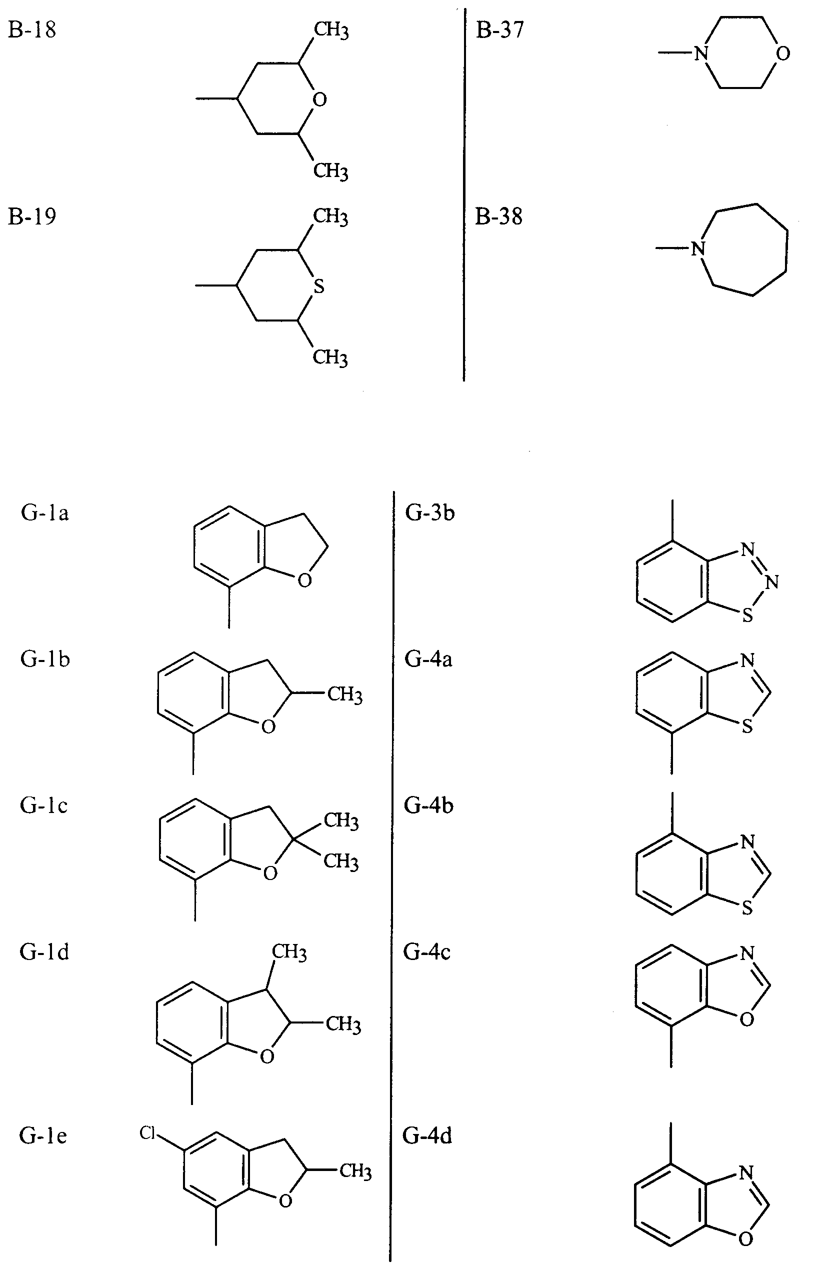

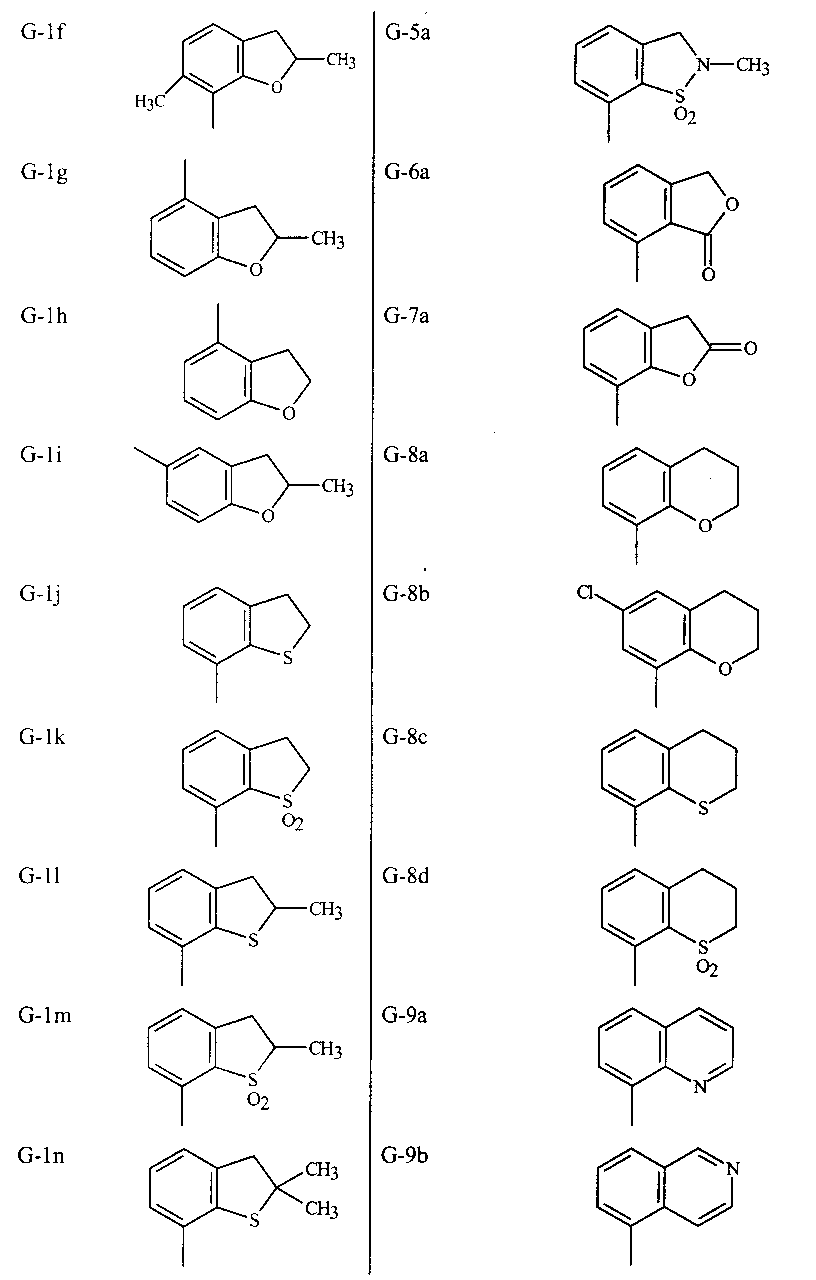

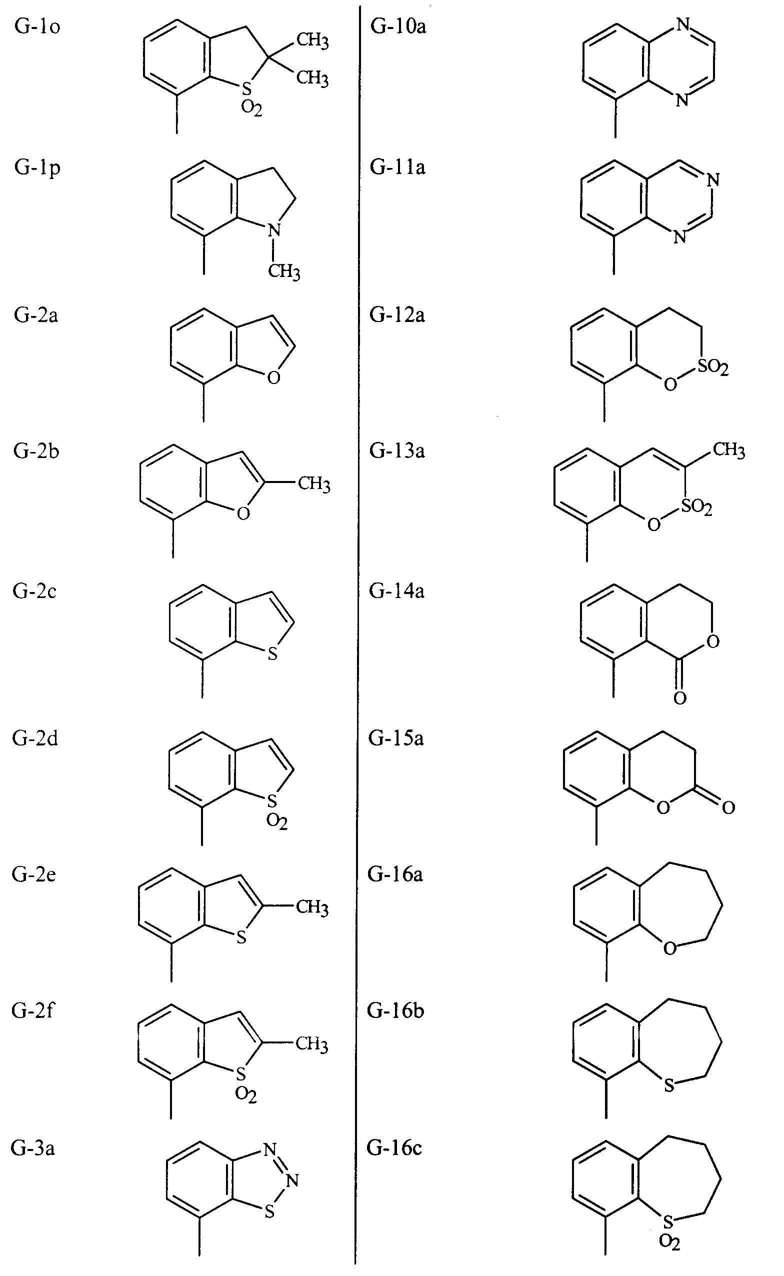

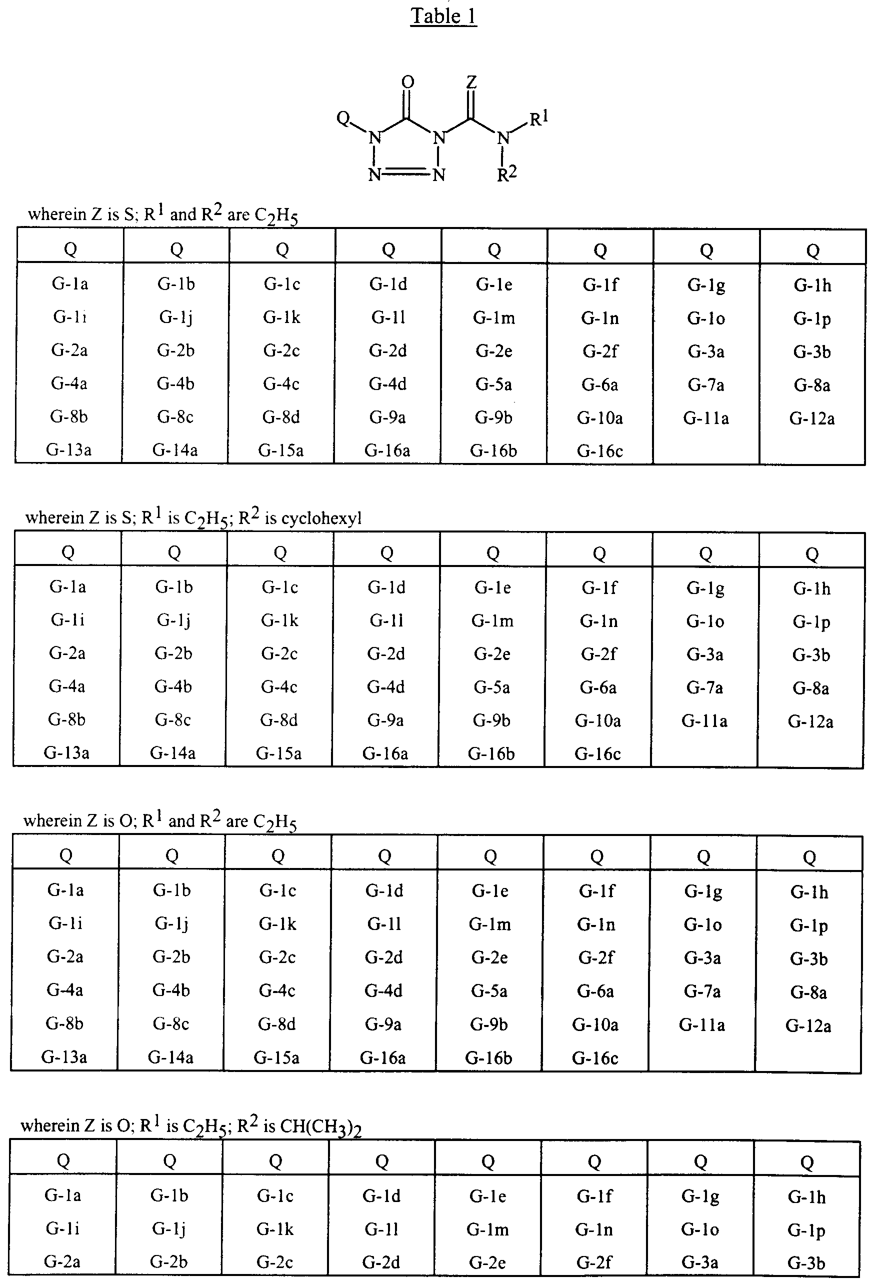

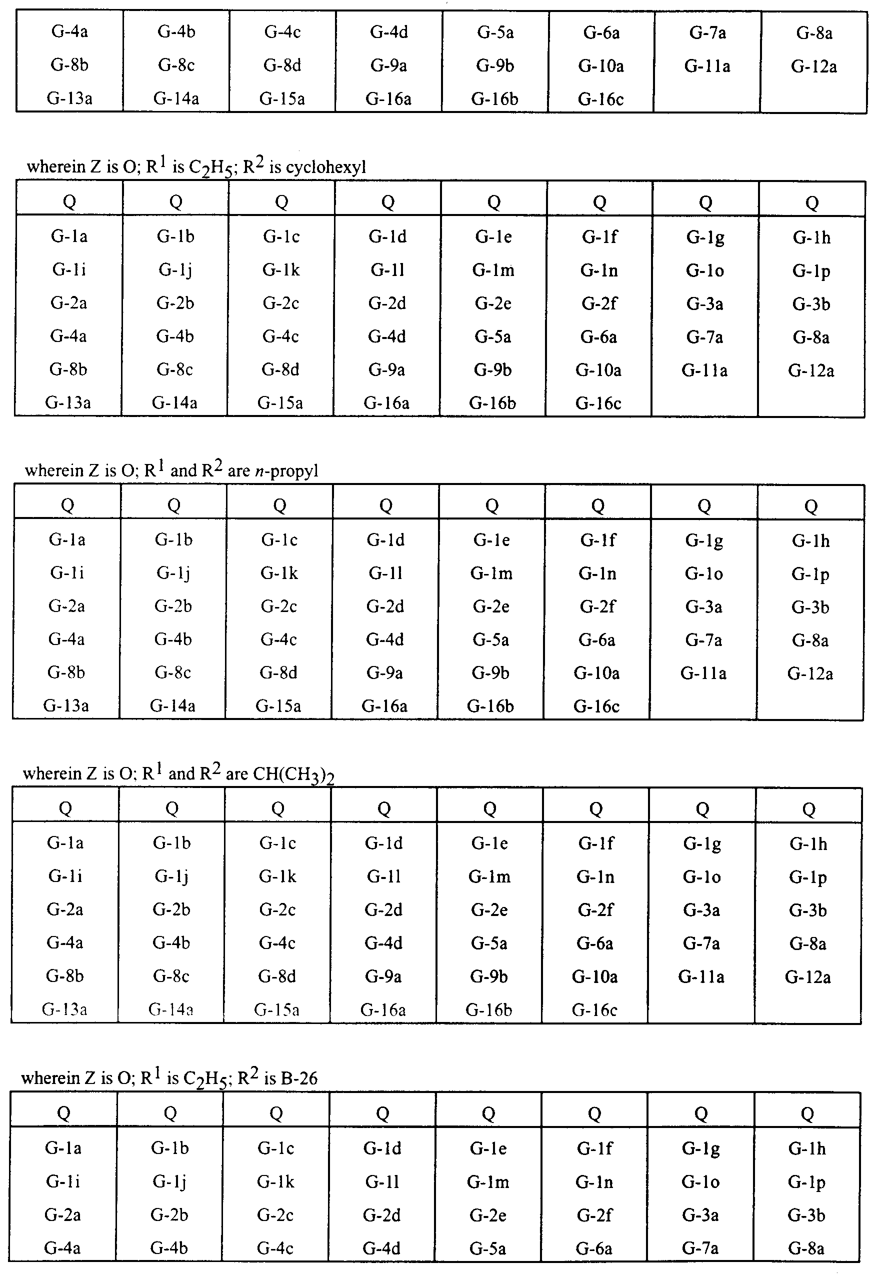

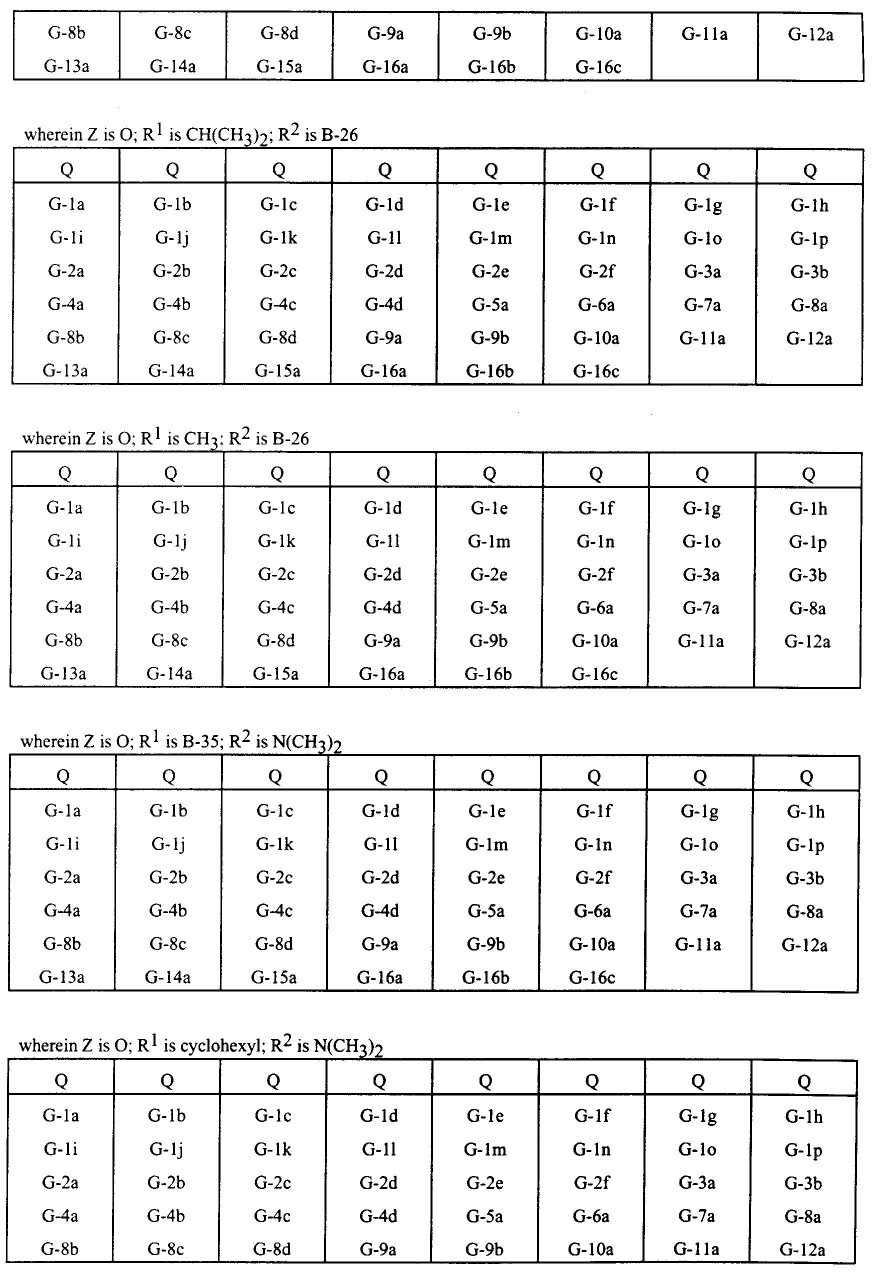

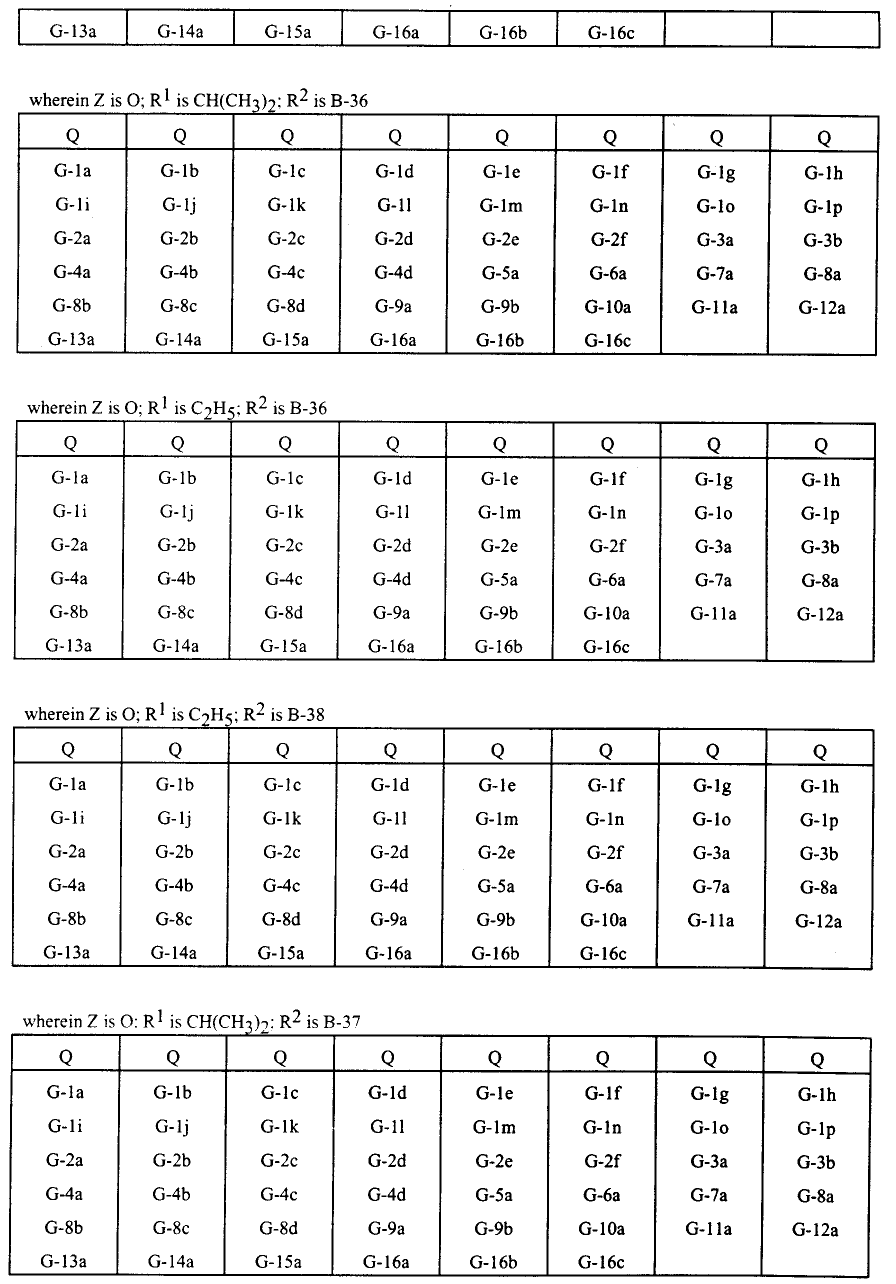

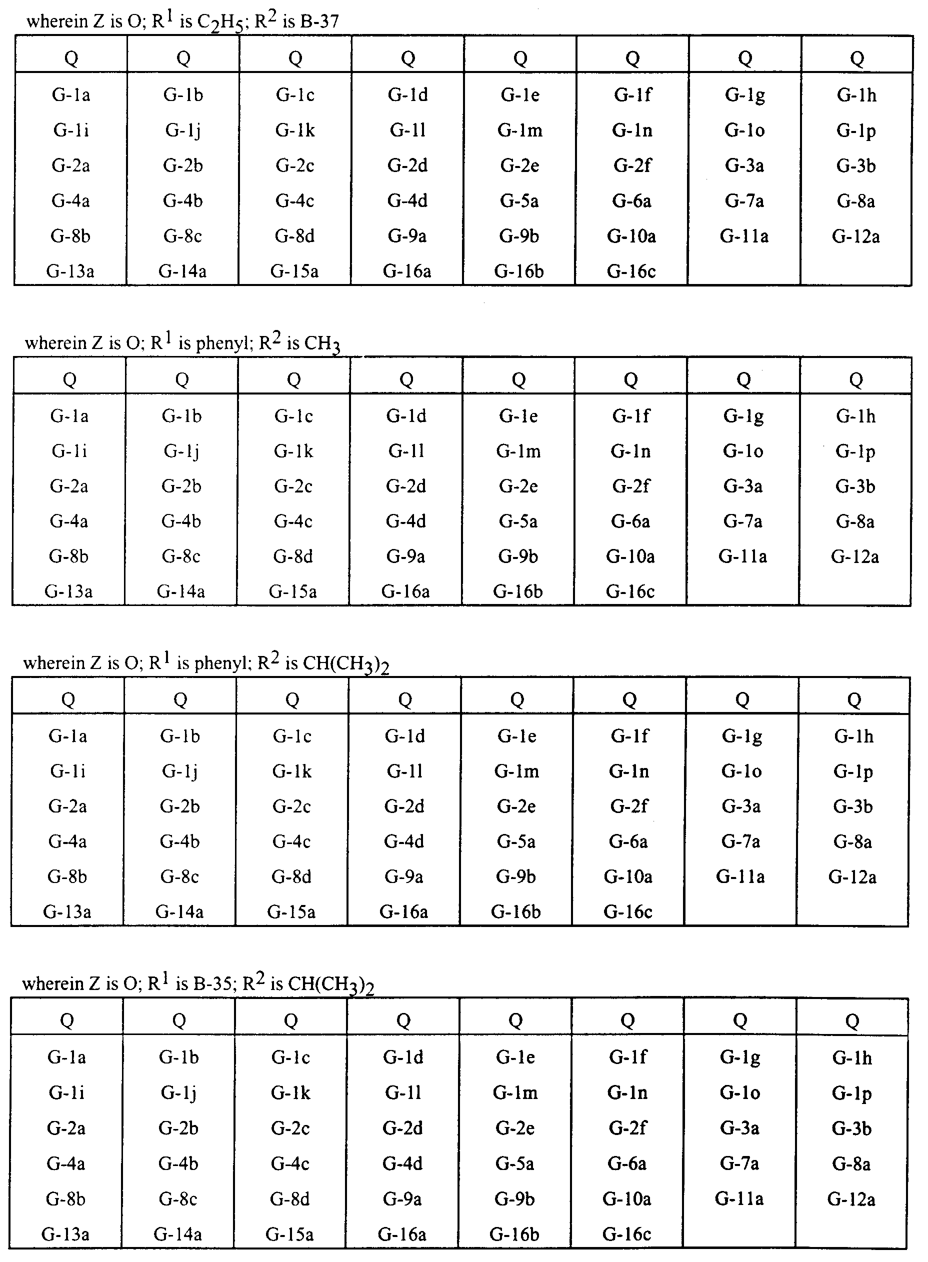

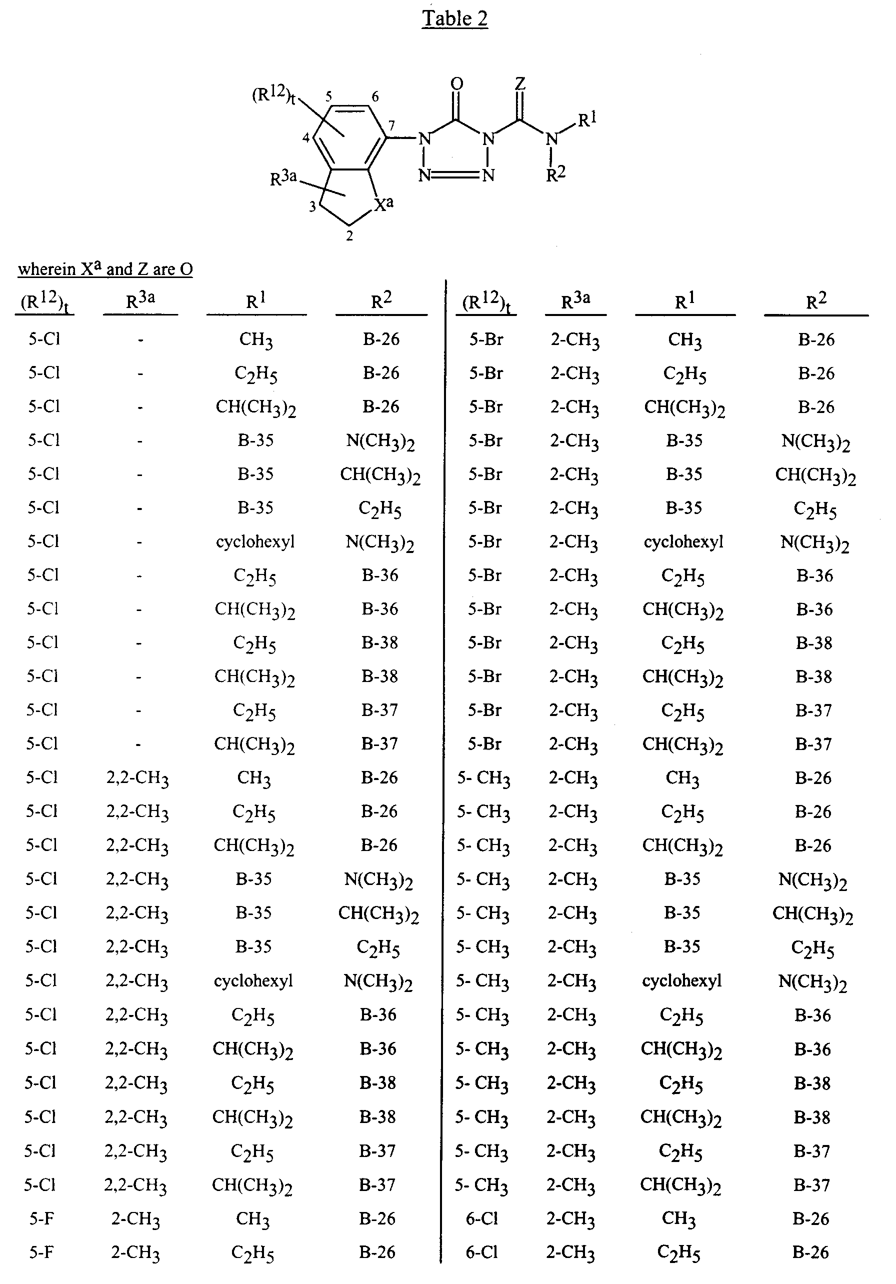

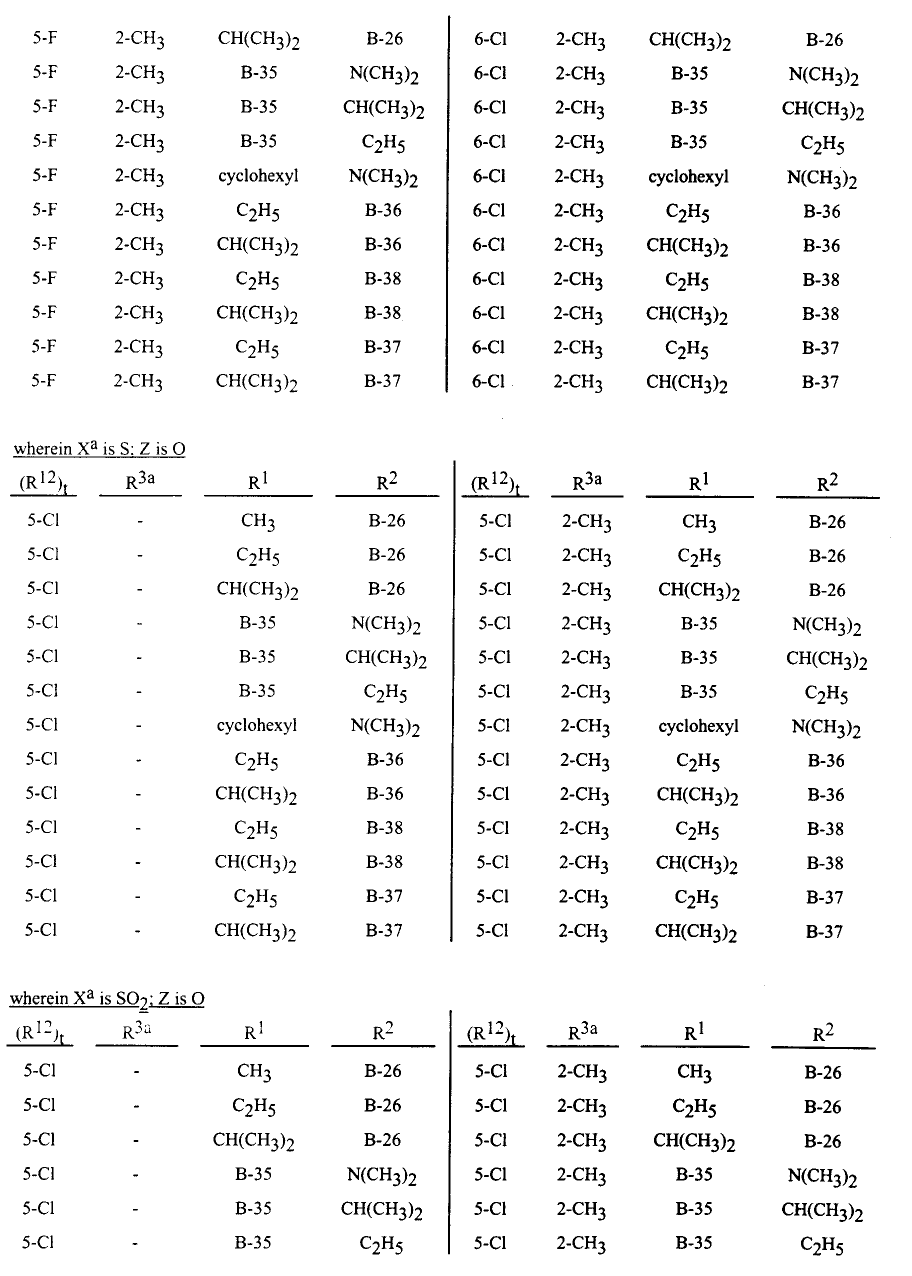

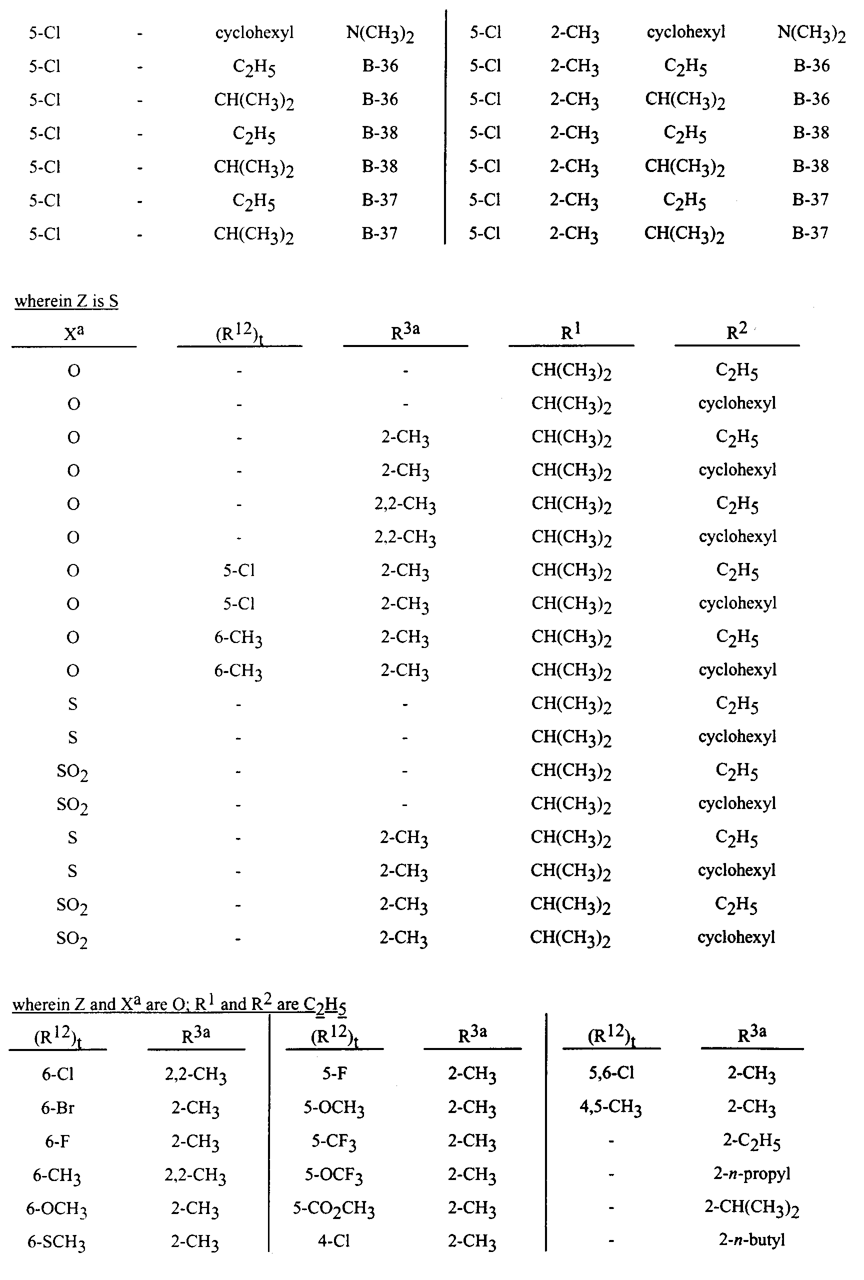

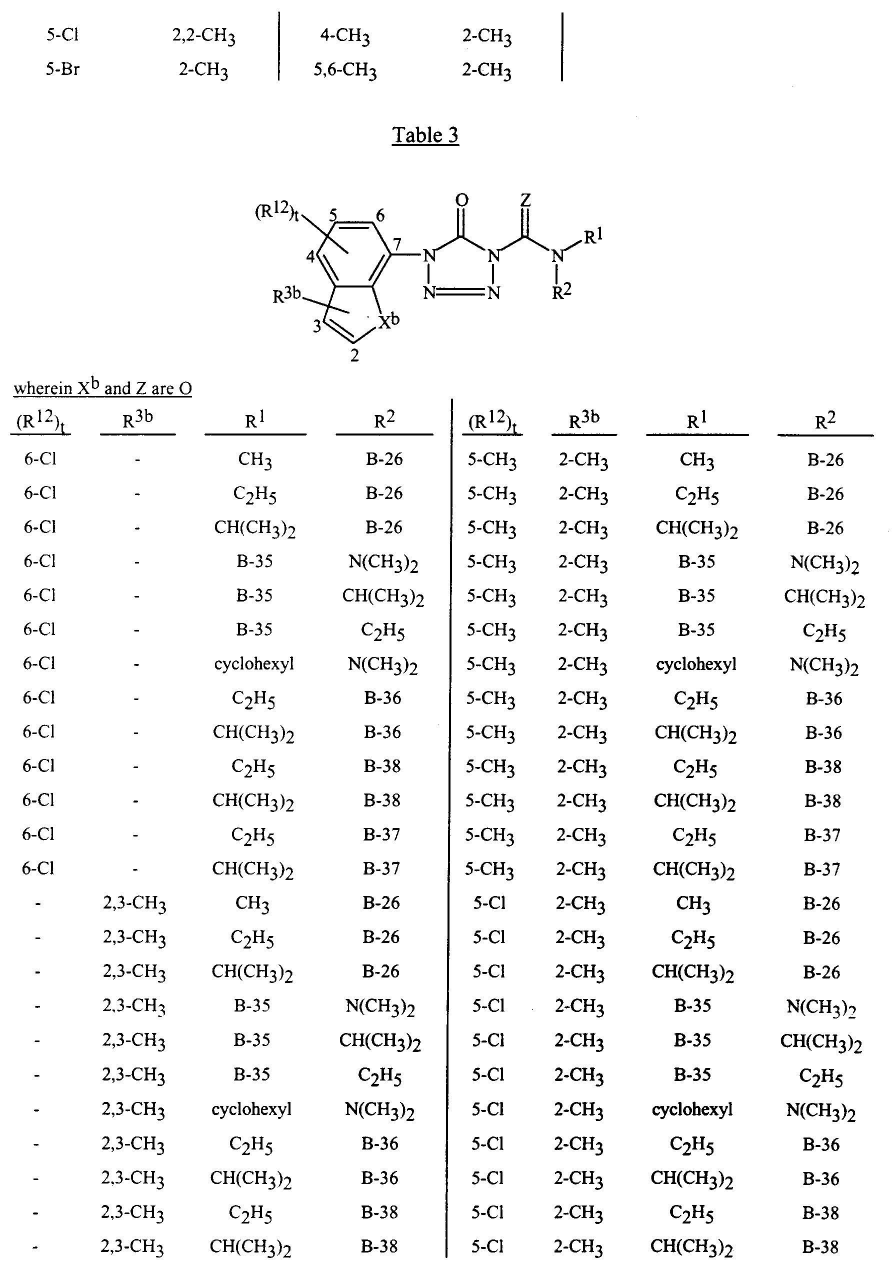

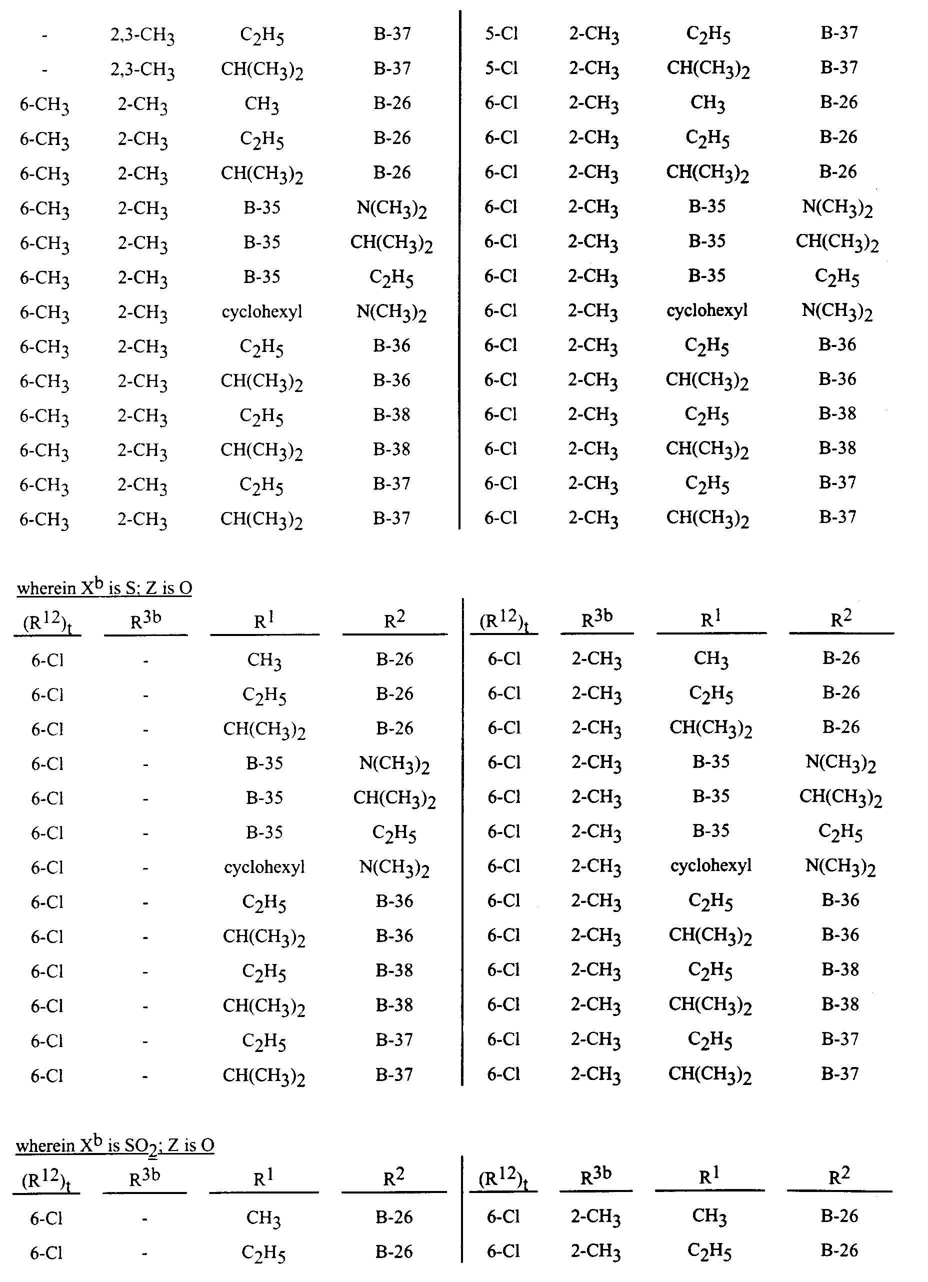

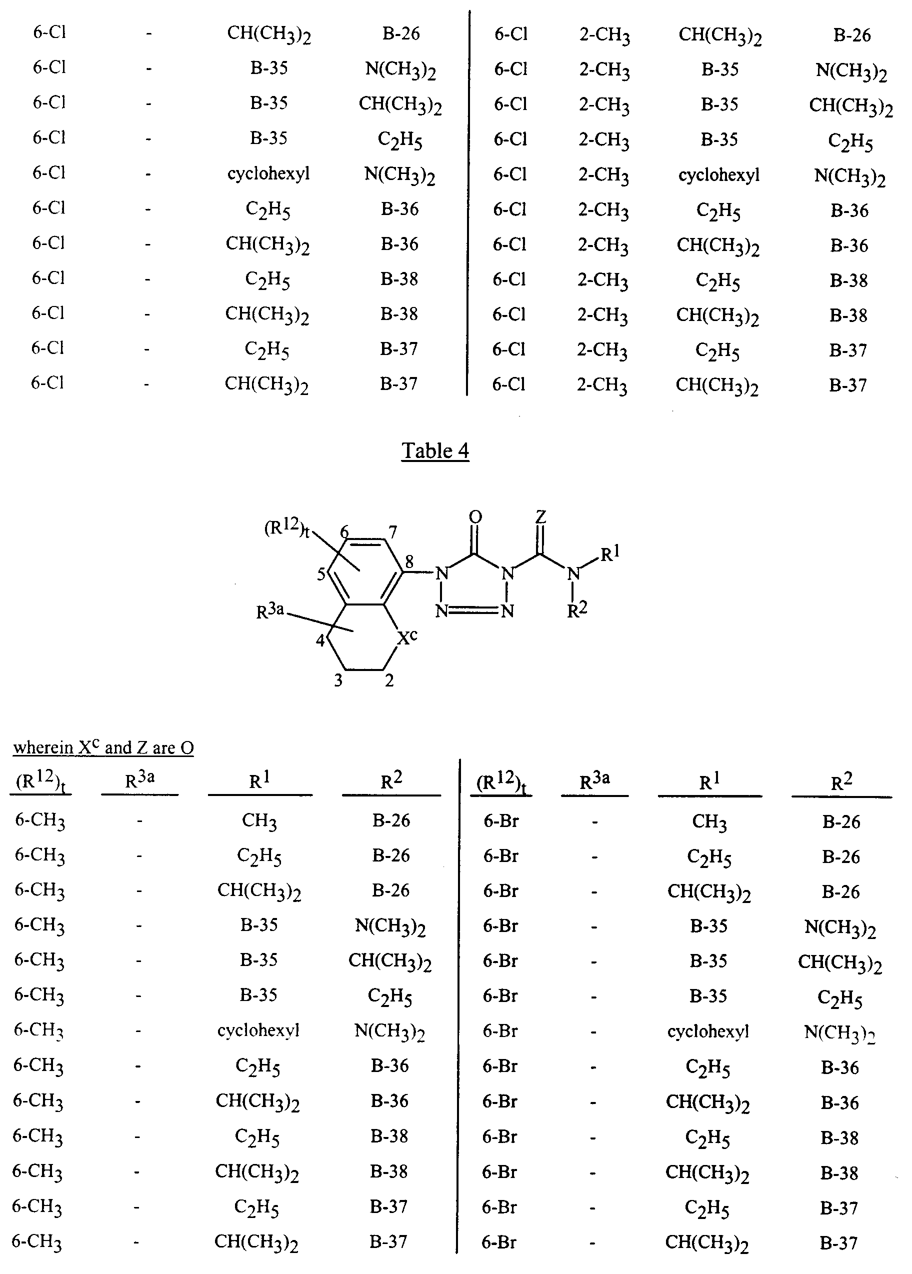

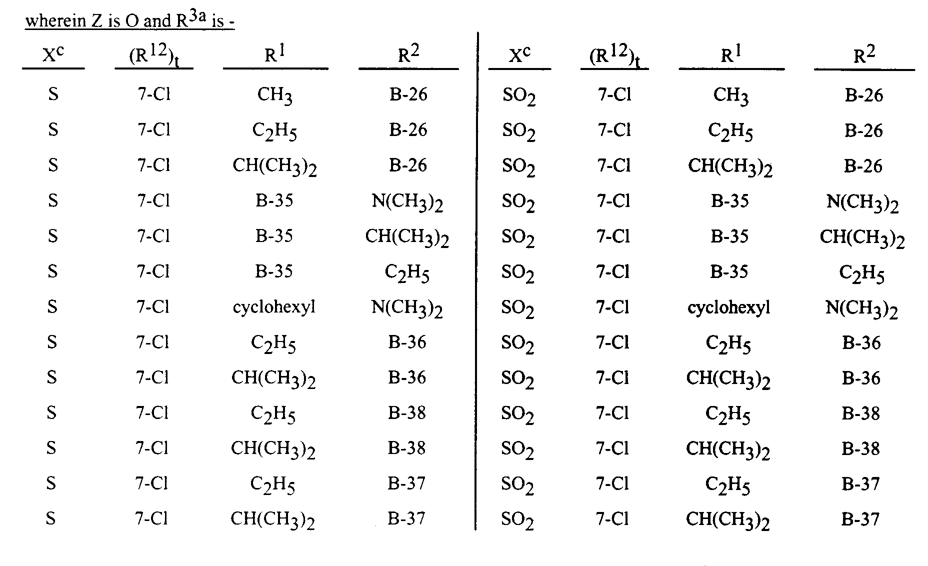

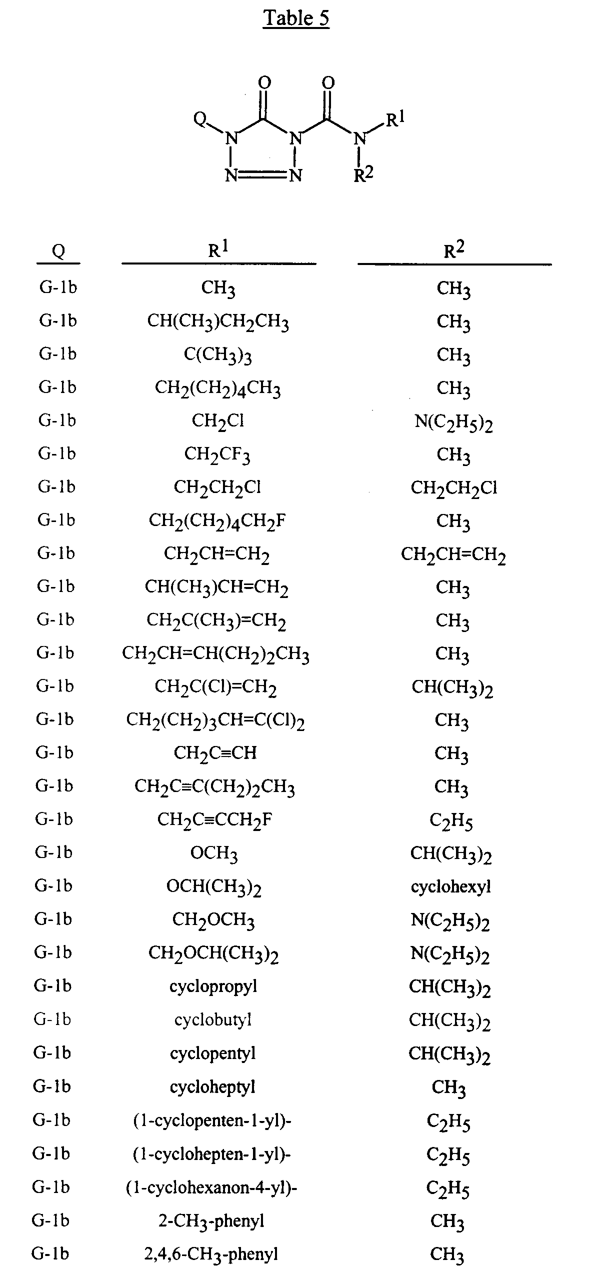

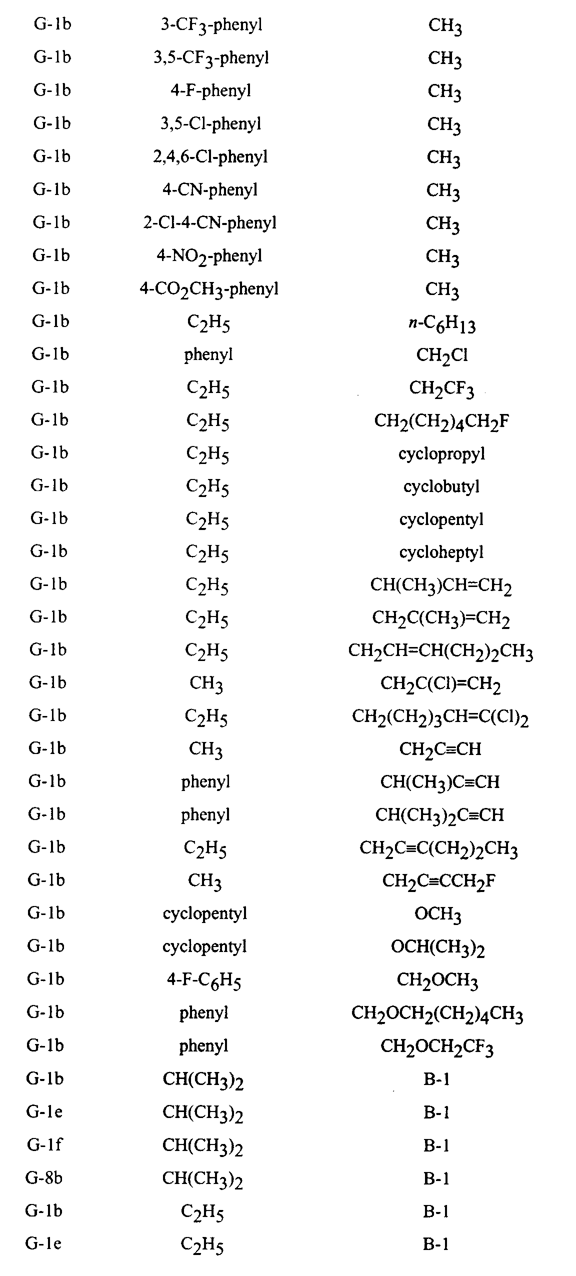

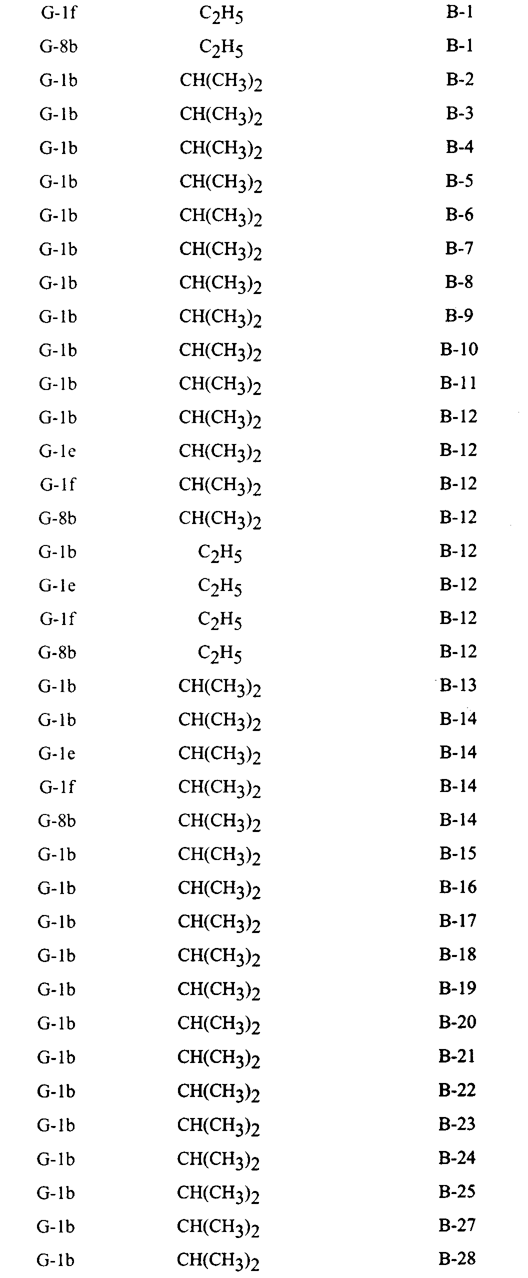

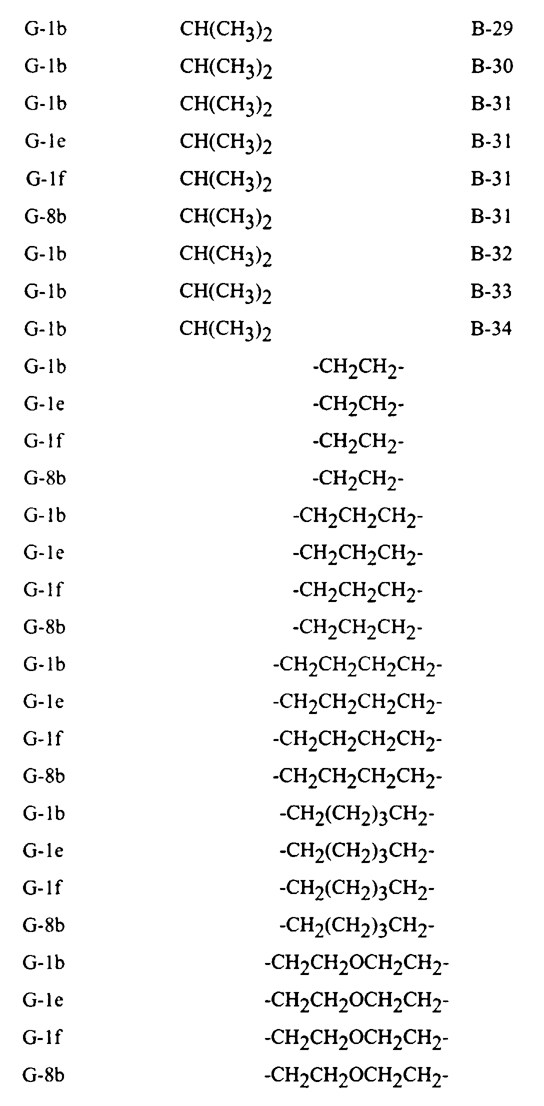

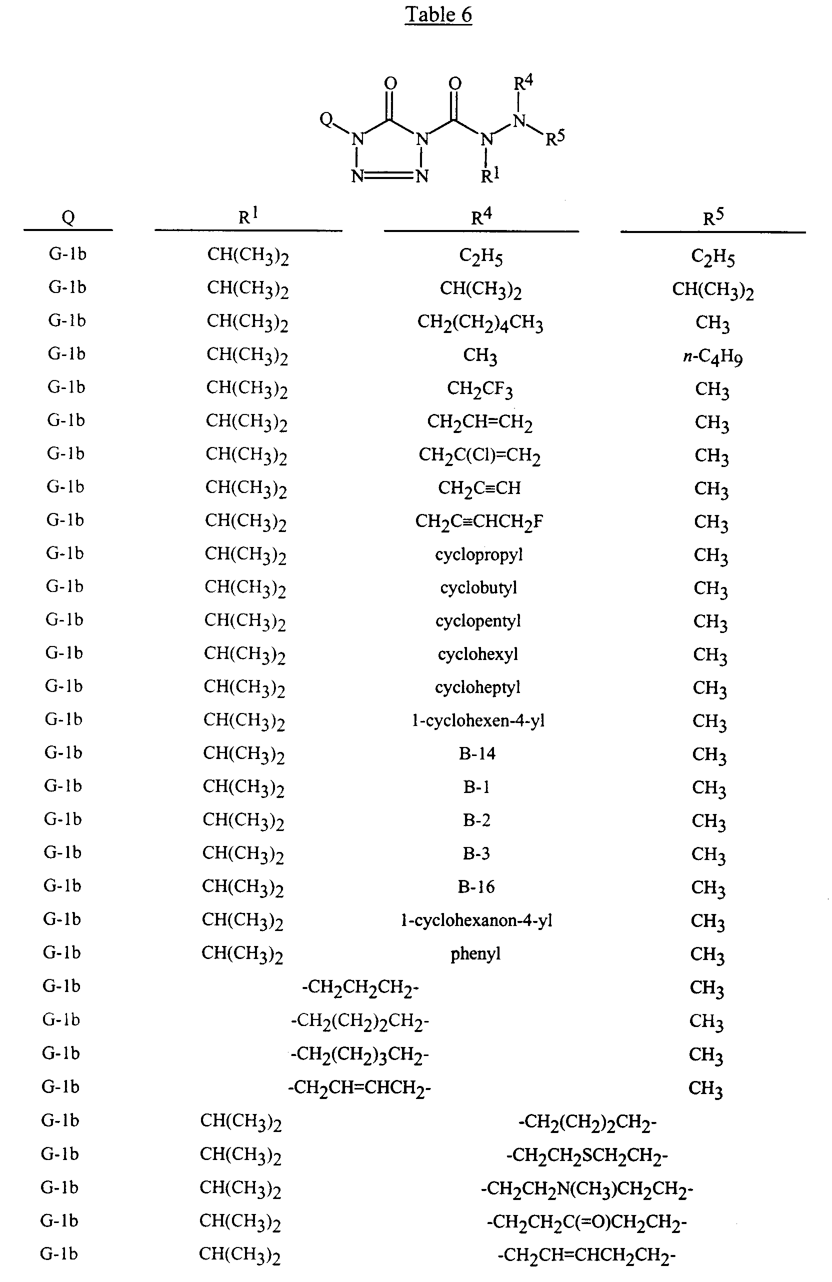

### Formulation/Utility

Compounds of this invention will generally be used as a formulation or composition with an agriculturally suitable carrier comprising at least one of a liquid diluent, a solid diluent or a surfactant. The formulation or composition ingredients are selected to be consistent with the physical properties of the active ingredient, mode of application and environmental factors such as soil type, moisture and temperature. Useful formulations include liquids such as solutions (including emulsifiable concentrates), suspensions, emulsions (including microemulsions and/or suspoemulsions) and the like which optionally can be thickened into gels. Useful formulations further include solids such as dusts, powders, granules, pellets, tablets, films, and the like which can be water-dispersible ("wettable") or water-soluble. Active ingredient can be (micro)encapsulated and further formed into a suspension or solid formulation; alternatively the entire formulation of active ingredient can be encapsulated (or "overcoated"). Encapsulation can control or delay release of the active ingredient. Sprayable formulations can be extended in suitable media and used at spray volumes from about one to several hundred liters per hectare. High-strength compositions are primarily used as intermediates for further formulation.

The formulations will typically contain effective amounts of active ingredient, diluent and surfactant within the following approximate ranges which add up to 100 percent by weight.

| | | Weight Percent | |
|---|---|---|---|
| | Active Ingredient | Diluent | Surfactant |
| Water-Dispersible and Water-soluble Granules, Tablets and Powders. | 5-90 | 0-94 | 1-15 |
| Suspensions, Emulsions, Solutions (including Emulsifiable Concentrates) | 5-50 | 40-95 | 0-15 |
| Dusts | 1-25 | 70-99 | 0-5 |
| Granules and Pellets | 0.01-99 | 5-99.99 | 0-15 |
| High Strength Compositions | 90-99 | 0-10 | 0-2 |

Typical solid diluents are described in Watkins, et al., *Handbook of Insecticide Dust Diluents and Carriers*, 2nd Ed., Dorland Books, Caldwell, New Jersey. Typical liquid diluents are described in Marsden, *Solvents Guide*, 2nd Ed., Interscience, New York, 1950. *McCutcheon's Detergents and Emulsifiers Annual*, Allured Publ. Corp., Ridgewood, New Jersey, as well as Sisely and Wood, *Encyclopedia of Surface Active Agents*, Chemical Publ. Co., Inc., New York, 1964, list surfactants and recommended uses. All formulations can contain minor amounts of additives to reduce foam, caking, corrosion, microbiological growth and the like, or thickeners to increase viscosity.

Surfactants include, for example, polyethoxylated alcohols, polyethoxylated alkylphenols, polyethoxylated sorbitan fatty acid esters, dialkyl sulfosuccinates, alkyl sulfates, alkylbenzene sulfonates, organosilicones, *N,N*-dialkyltaurates, lignin sulfonates, naphthalene sulfonate formaldehyde condensates, polycarboxylates, and polyoxyethylene/polyoxypropylene block copolymers. Solid diluents include, for example, clays such as bentonite, montmorillonite, attapulgite and kaolin, starch, sugar, silica, talc, diatomaceous earth, urea, calcium carbonate, sodium carbonate and bicarbonate, and sodium sulfate. Liquid diluents include, for example, water, *N,N*-dimethylformamide, dimethyl sulfoxide, *N*-alkylpyrrolidone, ethylene glycol, polypropylene glycol, paraffins, alkylbenzenes, alkylnaphthalenes, oils of olive, castor, linseed, tung, sesame, corn, peanut, cotton-seed, soybean, rape-seed and coconut, fatty acid esters, ketones such as cyclohexanone, 2-heptanone, isophorone and 4-hydroxy-4-methyl-2-pentanone, and alcohols such as methanol, cyclohexanol, decanol and tetrahydrofurfuryl alcohol.

Solutions, including emulsifiable concentrates, can be prepared by simply mixing the ingredients. Dusts and powders can be prepared by blending and, usually, grinding as in a hammer mill or fluid-energy mill. Suspensions are usually prepared by wet-milling; see, for example, U.S. 3,060,084. Granules and pellets can be prepared by spraying the active material upon preformed granular carriers or by agglomeration techniques. See Browning, "Agglomeration", *Chemical Engineering*, December 4, 1967, pp 147-48, *Perry's Chemical Engineer's Handbook*, 4th Ed., McGraw-Hill, New York, 1963, pages 8-57 and following, and WO 91/13546. Pellets can be prepared as described in U.S. 4,172,714. Water-dispersible and water-soluble granules can be prepared as taught in U.S. 4,144,050, U.S. 3,920,442 and DE 3,246,493. Tablets can be prepared as taught in U.S. 5,180,587, U.S. 5,232,701 and U.S. 5,208,030. Films can be prepared as taught in GB 2,095,558 and U.S. 3,299,566.

For further information regarding the art of formulation, see U.S. 3,235,361, Col. 6, line 16 through Col. 7, line 19 and Examples 10-41; U.S. 3,309,192, Col. 5, line 43 through Col. 7, line 62 and Examples 8, 12, 15, 39, 41, 52, 53, 58, 132, 138-140, 162-164, 166, 167 and 169-182; U.S. 2,891,855, Col. 3, line 66 through Col. 5, line 17 and Examples 1-4; Klingman, *Weed Control as a Science*, John Wiley and Sons, Inc., New York, 1961, pp 81-96; and Hance et al., *Weed Control Handbook*, 8th Ed., Blackwell Scientific Publications, Oxford, 1989.

In the following Examples, all percentages are by weight and all formulations are prepared in conventional ways. Compound numbers refer to compounds in Index Tables A-E.

### Example A

| High Strength Concentrate | |
|---|---|
| Compound 33 | 98.5% |
| silica aerogel | 0.5% |
| synthetic amorphous fine silica | 1.0%. |

### Example B

| Wettable Powder | |
|---|---|
| Compound 33 | 65.0% |
| dodecylphenol polyethylene glycol ether | 2.0% |
| sodium ligninsulfonate | 4.0% |
| sodium silicoaluminate | 6.0% |
| montmorillonite (calcined) | 23.0%. |

### Example C

| Granule | |
|---|---|
| Compound 33 | 10.0% |
| attapulgite granules (low volatile matter, | |
| 0.71/0.30 mm; U.S.S. No. 25-50 sieves) | 90.0%. |

### Example D

| Extruded Pellet | |
|---|---|
| Compound 33 | 25.0% |
| anhydrous sodium sulfate | 10.0% |
| crude calcium ligninsulfonate | 5.0% |
| sodium alkylnaphthalenesulfonate | 1.0% |
| calcium/magnesium bentonite | 59.0%. |

Test results indicate that the compounds of the present invention are highly active preemergent and postemergent herbicides or plant growth regulants. Many of them have utility for broad-spectrum pre- and/or postemergence weed control in areas where complete control of all vegetation is desired such as around fuel storage tanks, industrial storage areas, parking lots, drive-in theaters, air fields, river banks, irrigation and other waterways, around billboards and highway and railroad structures. Some of the compounds are useful for the control of selected grass and broadleaf weeds with tolerance to important agronomic crops which include but are not limited to alfalfa, barley, cotton, wheat, rape, sugar beets, corn (maize), sorghum, soybeans, rice, oats, peanuts, vegetables, tomato, potato, perennial plantation crops including coffee, cocoa, oil palm, rubber, sugarcane, citrus, grapes, fruit trees, nut trees, banana, plantain, pineapple, hops, tea and forests such as eucalyptus and conifers (e.g., loblolly pine), and turf species (e.g., Kentucky bluegrass, St. Augustine grass, Kentucky fescue and Bermuda grass). Those skilled in the art will appreciate that not all compounds are equally effective against all weeds. Alternatively, the subject compounds are useful to modify plant growth.

A herbicidally effective amount of the compounds of this invention is determined by a number of factors. These factors include: formulation selected, method of application, amount and type of vegetation present, growing conditions, etc. In general, a herbicidally effective amount of compounds of this invention is 0.001 to 20 kg/ha with a preferred range of 0.004 to 1.0 kg/ha. One skilled in the art can easily determine the herbicidally effective amount necessary for the desired level of weed control.

Compounds of this invention can be used alone or in combination with other commercial herbicides, insecticides or fungicides. Compounds of this invention can also be used in combination with commercial herbicide safeners such as benoxacor, dichlormid and furilazole to increase safety to certain crops. A mixture of one or more of the following herbicides with a compound of this invention may be particularly useful for weed control: acetochlor, acifluorfen and its sodium salt, aclonifen, acrolein (2-propenal), alachlor, ametryn, amidosulfuron, amitrole, ammonium sulfamate, anilofos, asulam, atrazine, azafenidin, azimsulfuron, benazolin, benazolin-ethyl, benfluralin, benfuresate, bensulfuron-methyl, bensulide, bentazone, bifenox, bispyribac and its sodium salt, bromacil, bromoxynil, bromoxynil octanoate, butachlor, butralin, butroxydim (ICIA0500), butylate, caloxydim (BAS 620H), carfentrazone-ethyl, chlomethoxyfen, chloramben, chlorbromuron, chloridazon, chlorimuron-ethyl, chlornitrofen, chlorotoluron, chlorpropham, chlorsulfuron, chlorthal-dimethyl, cinmethylin, cinosulfuron, clethodim, clomazone, clopyralid, clopyralid-olamine, cyanazine, cycloate, cyclosulfamuron, 2,4-D and its butotyl, butyl, isoctyl and isopropyl esters and its dimethylammonium, diolamine and trolamine salts, daimuron, dalapon, dalapon-sodium, dazomet, 2,4-DB and its dimethylammonium, potassium and sodium salts, desmedipham, desmetryn, dicamba and its diglycolammonium, dimethylammonium, potassium and sodium salts, dichlobenil, dichlorprop, diclofop-methyl, 2-[4,5-dihydro-4-methyl-4-(1-methylethyl)-5-oxo-1*H*-imidazol-2-yl]-5-methyl-3-pyridinecarboxylic acid (AC 263,222), difenzoquat metilsulfate, diflufenican, dimepiperate, dimethenamid, dimethylarsinic acid and its sodium salt, dinitramine, diphenamid, diquat dibromide, dithiopyr, diuron, DNOC, endothal, EPTC, esprocarb, ethalfluralin, ethametsulfuron-methyl, ethofumesate, ethoxysulfuron, fenoxaprop-ethyl, fenoxaprop-P-ethyl, fenuron, fenuron-TCA, flamprop-methyl, flamprop-M-isopropyl, flamprop-M-methyl, flazasulfuron, fluazifop-butyl, fluazifop-P-butyl, fluchloralin, flumetsulam, flumiclorac-pentyl, flumioxazin, fluometuron, fluoroglycofen-ethyl, flupoxam, flupyrsulfuron-methyl and its sodium salt, fluridone, flurochloridone, fluroxypyr, fluthiacet-methyl, fomesafen, fosamine-ammonium, glufosinate, glufosinate-ammonium, glyphosate, glyphosate-isopropylammonium, glyphosate-sesquisodium, glyphosate-trimesium, halosulfuron-methyl, haloxyfop-etotyl, haloxyfop-methyl, hexazinone, imazamethabenz-methyl, imazamox, imazapyr, imazaquin, imazaquin-ammonium, imazethapyr, imazethapyr-ammonium, imazosulfuron, ioxynil, ioxynil octanoate, ioxynil-sodium, isoproturon, isouron, isoxaben, isoxaflutole, lactofen, lenacil, linuron, maleic hydrazide, MCPA and its dimethylammonium, potassium and sodium salts, MCPA-isoctyl, mecoprop, mecoprop-P, mefenacet, mefluidide, metam-sodium, methabenzthiazuron, methylarsonic acid and its calcium, monoammonium, monosodium and disodium salts, methyl [[[1-[5-[2-chloro-4-(trifluoromethyl)phenoxy]-2-nitrophenyl]-2 methoxyethylidene]amino]oxy]acetate (AKH-7088), methyl 5-[[[[(4,6-dimethyl-2-pyrimidinyl)amino]carbonyl]amino]sulfonyl]-1-(2-pyridinyl)-1*H*-pyrazole-4-carboxylate (NC-330), metobenzuron, metolachlor, metosulam, metoxuron, metribuzin, metsulfuron-methyl, molinate, monolinuron, napropamide, naptalam, neburon, nicosulfuron, norflurazon, oryzalin, oxadiazon, oxasulfuron, oxyfluorfen, paraquat dichloride, pebulate, pendimethalin, pentoxazone (KPP-314), perfluidone, phenmedipham, picloram, picloram-potassium, pretilachlor, primisulfuron-methyl, prometon, prometryn, propachlor, propanil, propaquizafop, propazine, propham, propyzamide, prosulfuron, pyrazolynate, pyrazosulfuron-ethyl, pyridate, pyriminobac-methyl, pyrithiobac, pyrithiobac-sodium, quinclorac, quizalofop-ethyl, quizalofop-P-ethyl, quizalofop-P-tefuryl, rimsulfuron, sethoxydim, siduron, simazine, sulcotrione (ICIA0051), sulfentrazone, sulfometuron-methyl, TCA, TCA-sodium, tebuthiuron, terbacil, terbuthylazine, terbutryn, thenylchlor, thiafluamide (BAY 11390), thifensulfuron-methyl, thiobencarb, tralkoxydim, tri-allate, triasulfuron, triaziflam, tribenuron-methyl, triclopyr, triclopyr-butotyl, triclopyr-triethylammonium, tridiphane, trifluralin, triflusulfuron-methyl, and vernolate.

In certain instances, combinations with other herbicides having a similar spectrum of control but a different mode of action will be particularly advantageous for preventing the development of resistant weeds.

The following Tests demonstrate the control efficacy of the compounds of this invention against specific weeds. The weed control afforded by the compounds is not limited, however, to these species. See Index Tables A-E for compound descriptions. The following abbreviations are used in the Index Tables which follow: *t* = tertiary, *s* = secondary, *n* = normal, *i* = iso, *c* = cyclo, Me = methyl, Et = ethyl, Pr = propyl, *i*-Pr = isopropyl, Bu = butyl, Ph = phenyl, OMe = methoxy, OEt = ethoxy, SMe = methylthio, SEt = ethylthio, CN = cyano, NO₂ = nitro, TMS = trimethylsilyl, S(O)Me = methylsulfinyl, and S(O)₂Me = methylsulfonyl. The abbreviation "dec" indicates that the compound appeared to decompose on melting. The abbreviation "Ex." stands for "Example" and is followed by a number indicating in which example the compound is prepared.

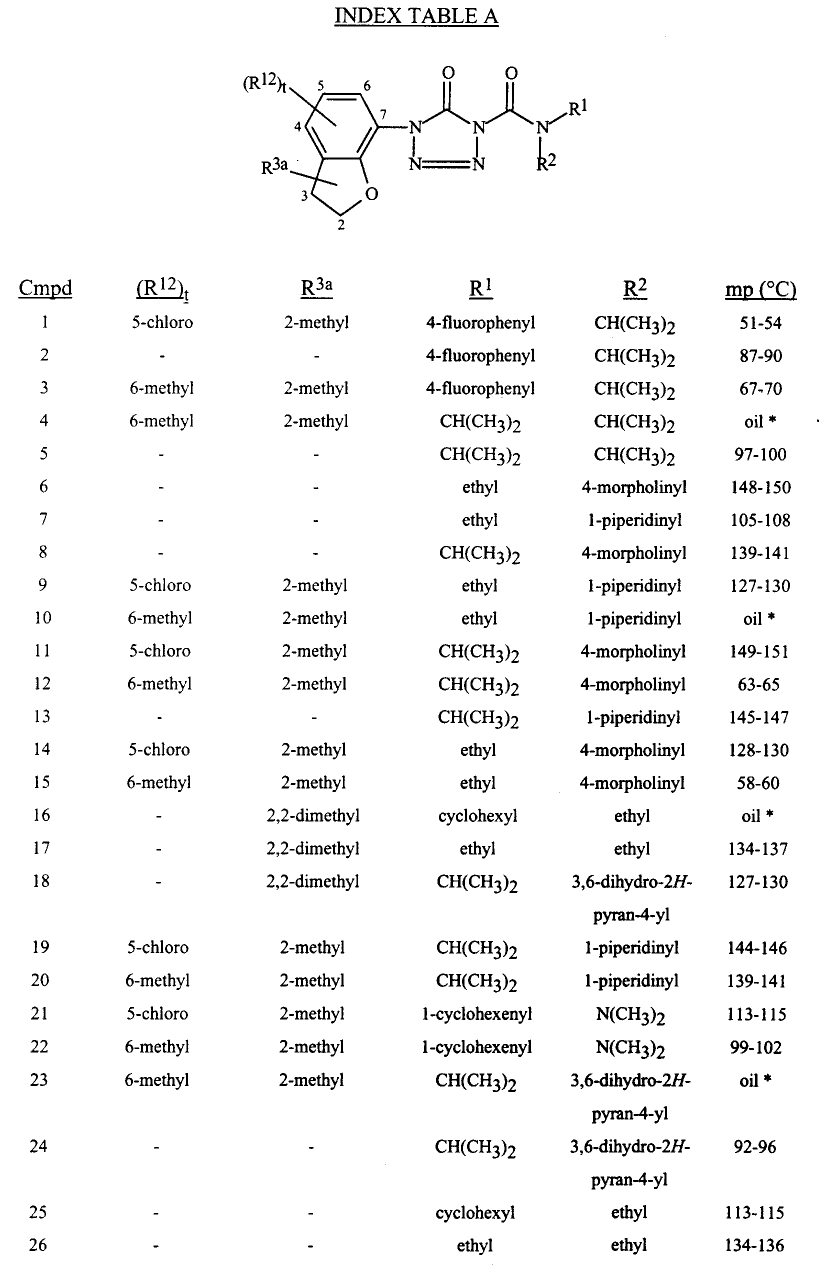

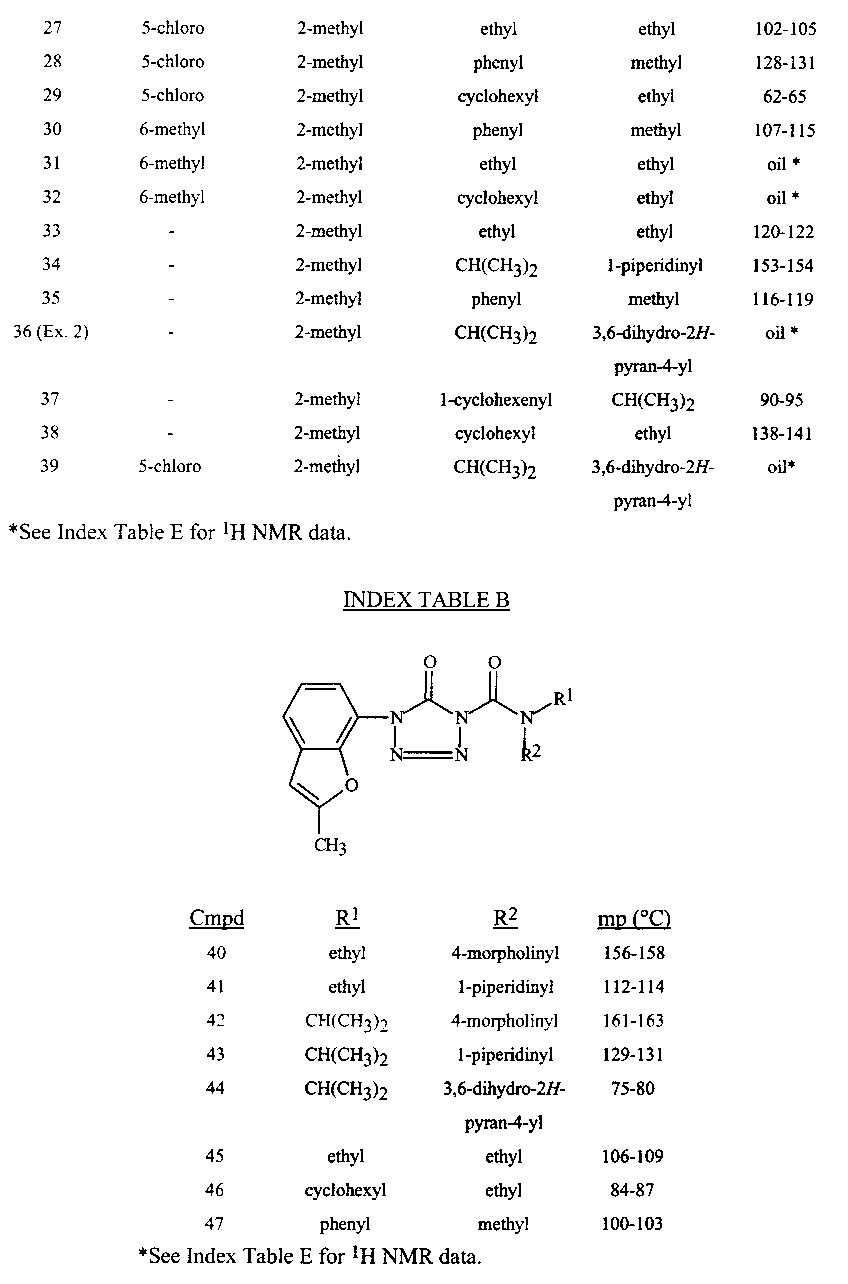

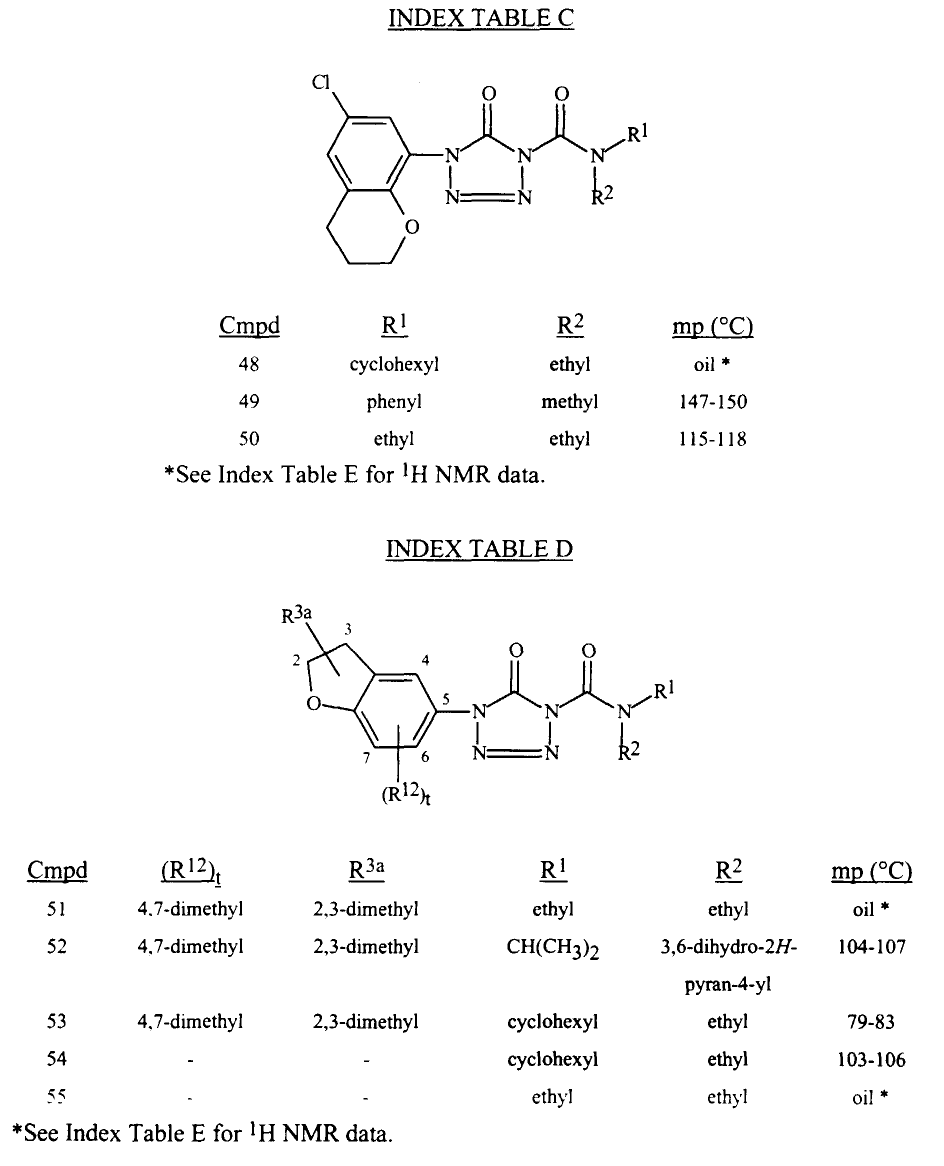

**INDEX TABLE E**

| Cmpd No. | 1H NMR Data (CDCl₃ solution unless indicated otherwise)^{a} |
|---|---|
| 4 | δ 1.4 (m, 15H), 2.2 (m, 3H), 2.8 (m, 1H), 3.3 (m, 1H), 3.64.1 (m, 2H), 5.0 (m, 1H), 6.75 (d, 1H), 7.15 (d, 1H). |
| 10 | δ 1.4 (m, 6H), 1.6 (m, 6H), 2.2 (s, 3H), 2.6 (m, 2H), 2.8 (m, 1H), 3.0 (m, 2H), 3.35 (m, 1H), 3.6 (m, 2H), 5.0 (m, 1H), 6.75 (m, 1H), 7.1 (m, 1H). |
| 16 | δ 1.25 (m, 6H), 1.5 (s, 6H), 1.6-2.1 (m, 8H), 3.1 (m, 2H), 3.45 (m, 2H), 6.9 (m, 1H), 7.25 (m, 2H). |
| 23 | δ 1.35-1.5 (m, 9H), 2.2 (m, 3H), 2.3 (m, 2H), 2.85 (m, 1H), 3.25 (m, 1H), 3.8 (m, 2H), 4.15 (m, 2H), 4.45 (m, 1H), 5.0 (m, 1H), 5.7 (m, 1H), 6.75 (d, 1H), 7.15 (d, 1H). |
| 31 | δ 1.2 (m, 6H), 1.35 (m, 3H), 2.1 (s, 3H), 2.75 (m, 1H), 3.25 (m, 1H), 3.35 (m, 2H), 3.5 (m, 2H), 4.9 (m, 1H), 6.7 (d, 1H), 7.05 (d, 1H). |
| 32 | δ 1.3-2.0 (m, 16H), 2.2 (s, 3H), 2.85 (m, 1H), 3.35 (m, 1H), 3.5 (m, 2H), 4.1 (m, 1H), 5.05 (m, 1H), 6.8 (d, 1H), 7.15 (d, 1H). |
| 36 | δ 1.4 (m, 6H), 1.45 (m, 3H), 2.35 (m, 2H), 2.9 (m, 1H), 3.4 (m, 1H), 3.8 (m, 2H), 4.15 (m, 2H), 4.45 (m, 1H), 5.05 (m, 1H), 5.7 (m, 1H), 6.9 (m, 1H), 7.2 (m. 1H), 7.25 (m, 1H). |
| 39 | δ 1.4 (m, 6H), 1.45 (m, 3H), 2.35 (m, 2H), 2.9 (m, 1H), 3.4 (m, 1H), 3.8 (m. 2H), 4.15 (m, 2H), 4.4 (m, 1H), 5.1 (m, 1H), 5.7 (m, 1H), 7.2 (m, 2H). |
| 48 | δ 1.2-2.0 (m, 14H), 2.1 (m, 2H), 2.8 (m, 2H), 3.45 (m, 2H), 4.2 (m, 2H), 7.15 (m, 1H), 7.25 (m, 1H). |
| 51 | δ 1.1-1.5 (m, 12H), 2.1-2.3 (m, 6H), 3.1-3.6 (m, 4H), 4.1-5.3 (m, 2H), 6.95 (m, 1H). |
| 55 | δ 1.3 (m, 6H), 3.3 (m, 2H), 3.4-3.65 (m, 4H), 4.65 (m, 2H), 6.9 (m, 1H), 7.55 (m, 1H), 7.65 (m, 1H). |

| | |
|---|---|
| ^{a 1}H NMR data are in ppm downfield from tetramethylsilane. Couplings are designated by (s)-singlet, (d)-doublet, (t)-triplet, (q)-quartet, (m)-multiplet, (dd)-doublet of doublets, (dt)-doublet of triplets, (br s)-broad singlet. | |

### BIOLOGICAL EXAMPLES OF THE INVENTION

### TEST A

Seeds of bedstraw (*Galium aparine*), blackgrass (*Alopecurus myosuroides*), broadleaf signalgrass (*Brachiaria decumbens*), cocklebur (*Xanthium strumarium*), corn (*Zea mays*), crabgrass (*Digitaria sanguinalis*), giant foxtail (*Setaria faberii*), lambsquarters (*Chenopodium album*), morningglory (*Ipomoea hederacea*), pigweed (*Amaranthus retroflexus*), rape (*Brassica napus*), soybean (*Glycine max*), sugar beet (*Beta vulgaris*), velvetleaf (*Abutilon theophrasti*), wheat (*Triticum aestivum*), wild oat (*Avena fatua*) and purple nutsedge (*Cyperus rotundus*) tubers were planted and treated preemergence with test chemicals formulated in a non-phytotoxic solvent mixture which included a surfactant.

At the same time, these crop and weed species were also treated with postemergence applications of test chemicals formulated in the same manner. Plants ranged in height from 2 to 18 cm (1 - to 4-leaf stage) for postemergence treatments. Plant species in the flood test consisted of rice (*Oryza sativa*), smallflower flatsedge (*Cyperus difformis*), duck salad (*Heteranthera limosa*) and barnyardgrass (*Echinochloa crus-galli*) grown to the 2-leaf stage for testing. Treated plants and controls were maintained in a greenhouse for twelve to sixteen days, after which all species were compared to controls and visually evaluated. Plant response ratings, summarized in Table A, are based on a scale of 0 to 10 where 0 is no effect and 10 is complete control. A dash (-) response means no test result.

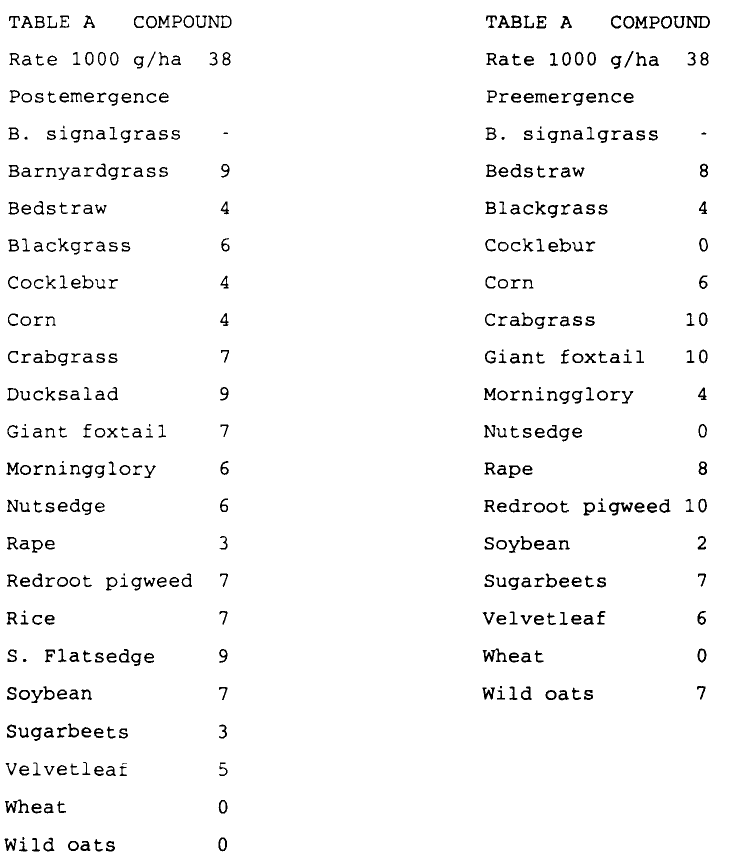

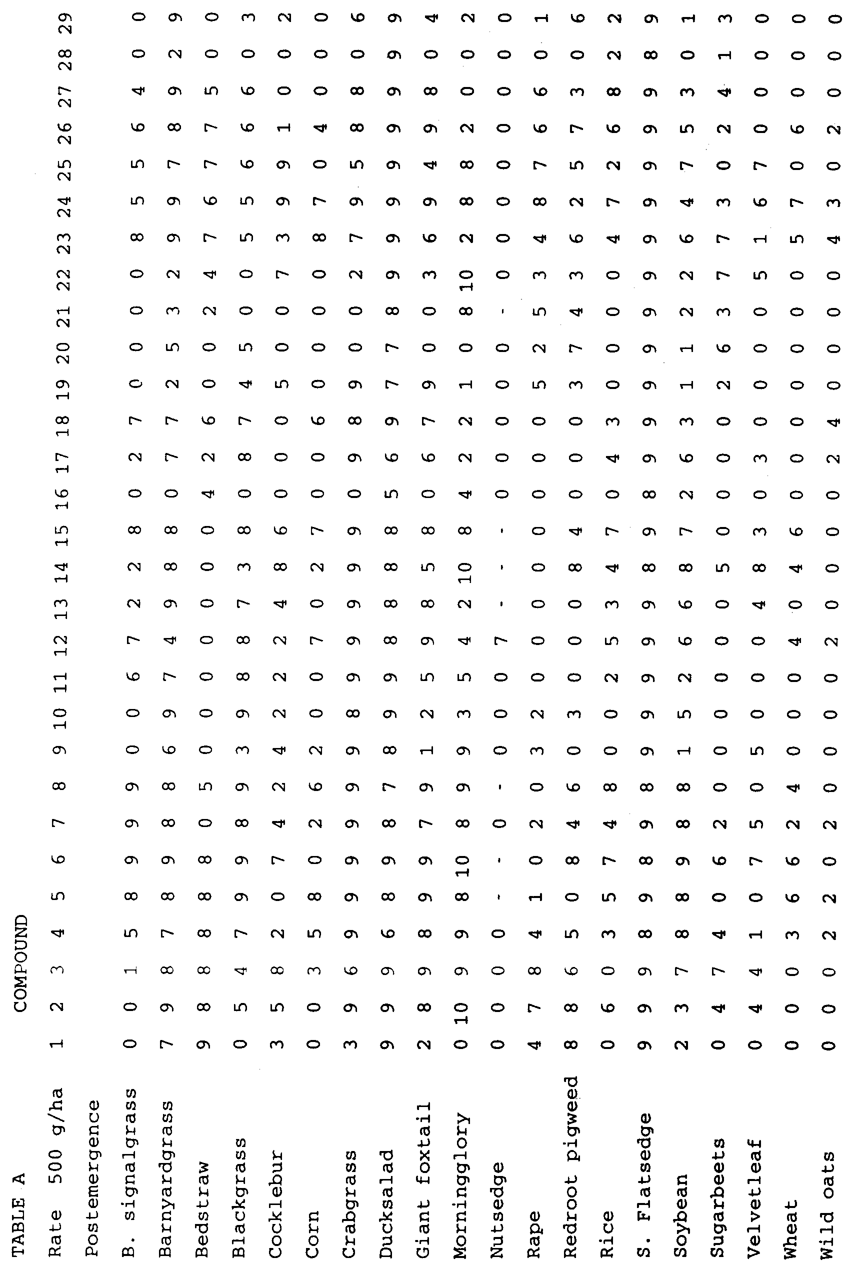

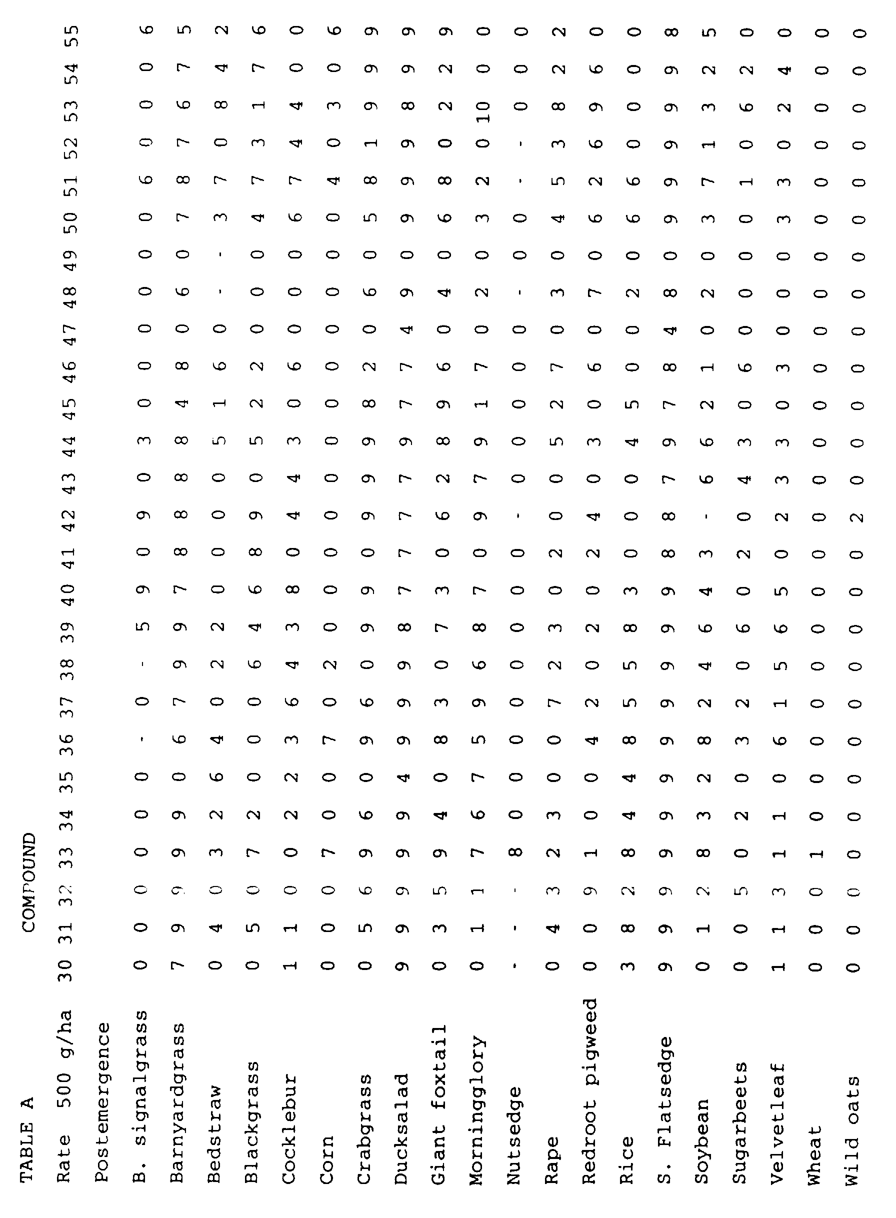

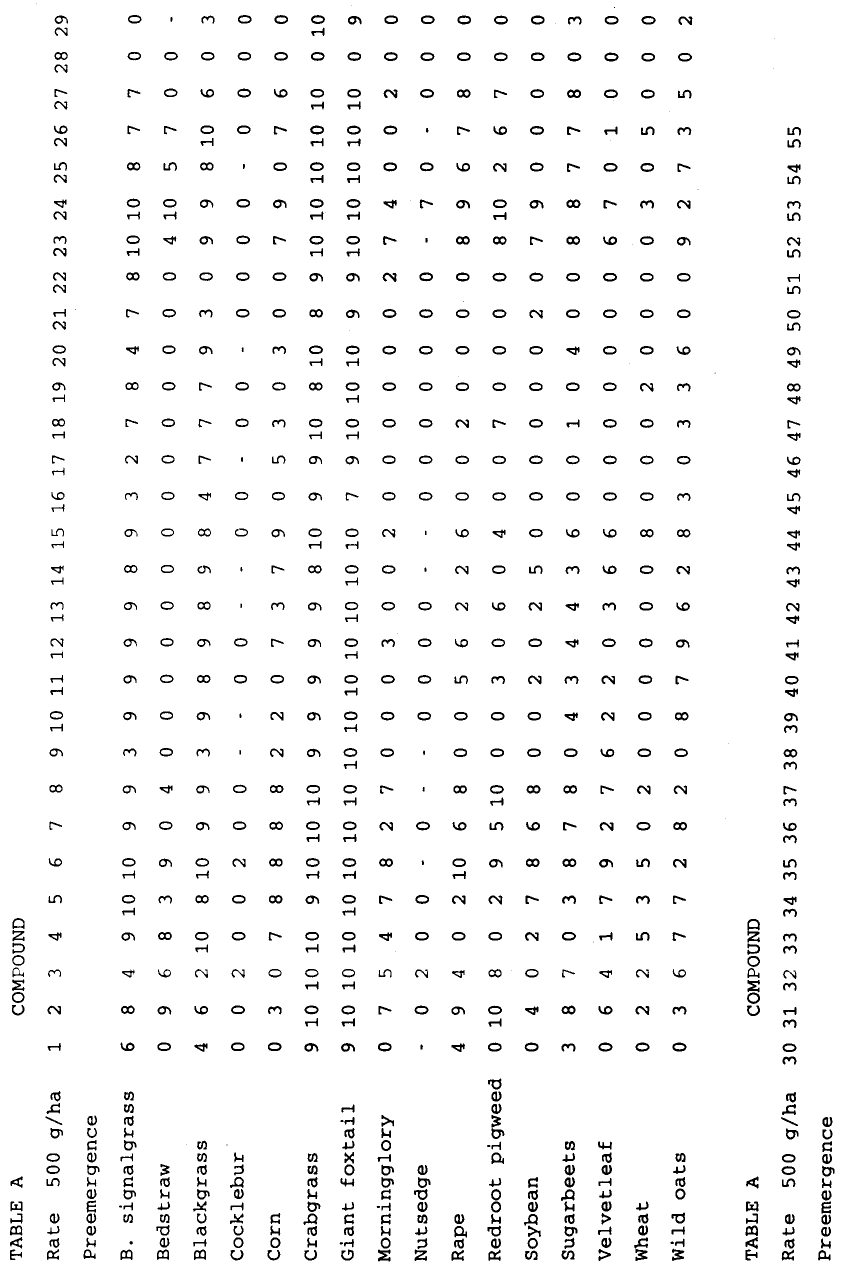

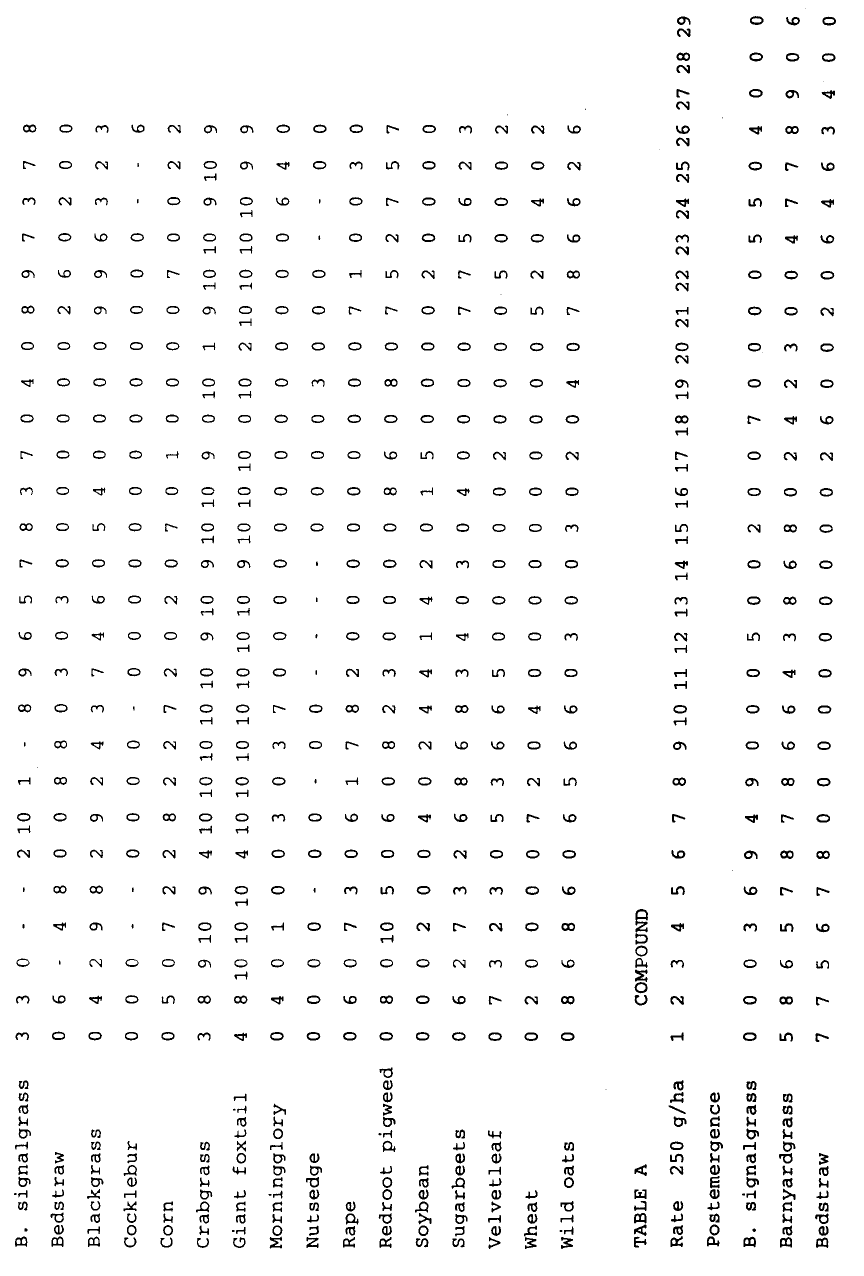

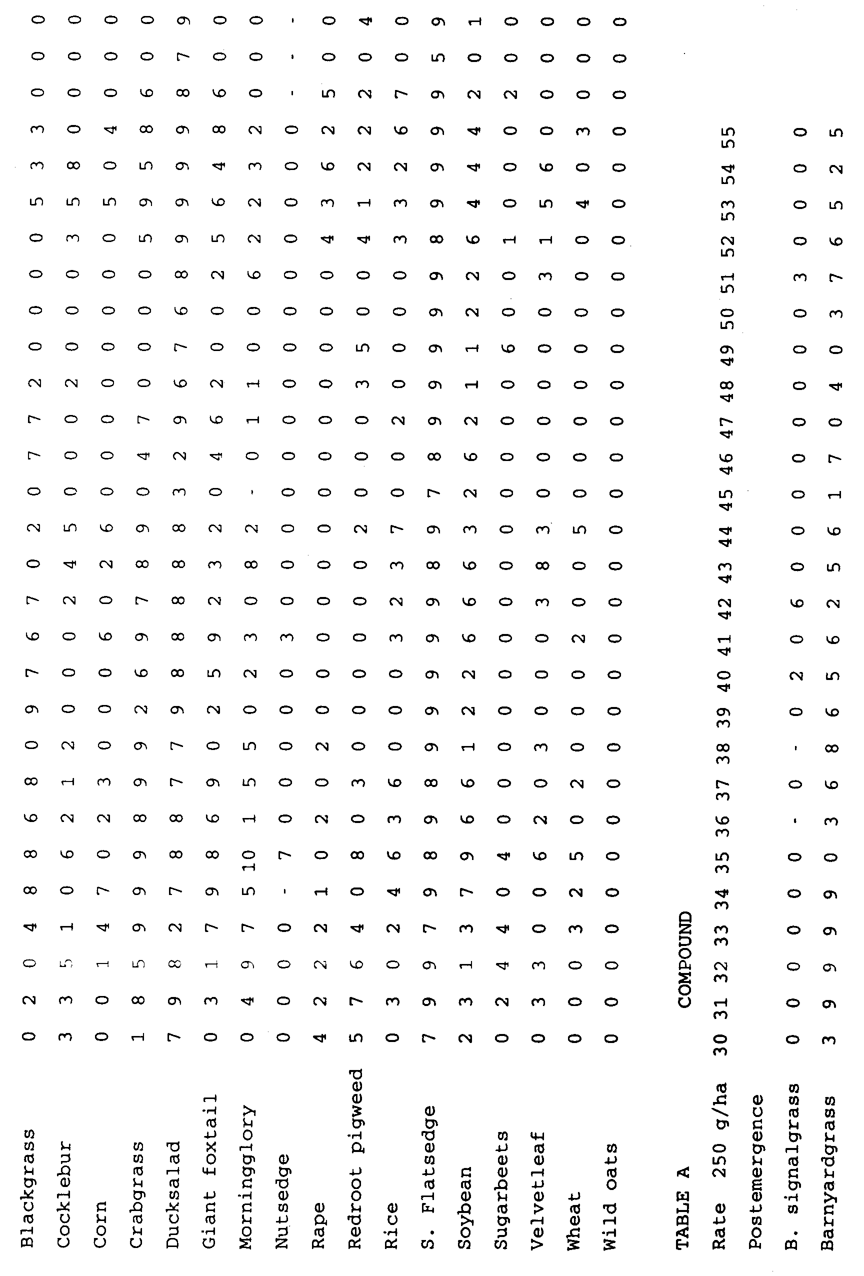

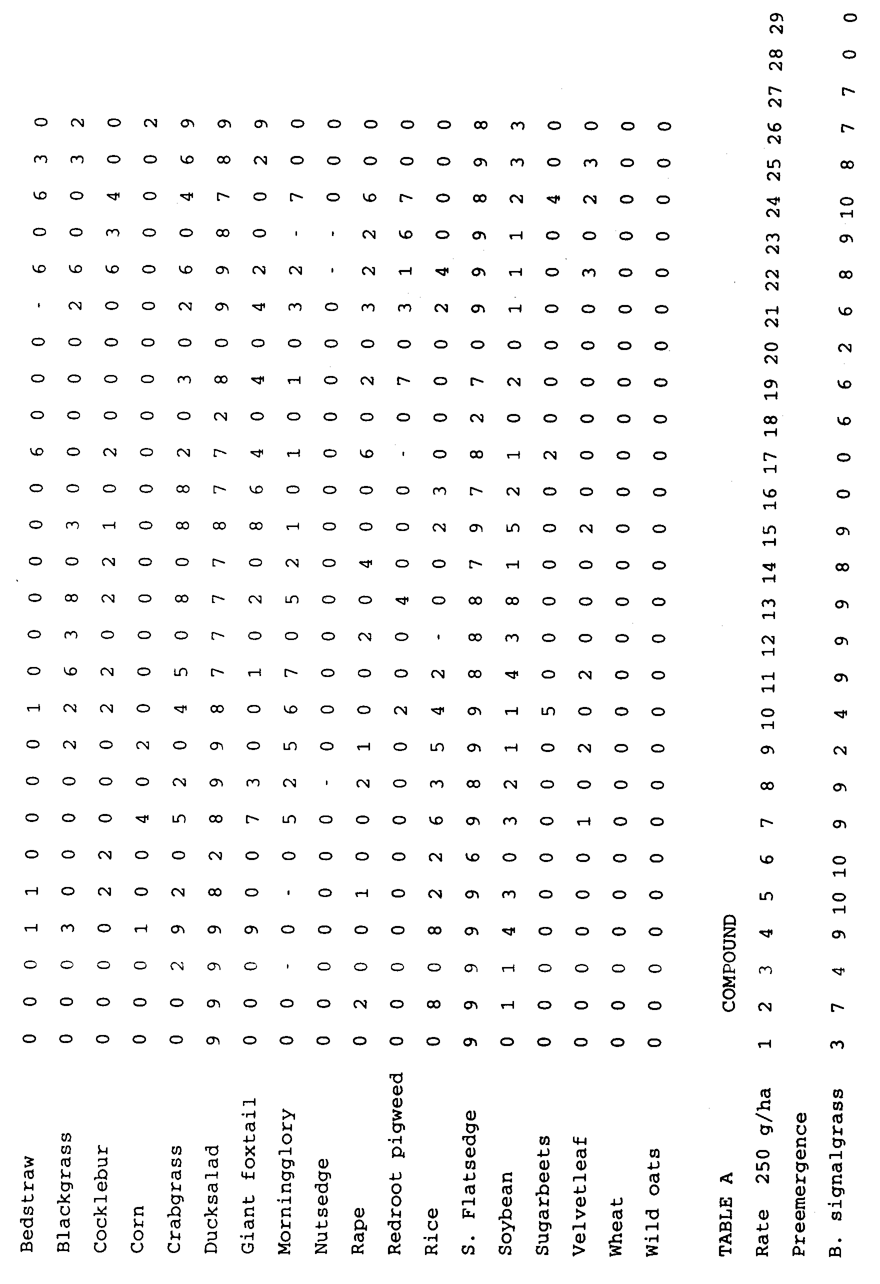

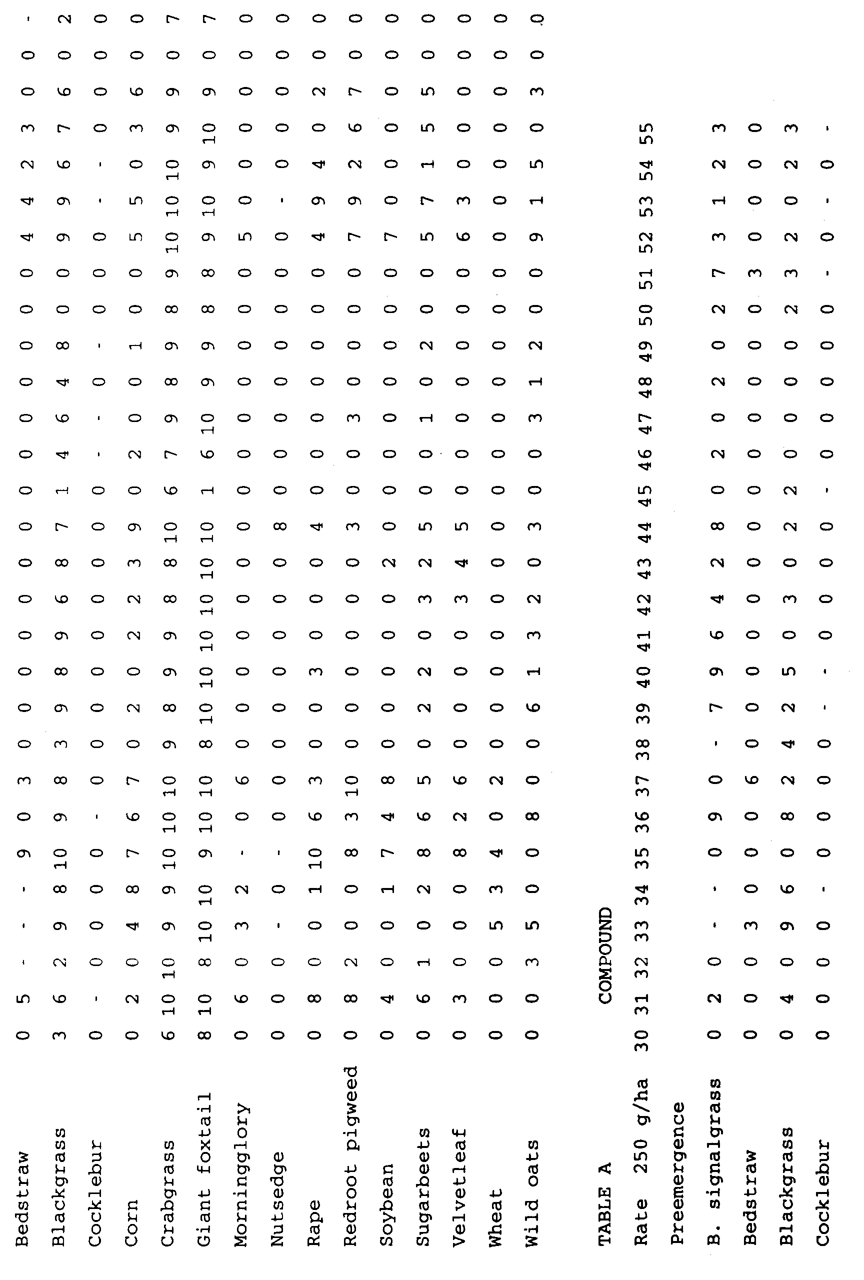

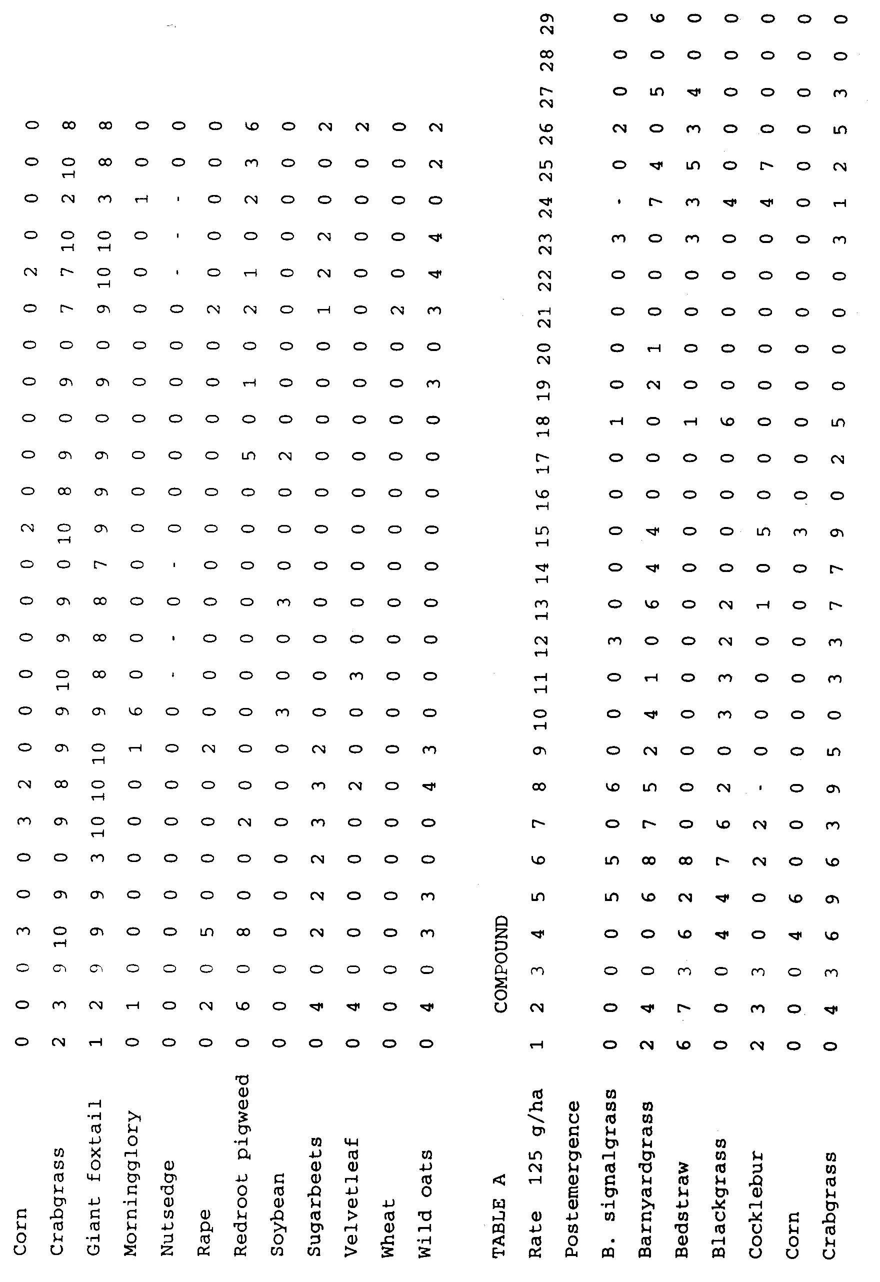

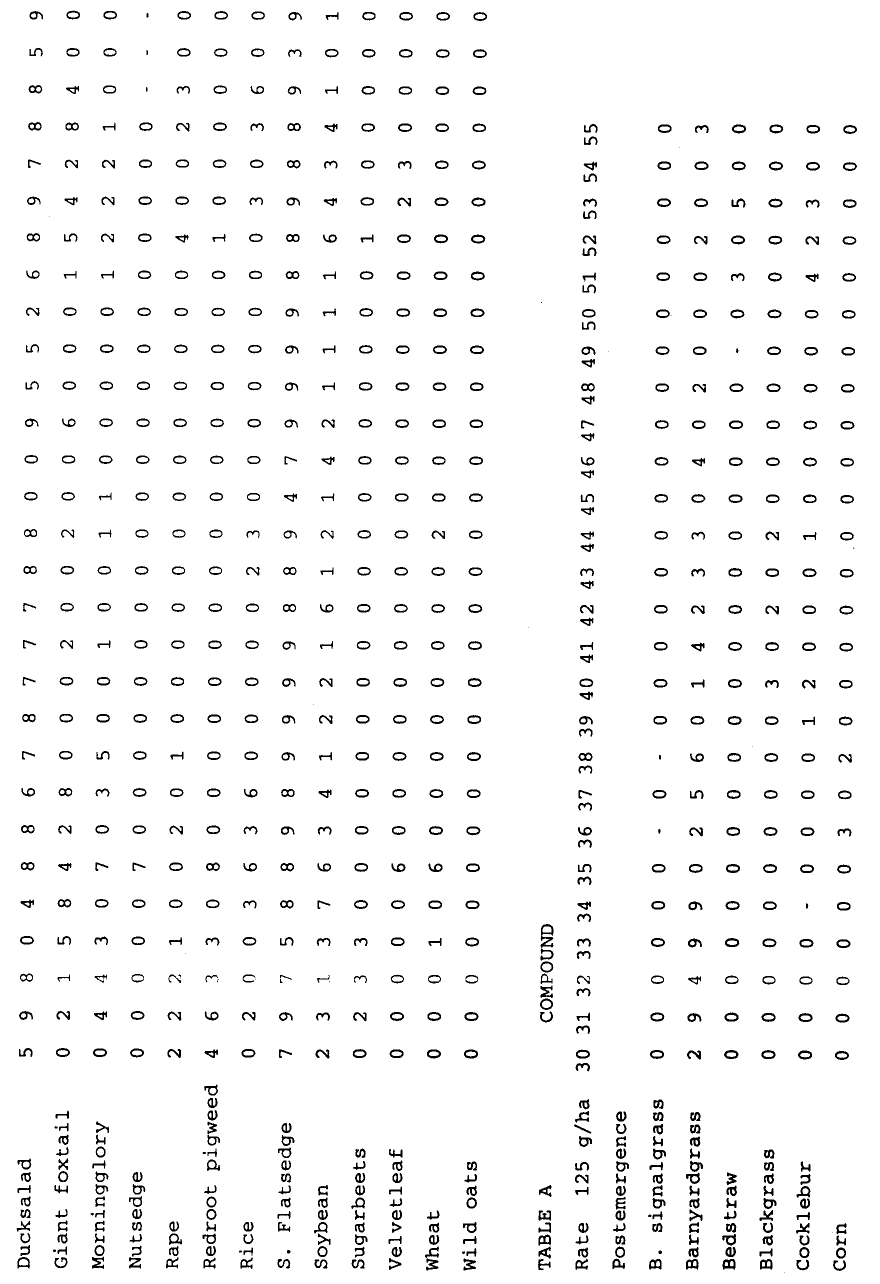

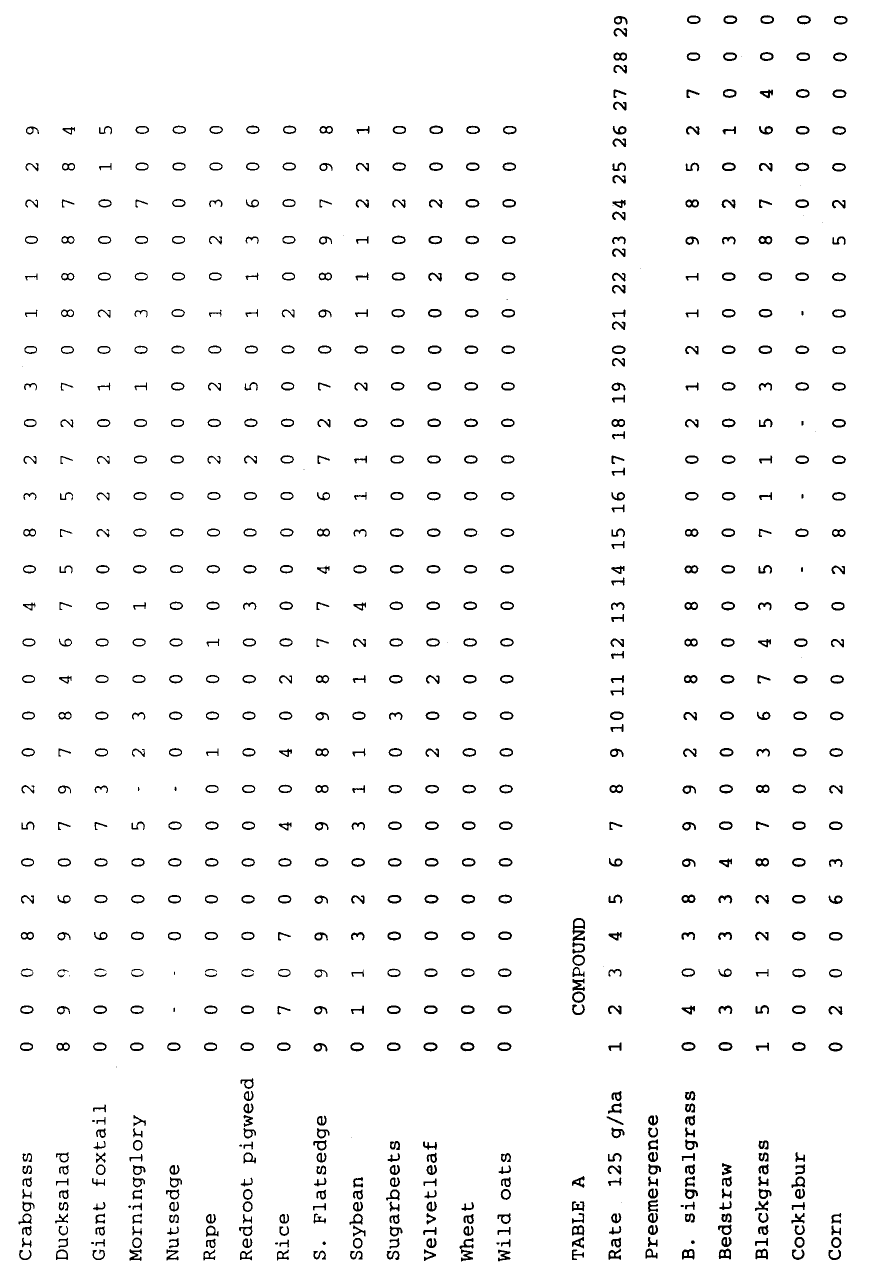

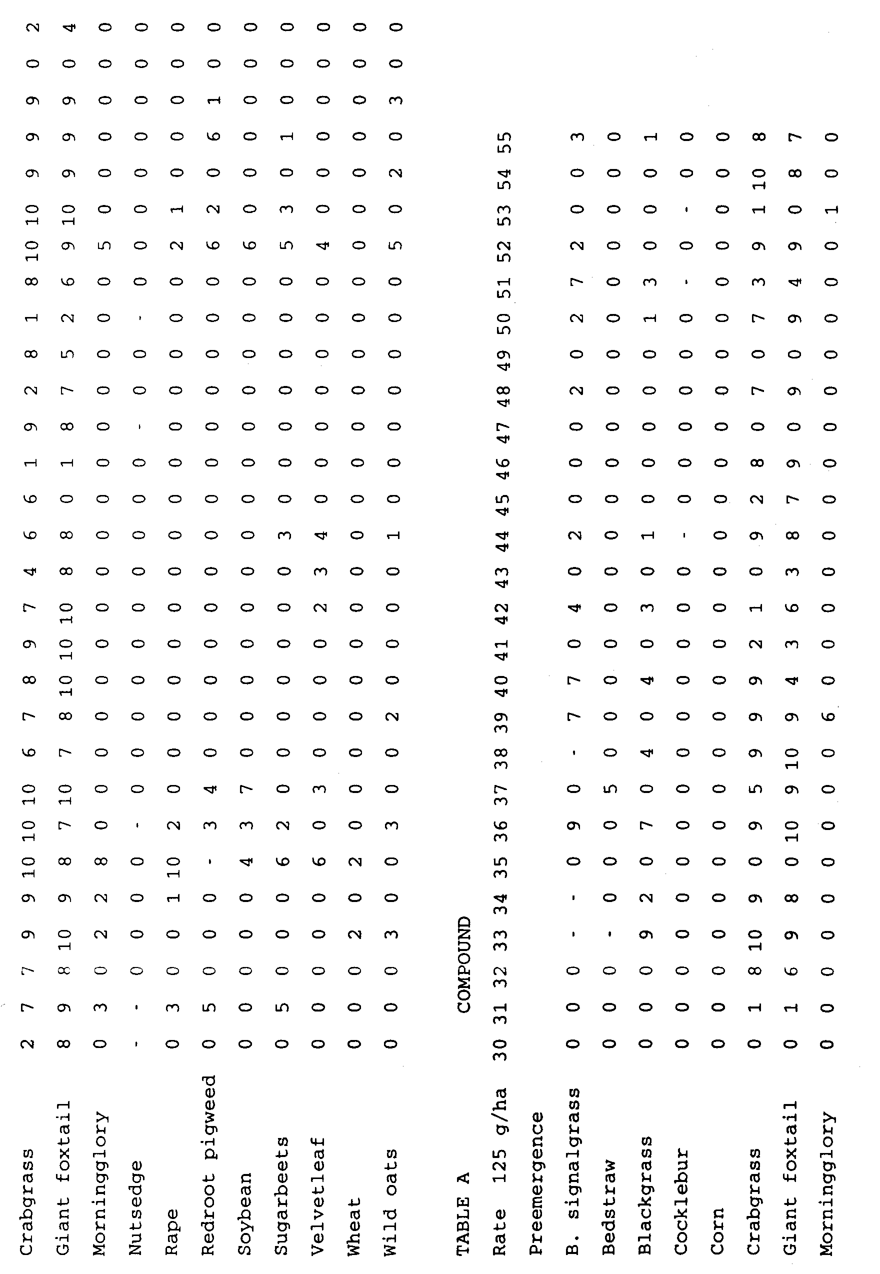

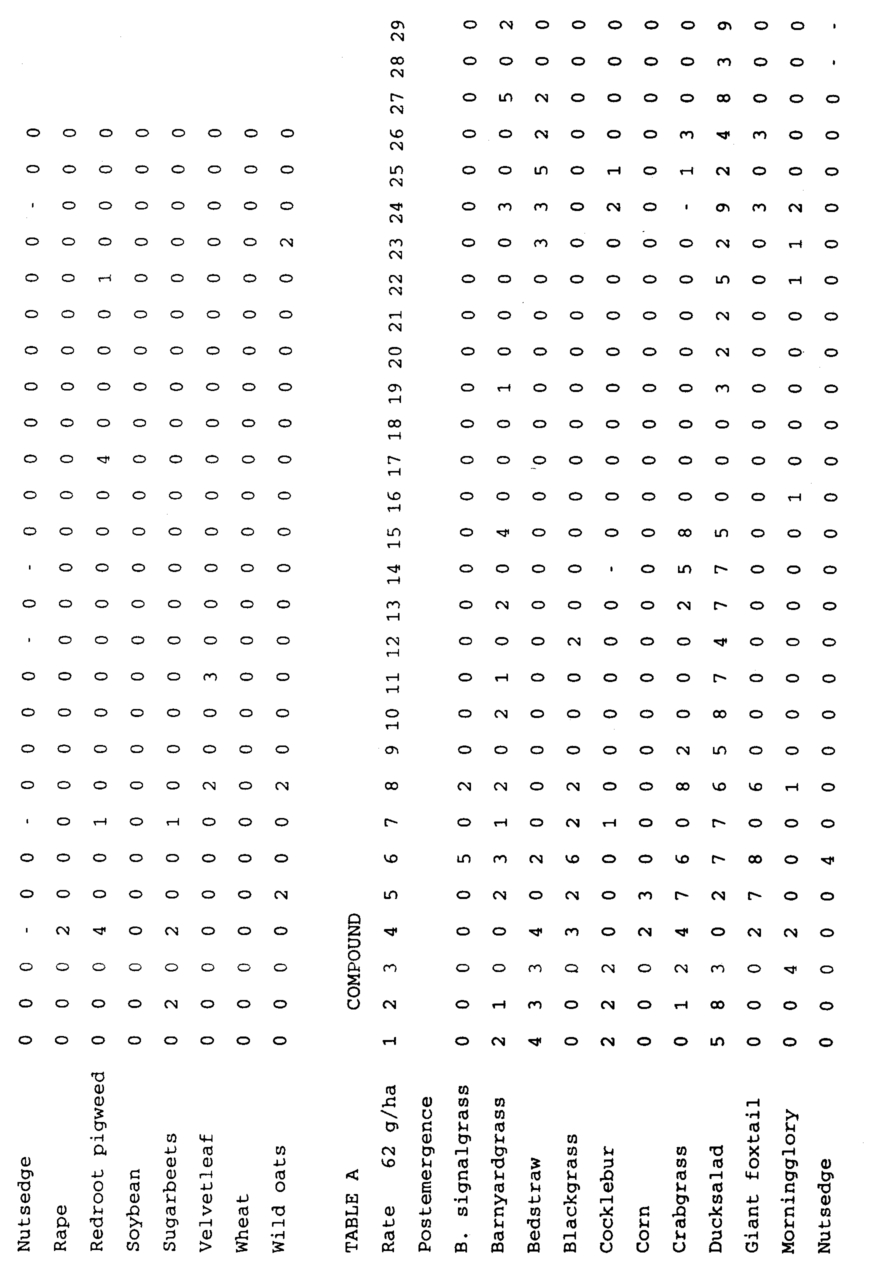

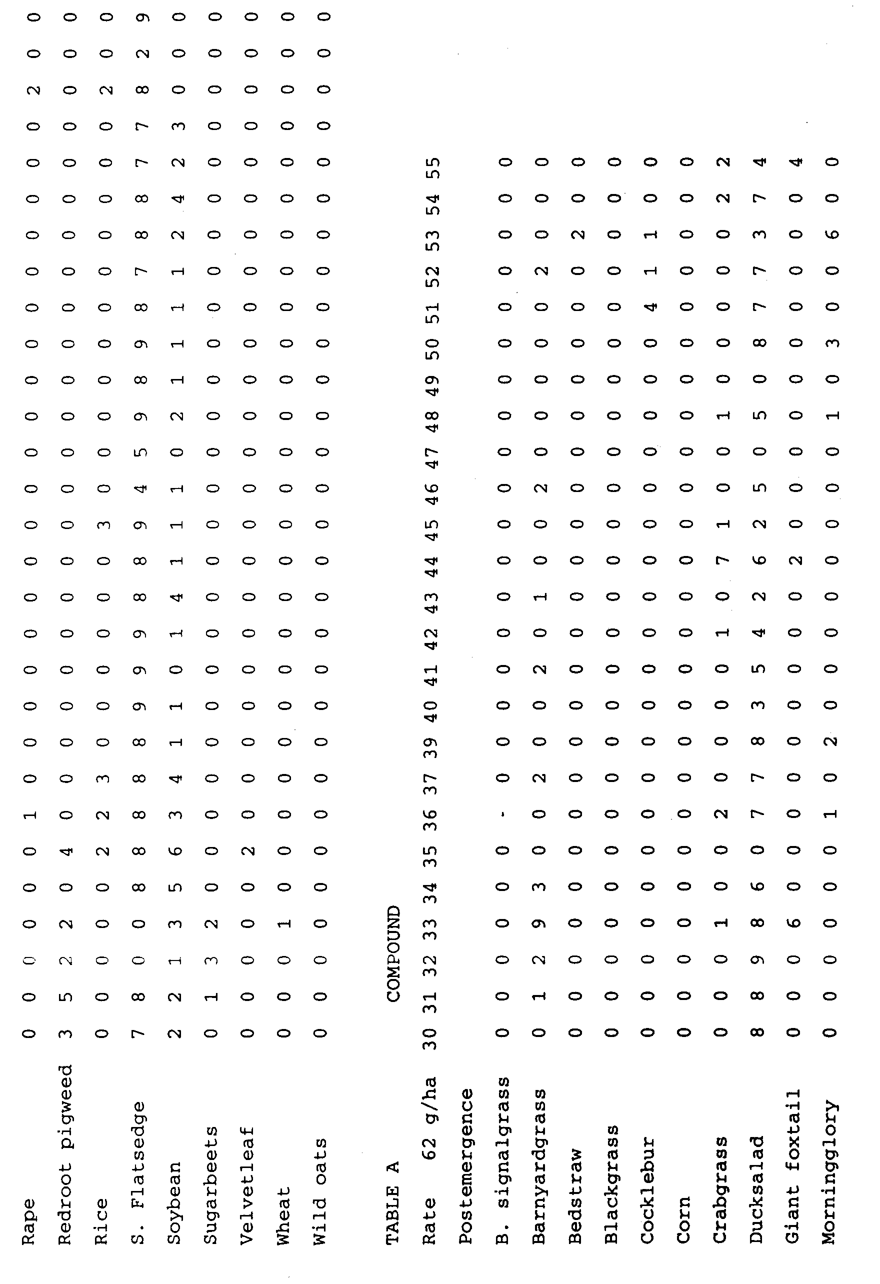

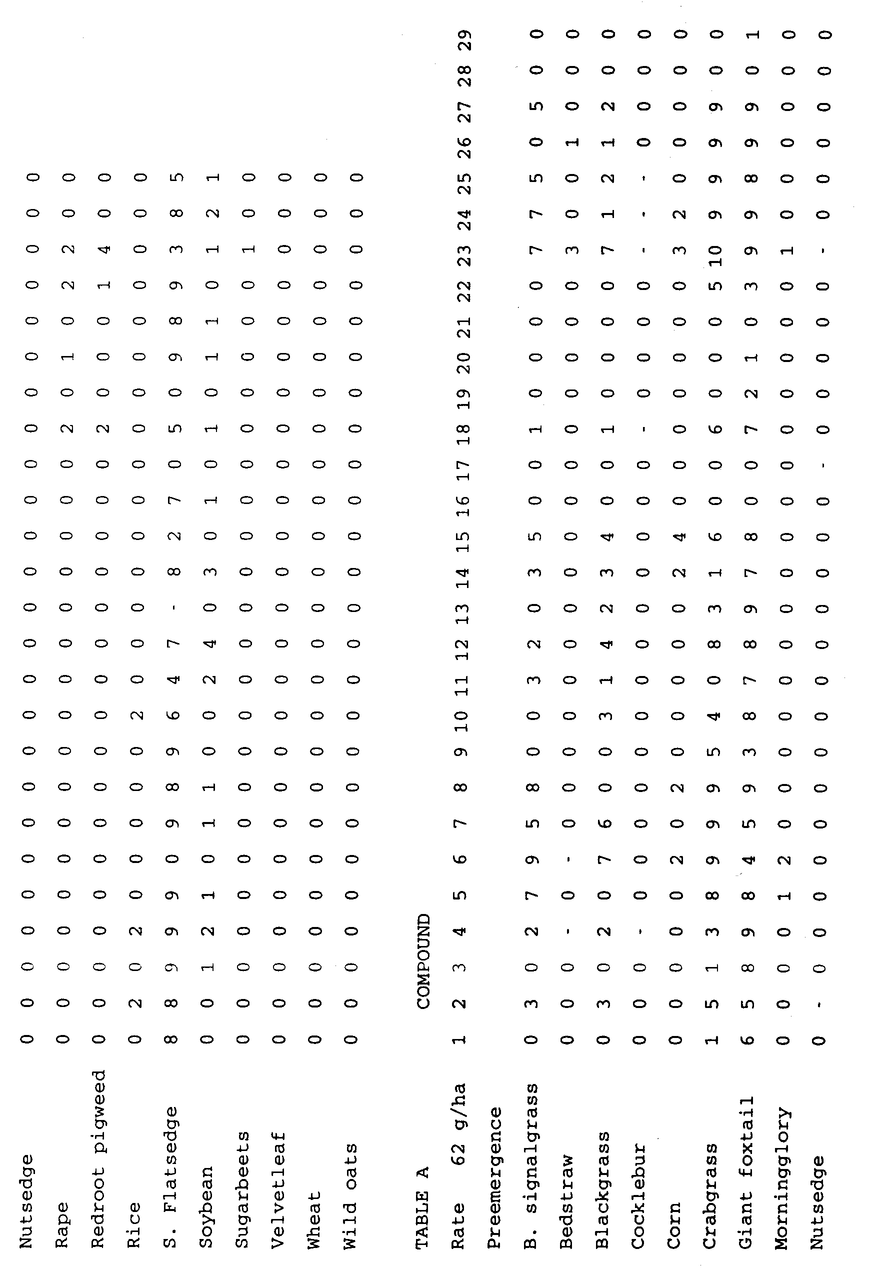

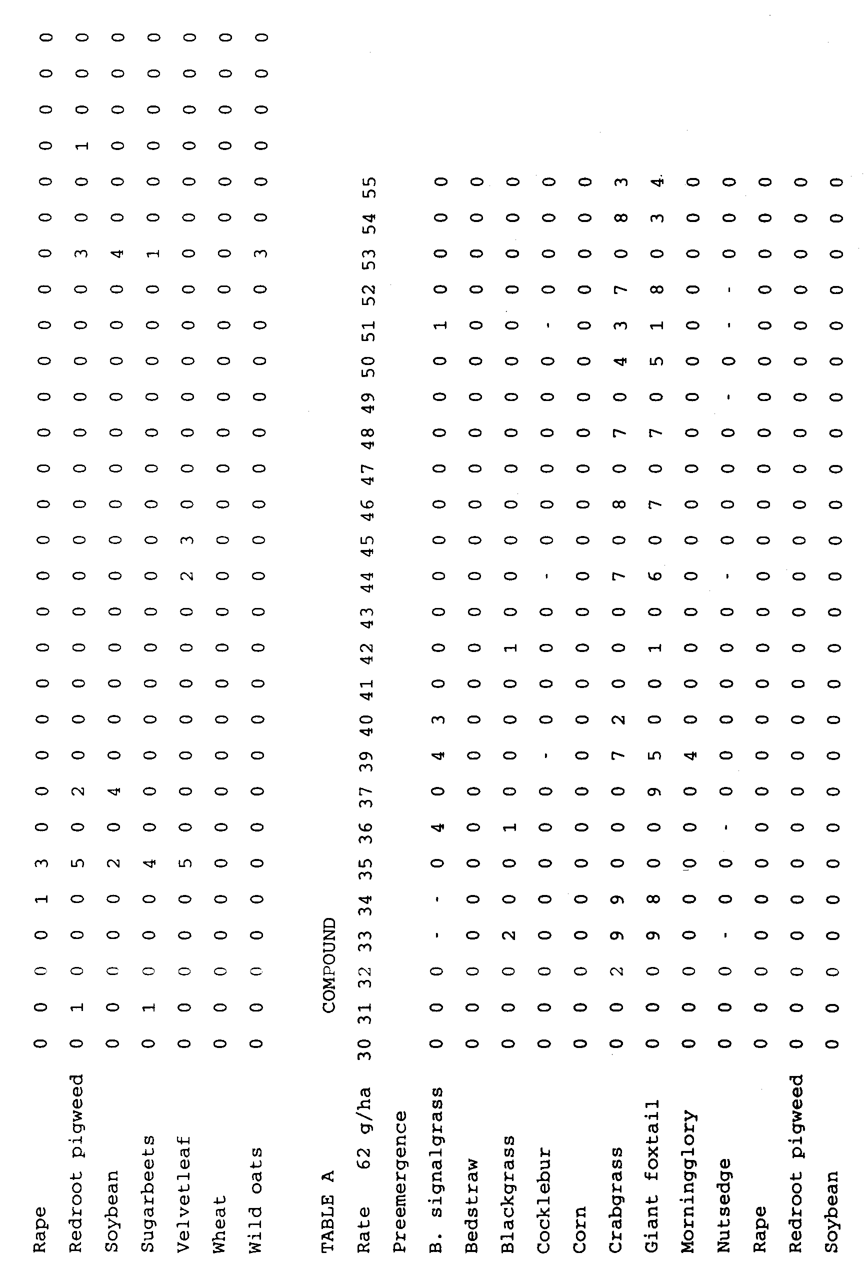

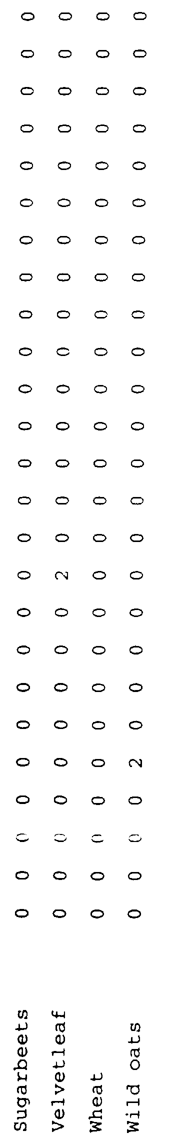

### TEST B

Compounds evaluated in this test were formulated in a non-phytotoxic solvent mixture which included a surfactant and applied to the surface of soil into which the appropriate weed and crop species had previously been seeded. A mixture of sandy loam soil was used for this test.

All plant species were grown using normal greenhouse practices. Crop and weed species include arrowleaf sida (*Sida rhombifolia*), barnyardgrass (*Echinochloa crus-galli*), cocklebur (*Xanthium strumarium*), common ragweed (*Ambrosia elatior*), corn 1 (*Zea mays*), cotton (*Gossypium hirsutum*), eastern black nightshade (*Solanum ptycanthum*), fall panicum (*Panicum dichotomiflorum*), field bindweed (*Convolvulus arvensis*), giant foxtail (*Setaria faberii*), hairy beggarticks (*Bidens pilosa*), ivyleaf morningglory (*Ipomoea hederacea*), johnsongrass (*Sorghum halepense*), ladysthumb smartweed (*Polygonum persicaria*), lambsquarters (*Chenopodium album*), large crabgrass (*Digitaria sanguinalis*), purple nutsedge (*Cyperus rotundus*), redroot pigweed (*Amaranthus retroflexus*), soybean 1(*Glycine max*), surinam grass (*Brachiaria decumbens*), velvetleaf (*Abutilon theophrasti*) and wild poinsettia (*Euphorbia heterophylla*).

Treated plants and untreated controls were maintained in a greenhouse for approximately 21 days, after which all treated plants were compared to untreated controls and visually evaluated. Plant response ratings, summarized in Table B, were based upon a 0 to 100 scale where 0 was no effect and 100 was complete control. A dash response (-) means no test result.

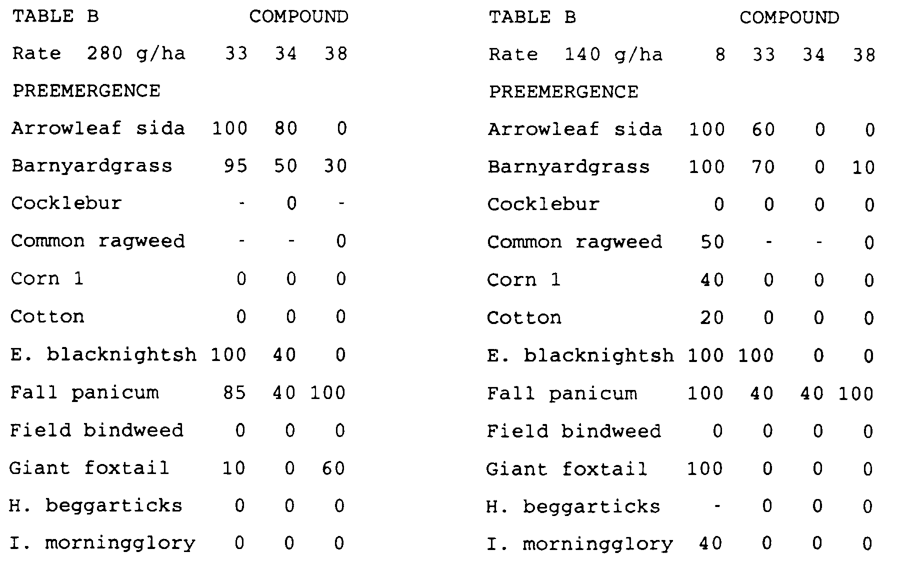

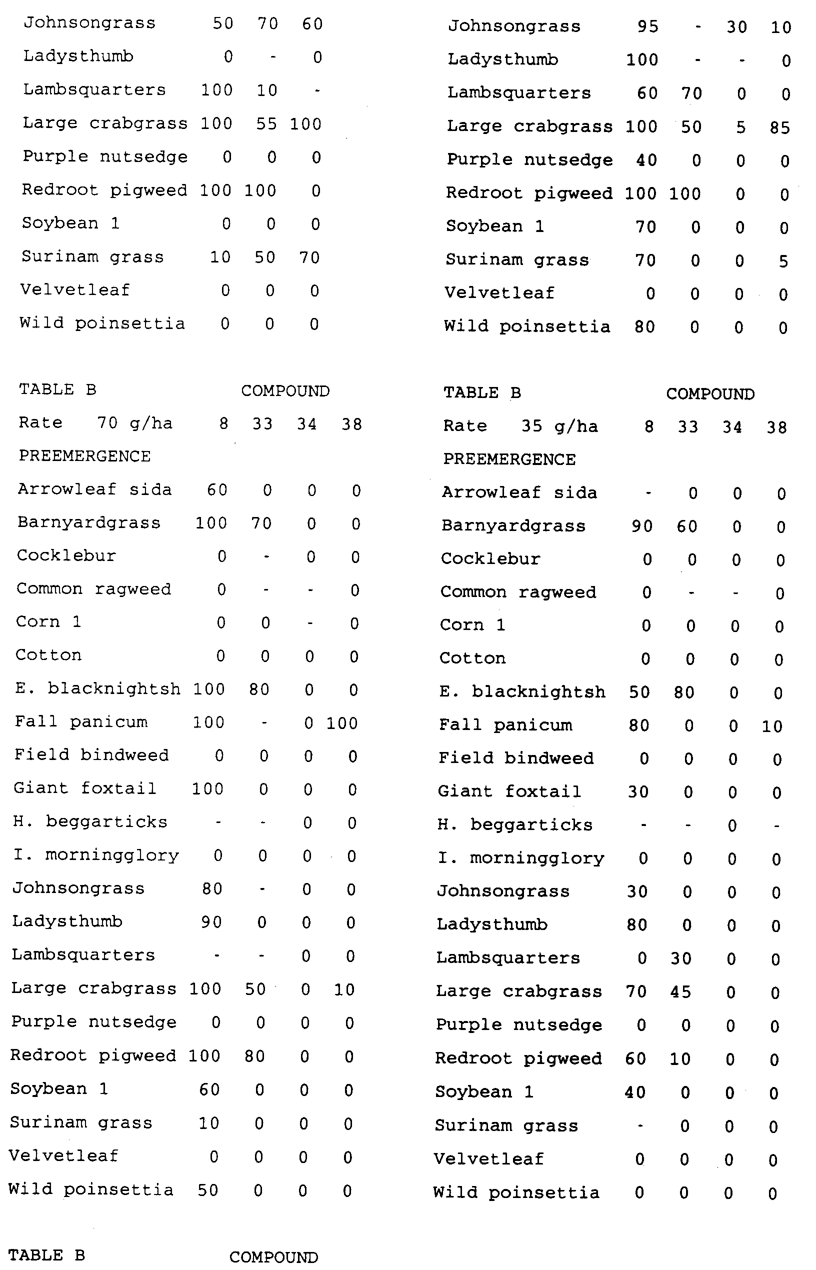

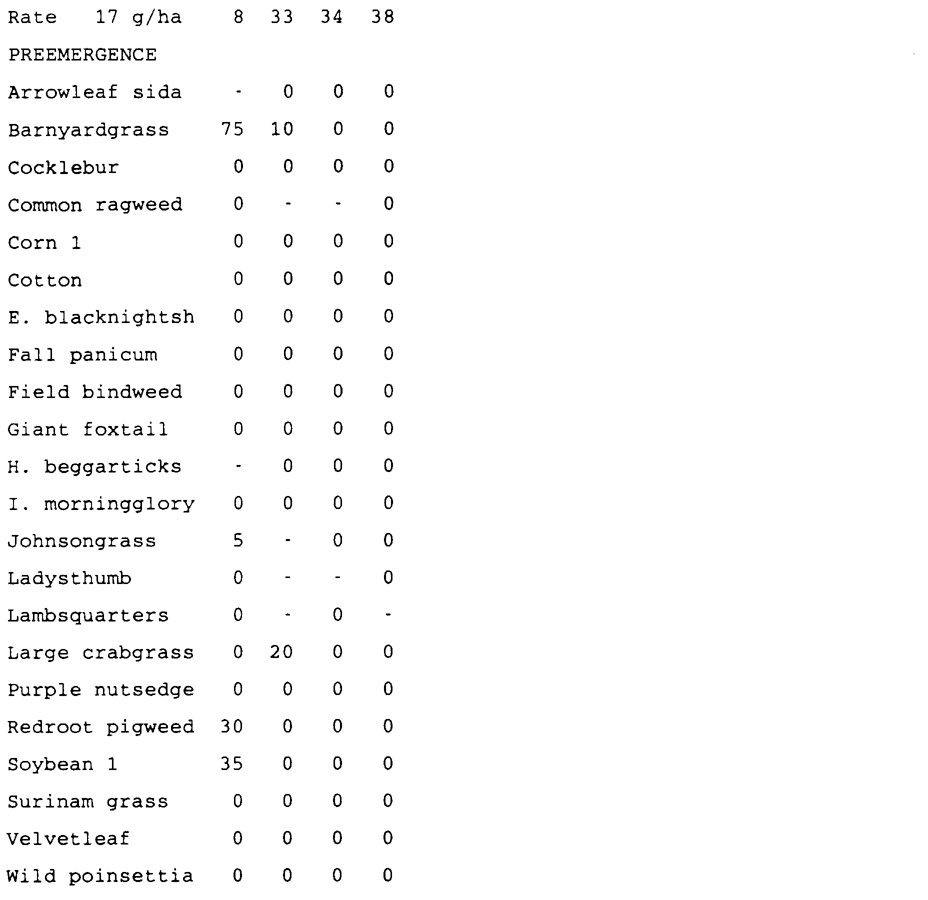

### TEST C

Seeds, tubers, or plant parts of alexandergrass (*Brachiaria plantaginea*), bermudagrass (*Cynodon dactylon*), common purslane (*Portulaca oleracea*), common ragweed (*Ambrosia elatior*), common groundsel (*Senecio vulgaris*), dallisgrass (*Paspalum dilatatum*), goosegrass (*Eleusine indica*), guineagrass (*Panicum maximum*), itchgrass (*Rottboellia exaltata*), johnson grass (*Sorghum halepense*), large crabgrass (*Digitaria sanguinalis*), pitted morningglory (*Ipomoea lacunosa*), purple nutsedge (*Cyperus rotundus*), sandbur (*Cenchrus echinatus*), sourgrass (*Trichachne insularis*), Spanishneedles (*Bidens bipinnata*) and tall mallow (*Malva sylvestris*) were planted into greenhouse pots of flats containing greenhouse planting medium. Plant species were grown grown in separate pots or individual compartments. Preemergence applications were made within one day of planting the seed or plant part.

Test chemicals were formulated in a non-phytotoxic solvent mixture which included a surfactant and applied preemergence to the plants. Untreated control plants and treated plants were placed in the greenhouse and visually evaluated for injury 13 to 21 days after herbicide application. Plant response ratings, summarized in Table C, are based on a 0 to 100 scale where 0 is no injury and 100 is complete control. A dash (-) response means no test result.

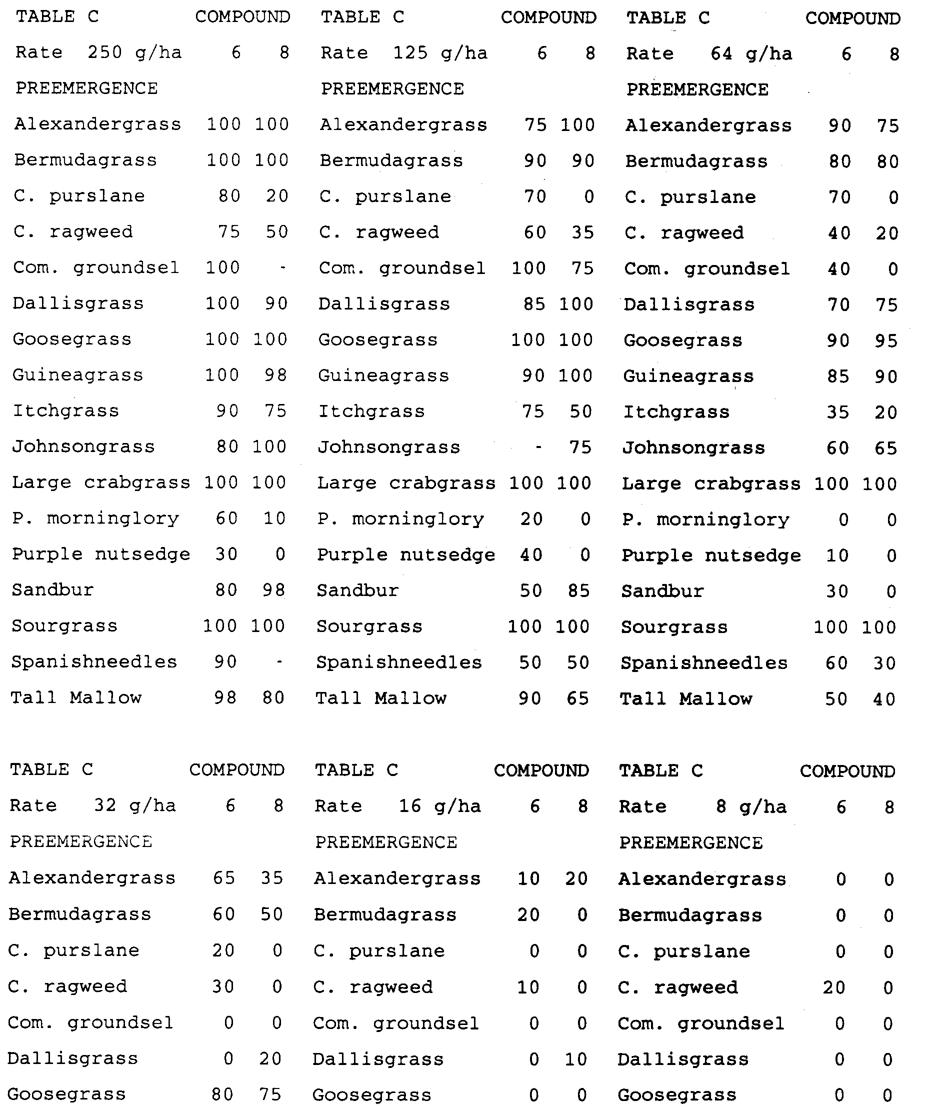

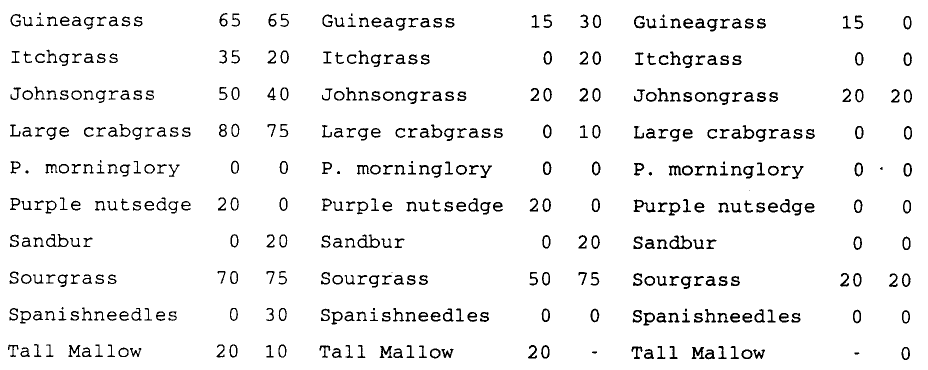

### TEST D

Compounds evaluated in this test were formulated in a non-phytotoxic solvent mixture and applied to the surface of the water which was contained in each pot. Individual containers of barnyardgrass (*Echinochloa oryzicola*), small flower flatsedge (*Cyperus difformus*), common falsepimpernel (*Lindernia procumbens*), monochoria (*Monochoria vaginalis*) and bulrush (*Scirpus juncoides*) were seeded and allowed to grow until the leaf stage of development was reached. A Saita clay loam soil was used for this propagation. Japonica rice (*Oryza sativa*) was transplanted at 0 and 2 cm depth (designated Rice japonica 1 and Rice japonica 2 respectively) five days before application of the test compound to the water surface. An early and late stage of each weed species was treated, the stage of development being related to the concurrent planting of Scirpus juncoides which was then treated at the 1.5 (early (1)) and the 2.5 (late (2)) leaf stage.

Treated plants and untreated controls were maintained under greenhouse conditions for twenty to thirty days at which time treated plants were compared to untreated controls and visually evaluated. Plant response ratings, summarized in Table D, are based upon a 0 to 100 scale where 0 is no effect and 100 is complete control. A dash response (-) indicated that no test result was recorded.

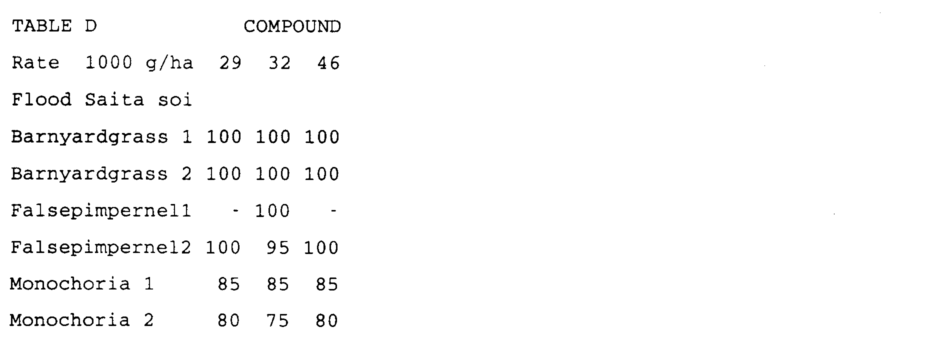

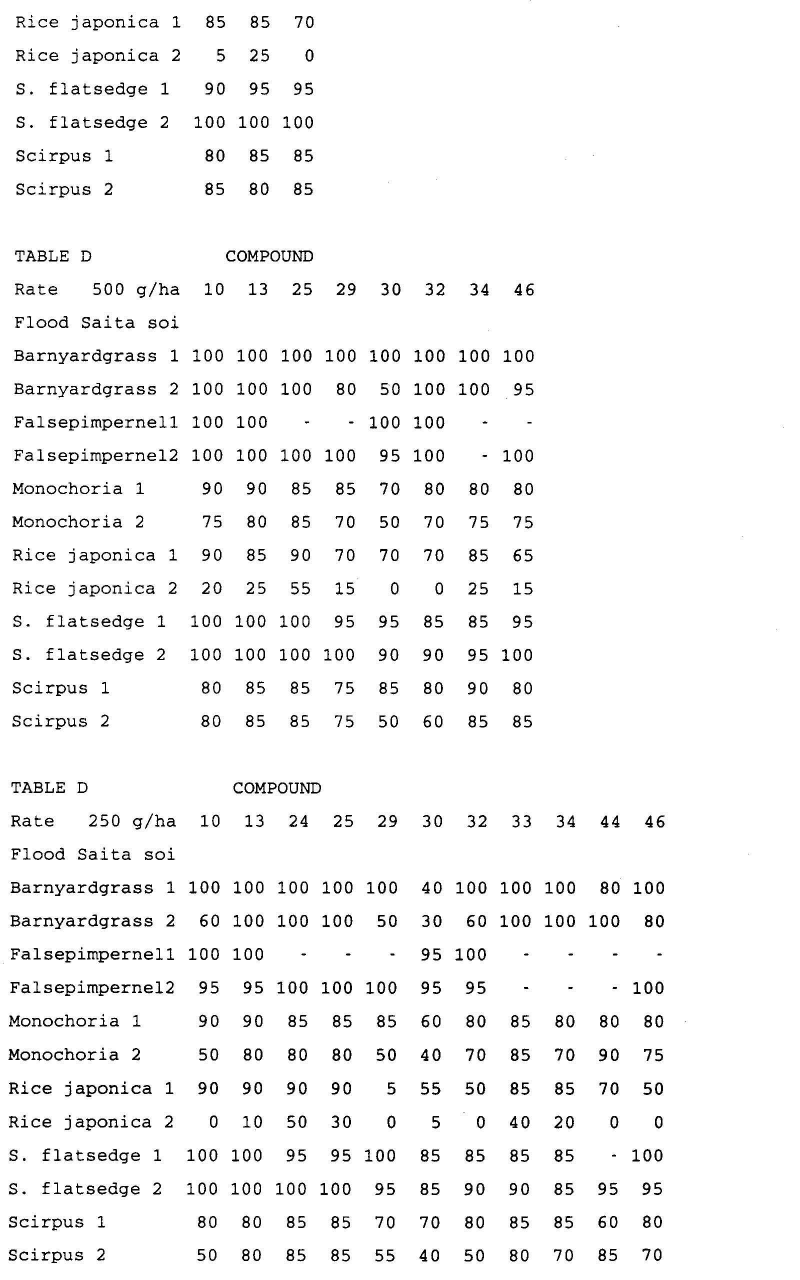

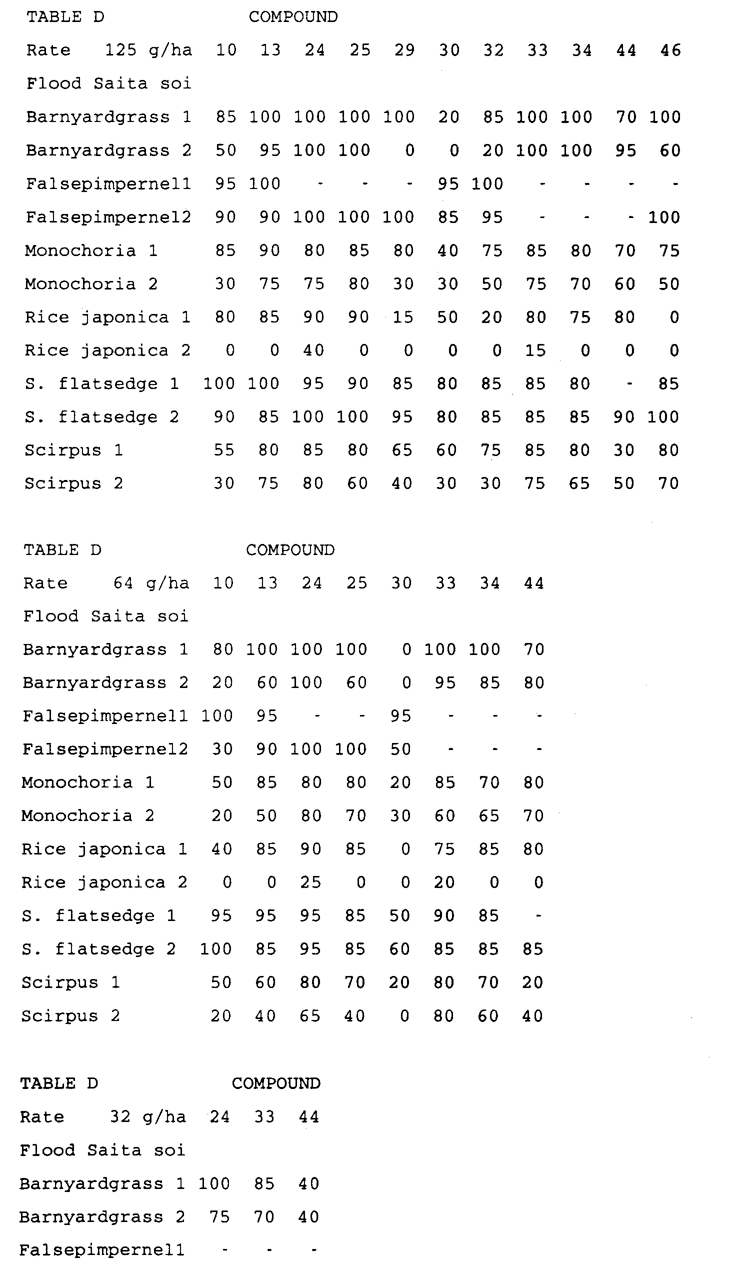

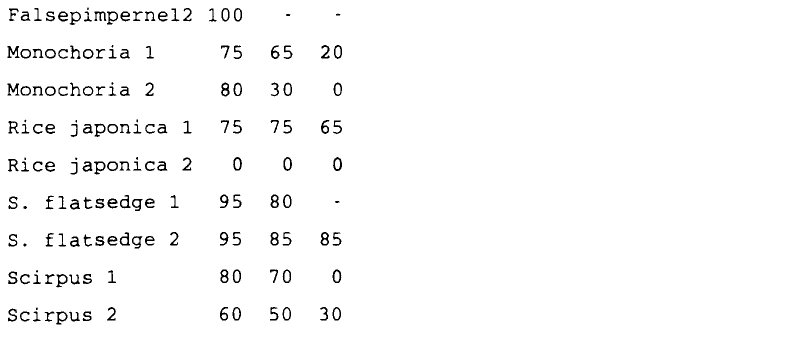

### TEST E

Compounds evaluated in this test were formulated in a non-phytotoxic solvent mixture which included a surfactant and applied to the surface of pots filled with a sandy loam soil and seeded with the appropriate species for testing.

All plant species were grown using normal greenhouse practices. Crop and weed species include annual bluegrass (*Poa annua*), blackgrass 2 (*Alopecurus myosuroides*), black nightshade (*Solanum nigra*), chickweed (*Stellaria media*), common poppy (*Papaver rhoeas*), deadnettle (*Lamium amplexicaule*), downy brome (*Bromus tectorum*), field violet (*Viola arvensis*), galium 2 (*Galium aparine*), green foxtail (*Setaria viridis*), Italian ryegrass (*Lolium multiflorum*), jointed goatgrass (*Aegilops cylindrica*), kochia (*Kochia scoparia*), lambsquarters (*Chenopodium album*), littleseed canarygrass (*Phalaris minor*), rape 1 (*Brassica napus*), redroot pigweed (*Amaranthus retroflexus*), Russian thistle (*Salsola kali*), scentless chamomile (*Matricaria inodora*), spring barley (*Hordeum vulgare*), sugar beet (*Beta vulgaris*), sunflower (*Helianthus annuus*), ivyleaf speedwell (*Veronica hederaefolia*), spring wheat (*Triticum aestivum*), winter wheat (*Triticum aestivum*), wild buckwheat (*Polygonum convolvulus*), wild mustard (*Sinapis arvensis*), wild oat 1 (*Avena fatua*), windgrass (*Apera spica-venti*) and winter barley (*Hordeum vulgare*).

Treated plants and untreated controls were maintained in a greenhouse for approximately 21 to 28 days, after which all treated plants were compared to untreated controls and visually evaluated. Plant response ratings, summarized in Table E, are based upon a 0 to 100 scale where 0 is no effect and 100 is complete control. A dash response (-) means no test result.

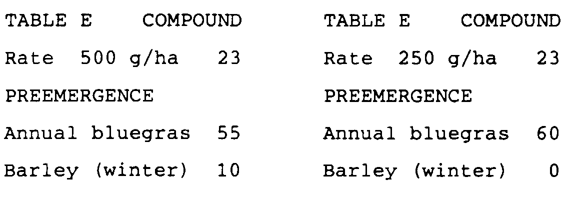

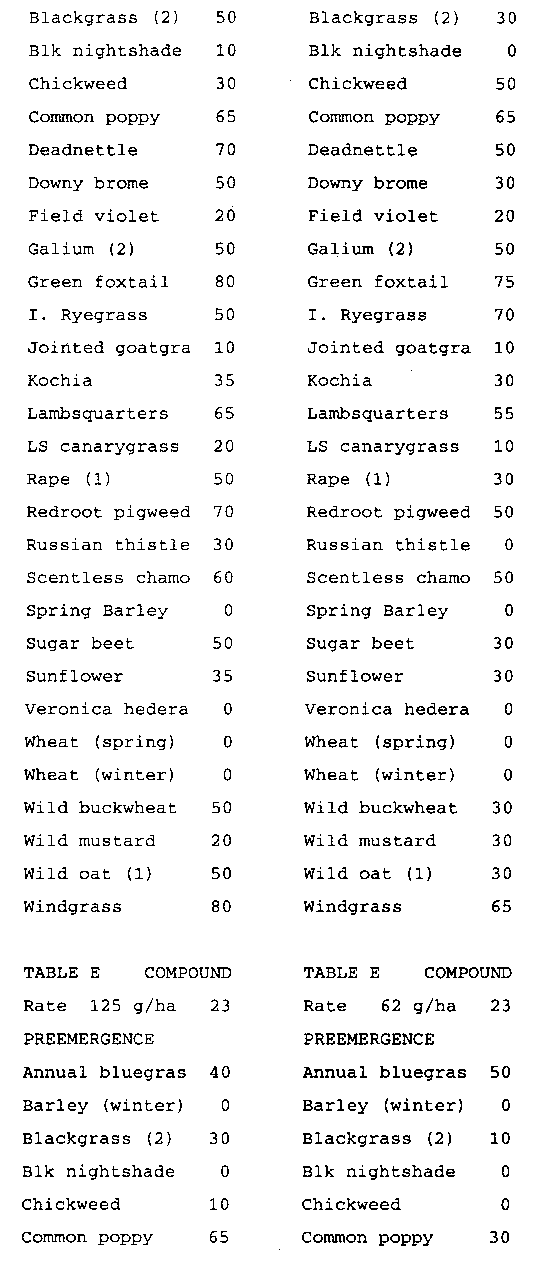

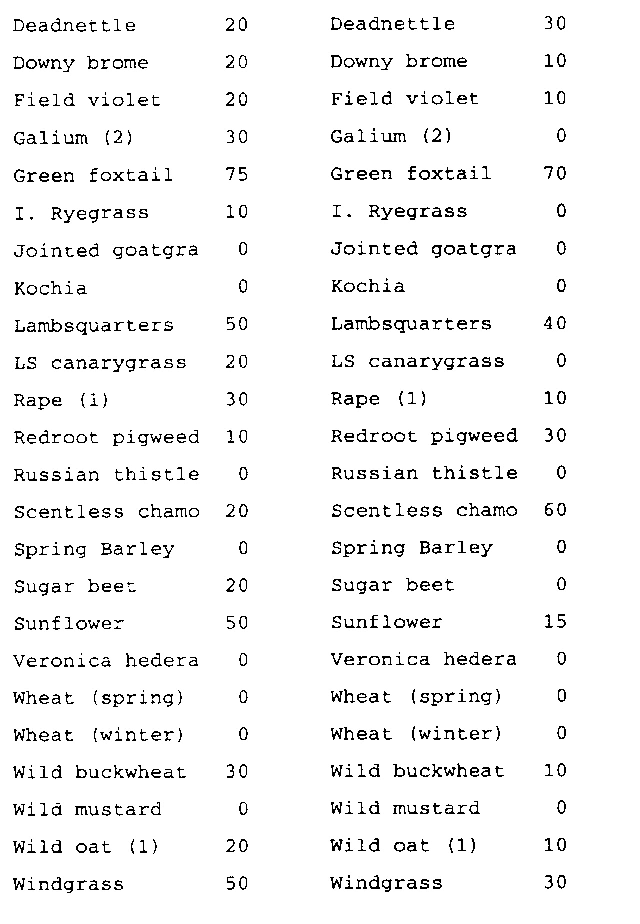

## Claims

1. A compound selected from Formula I, geometric or stereoisomers thereof, *N*-oxides thereof and agriculturally suitable salts thereof, 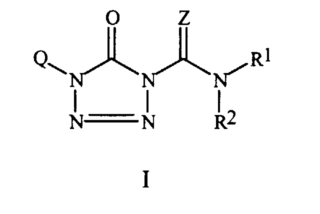 wherein
Q is 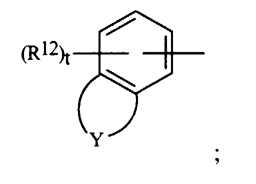
Y is, together with the carbons to which it is attached, a fully or partially saturated 5-, 6- or 7-membered heterocyclic ring which contains one or two X^{a} and is optionally substituted with 1-4 R^{3a}, provided that when said heterocyclic ring contains two X^{a}, then one X^{a} is other than O; or
Y is, together with the carbons to which it is attached, a 5- or 6-membered aromatic heterocyclic ring which contains one, two or three X^{b}, and is optionally substituted with 1-2 R^{3b}, provided that (a) when Y together with the carbons to which it is attached form a 6-membered aromatic heterocyclic ring, then X^{b} is N, and (b) when X^{b} is N substituted with R^{3b}, then R^{3b} is other than halogen;
X^{a} is O, S(O)ₙ or NR¹⁰;
X^{b} is O, S or N;
Z is O or S;
R¹ is C₁-C₆ alkyl, C₁-C₆ haloalkyl, C₃-C₆ alkenyl, C₃-C₆ haloalkenyl, C₃-C₆ alkynyl, C₃-C₆ haloalkynyl, C₁-C₆ alkoxy, C₂-C₆ alkoxyalkyl, or C₂-C₆ haloalkoxyalkyl; or R¹ is C₃-C₇ cycloalkyl or C₃-C₇ cycloalkenyl, each optionally substituted with 1-4 R¹³; or R¹ is phenyl optionally substituted with C₁-C₃ alkyl, C₁-C₃ alkoxy, C₁-C₃ haloalkoxy, C₁-C₃ haloalkyl, halogen, cyano, nitro or C₂-C₄ alkoxycarbonyl;
R² is C₁-C₆ alkyl, C₁-C₆ haloalkyl, C₃-C₇ cycloalkyl, C₃-C₆ alkenyl, C₃-C₆ haloalkenyl, C₃-C₆ alkynyl, C₃-C₆ haloalkynyl, C₁-C₆ alkoxy, C₂-C₆ alkoxyalkyl, C₂-C₆ haloalkoxyalkyl or NR⁴R⁵; or
R² is 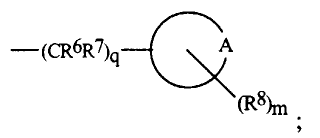 or
R¹ and R² can be taken together as -CH₂CH₂-, -CH₂CH₂CH₂-, -CH₂CH₂CH₂CH₂-, -CH₂CH₂CH₂CH₂-CH₂- or -CH₂CH₂OCH₂-CH₂-;
each R^{3a} is independently halogen, C₁-C₄ alkyl or C₁-C₄ alkoxy; or when two R^{3a} are attached to the same carbon, then said two R^{3a} groups can be taken together as C(=O);
each R^{3b} is independently halogen, C₁-C₄ alkyl, C₁-C₄ haloalkyl or C₁-C₄ alkoxy;
R⁴ is C₁-C₆ alkyl, C₁-C₆ haloalkyl, C₃-C₆ alkenyl, C₃-C₆ haloalkenyl, C₃-C₆ alkynyl, C₃-C₆ haloalkynyl; or R⁴ is C₃-C₇ cycloalkyl or C₃-C₇ cycloalkenyl, each optionally substituted with 1-4 R¹³; or R⁴ is a saturated or partially saturated 5-, 6- or 7-membered heterocyclic ring containing 1 to 2 heteroatoms independently selected from the group nitrogen, oxygen, and sulfur, and each heterocyclic ring is optionally substituted with 1-4 R^{3a}; or R⁴ is phenyl optionally substituted on the phenyl ring with C₁-C₃ alkyl, C₁-C₃ haloalkyl, halogen, cyano or nitro; or
R¹ and R⁴ can be taken together with the two nitrogen atoms to which they are attached to form a saturated or partially saturated 5-, 6- or 7-membered heterocyclic ring containing an optional third heteroatom selected from oxygen, sulfur or nitrogen, and said heterocyclic ring can be optionally substituted with 1-4 R⁹;
R⁵ is H or C₁-C₄ alkyl; or
R⁴ and R⁵ can be taken together with the nitrogen atom to which they are attached to form a saturated or partially saturated 5-, 6- or 7-membered heterocyclic ring containing an optional second heteroatom selected from oxygen, sulfur or nitrogen, and said heterocyclic ring can be optionally substituted with 1-4 R⁹;
R⁶ is H or C₁-C₄ alkyl;
R⁷ is H or C₁-C₄ alkyl;
each R⁸ is independently C₁-C₄ alkyl;
each R⁹ is independently C₁-C₄ alkyl; or when two R⁹ are attached to the same carbon, then said two R⁹ groups can be taken together as C(=O);
A is, together with the carbon to which it is attached, a fully or partially saturated 5-, 6- or 7-membered heterocyclic ring containing one or two X, provided that (a) when X is other than O or S(O)ₙ, then only one X may be present; (b) when two X are present in the ring, they cannot be bonded directly to each other; and (c) said heterocyclic ring is bonded to the group (CR⁶R⁷)_{q} through other than X;
X is O, S(O)ₙ, NR¹⁰ or Si(R¹¹)₂;
R¹⁰ is H, C₁-C₄ alkyl, C₁-C₄ haloalkyl, C₃-C₄ alkenyl, C₃-C₄ alkynyl, C₂-C₄ alkoxycarbonyl or C₂-C₄ alkylcarbonyl; or R¹⁰ is phenyl optionally substituted on the phenyl ring with C₁-C₃ alkyl, halogen, cyano, nitro or C₂-C₄ alkoxycarbonyl;
each R¹¹ is independently C₁-C₄ alkyl;
R¹² is halogen, C₁-C₄ alkyl, C₁-C₄ haloalkyl, C₁-C₄ alkoxy, C₁-C₄ haloalkoxy, C₁-C₄ alkylthio, C₁-C₄ thioalkoxy, C₁-C₄ halothioalkoxy or C₂-C₄ alkoxycarbonyl;
each R¹³ is independently halogen, C₁-C₄ alkyl or C₁-C₄ alkoxy; or when two R¹³ are attached to the same carbon, then said two R¹³ groups can be taken together as C(=O):
m is 0, 1, 2, 3 or 4;
each n is independently 0, 1 or 2;
q is 0, 1 or 2; and
t is 0, 1 or 2.

2. The compound of Claim 1 wherein
Q is 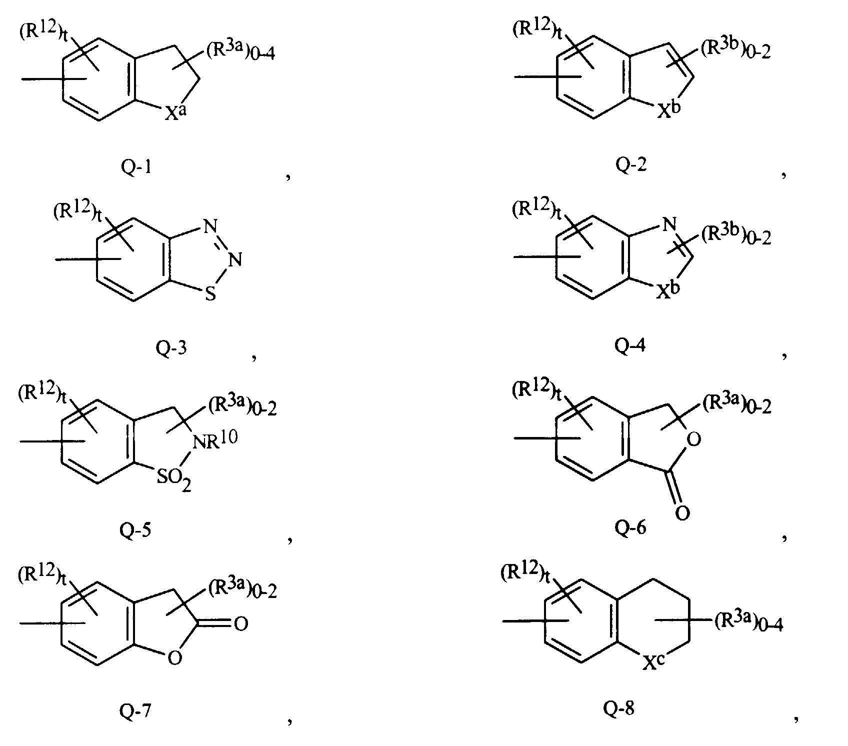 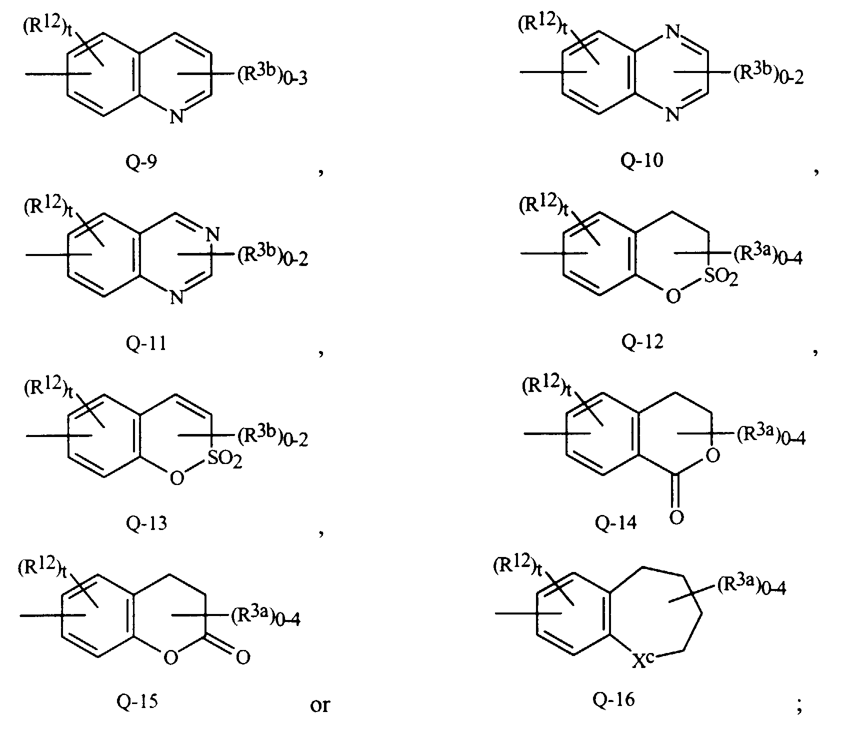 and
X^{c} is O or S(O)ₙ.

3. The compound of Claim 2 wherein
Q is Q-1, Q-2 or Q-8.

4. The compound of Claim 3 wherein
Q is 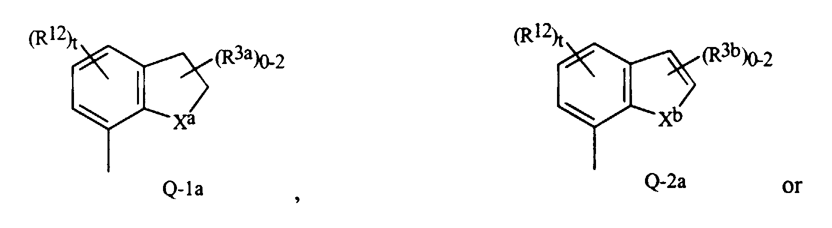 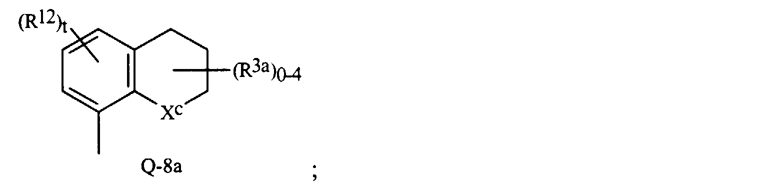 and
R^{3a} is C₁-C₄ alkyl.

5. The compound of Claim 4 wherein
X^{a}, X^{b}, and X^{c} are independently O or S.

6. The compound of Claim 5 wherein
R¹ is C₁-C₆ alkyl or C₁-C₆ haloalkyl; or R¹ is C₃-C₇ cycloalkyl or C₃-C₇ cycloalkenyl, each optionally substituted with 1-4 R¹³;
R² is C₁-C₆ alkyl or C₃-C₇ cycloalkyl; or
R¹ and R² can be taken together as -CH₂CH₂-, -CH₂CH₂CH₂-, -CH₂CH₂CH₂CH₂-, -CH₂CH₂CH₂CH₂CH₂- or -CH₂CH₂OCH₂CH₂-.

7. The compound of Claim 5 wherein
R¹ is C₁-C₆ alkyl or C₁-C₆ haloalkyl; or R¹ is C₃-C₇ cycloalkyl or C₃-C₇ cycloalkenyl, each optionally substituted with 1-4 R¹³;
R² is 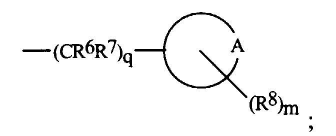
A together with the carbon to which it is attached form a fully or partially saturated 5-, 6- or 7-membered heterocyclic ring containing one X;
X is O, S or NR¹⁰; and
q is 0.

8. The compound of Claim 5 wherein
R¹ is C₃-C₇ cycloalkyl or C₃-C₇ cycloalkenyl, each optionally substituted with 1-4 R¹³;
R² is NR⁴R⁵; and
R⁴ is C₁-C₆ alkyl.

9. The compound of Claim 5 wherein
R¹ is C₁-C₆ alkyl;
R² is NR⁴R⁵; and
R⁴ is C₃-C₇ cycloalkyl or C₃-C₇ cycloalkenyl, each optionally substituted with 1-4 R¹³.

10. The compound of Claim 5 which is 4-(2,3-dihydro-2-methyl-7-benzofuranyl)-*N,N*-diethyl-4,5-dihydro-5-oxo-1*H*-tetrazol-1-carboxamide.

11. A herbicidal composition comprising a herbicidally effective amount of a compound of Claim 1 and at least one of a surfactant, a solid diluent or a liquid diluent.

12. A method for controlling the growth of undesired vegetation comprising contacting the vegetation or its environment with a herbicidally effective amount of a compound of Claim 1.
